# EUROPEAN PATENT SPECIFICATION

(11) **EP 3 011 331 B1**
(45) Date of publication and mention of the grant of the patent: **13.03.2019**
(21) Application number: 14813743.3
(22) Date of filing: 16.06.2014
(51) Int. Cl.: G01N 33/48, A61K 31/18, A61K 31/404, A61K 31/4439, A61K 31/472, A61K 31/473, A61K 31/4188, A61K 31/4427, A61K 31/4745, G01N 33/68

(54) **GI PROTEIN PHOSPHORYLATION AS MARKER FOR SCOLIOSIS AND SCOLIOSIS PROGRESSION, METHODS OF INCREASING GIPCR SIGNALING IN SCOLIOTIC SUBJECTS**
GI-PROTEINPHOSPHORYLIERUNG ALS MARKER FÜR SKOLIOSE UND SKOLIOSEPROGRESSION SOWIE VERFAHREN ZUR ERHÖHUNG DER GIPCR-SIGNALISIERUNG BEI PERSONEN MIT SKOLIOSE
PHOSPHORYLATION DE LA PROTÉINE GI EN TANT QUE MARQUEUR POUR LA SCOLIOSE ET POUR LA PROGRESSION DE LA SCOLIOSE, MÉTHODES D'AUGMENTATION DE LA SIGNALISATION GIPCR CHEZ DES SUJETS SCOLIOTIQUES

(30) Priority: 17.06.2013 US 201361835839 P
(43) Date of publication of application: 27.04.2016
(73) Proprietor: CHU Sainte-Justine, Montréal, Québec H3T 1C5 (CA)
(72) Inventor: MOREAU, Alain, Montréal Québec H1A 5T5 (CA); AKOUME NDONG, Marie-Yvonne, Montréal Québec H4L 5C1 (CA)
(74) Representative: Regimbeau
(86) International application number: PCT/CA2014/050562
(87) International publication number: WO 2014/201557

(56) References cited:
- WO-A1-2010/040234
- BAGNALL KEITH M ET AL: "The International Research Society of Spinal Deformities (IRSSD) and its contribution to science", SCOLIOSIS, BIOMED CENTRAL LTD, LO, vol. 4, no. 1, 22 December 2009 (2009-12-22), page 28, XP021069416, ISSN: 1748-7161
- MOREAU ALAIN ET AL: "Melatonin signaling dysfunction in adolescent idiopathic scoliosis", SPINE, LIPPINCOTT WILLIAMS & WILKINS, US, vol. 29, no. 16, 15 August 2004 (2004-08-15), pages 1772-1781, XP009129278, ISSN: 0362-2436, DOI: 10.1097/01.BRS.0000134567.52303.1A
- MARIE-YVONNE AKOUME ET AL: "Cell-based Assay Protocol for the Prognostic Prediction of Idiopathic Scoliosis Using Cellular Dielectric Spectroscopy", JOURNAL OF VISUALIZED EXPERIMENTS, vol. 80, no. E50768, 16 October 2013 (2013-10-16), pages 1-9, XP055323360, DOI: 10.3791/50768
- MOREAU ET AL.: 'Melatonin signalling dysfunction in adolescent idiopathic scoliosis' SPINE vol. 29, no. 16, 15 August 2004, pages 1772 - 1781, XP009129278
- LETELLIER ET AL.: 'Récents progrès dans l'étiopathogénie de la scoliose idiopathique de l'adolescent et nouveaux concepts moléculaires' MEDECINE/SCIENCES vol. 23, 2007, pages 910 - 916, XP055020714

### SEQUENCE LISTING

<110> Hopital Ste-Justine Moreau, Alain Akoume Ndong, Marie-Yvonne
<120> GI PROTEIN PHOSPHORYLATION AS MARKER FOR SCOLIOSIS AND SCOLIOSIS PROGRESSION, METHODS OF INCREASING GIPCR SIGNALING IN SCOLIOTIC SUBJECTS
<130> 765/14033_122
<140> N/A
   <141> 2014-06-16
<150> US 61/835,839
   <151> 2013-06-17
<160> 100
<170> PatentIn version 3.5
<210> 1
   <211> 23
   <212> DNA
   <213> ARTIFICIAL
<220>
   <223> Synthetic Construct
<400> 1
   agggctatgg ggaggttgaa gat 23
<210> 2
   <211> 22
   <212> DNA
   <213> ARTIFICIAL
<220>
   <223> Synthetic Construct
<400> 2
   actccagcaa gttctgcagt ca 22
<210> 3
   <211> 24
   <212> DNA
   <213> ARTIFICIAL
<220>
   <223> Synthetic Construct
<400> 3
   agggaatacc agctcaacga ctca 24
<210> 4
   <211> 24
   <212> DNA
   <213> ARTIFICIAL
<220>
   <223> Synthetic Construct
<400> 4
   tgtgtgggga tgtagtcact ctgt 24
<210> 5
   <211> 22
   <212> DNA
   <213> ARTIFICIAL
<220>
   <223> Synthetic Construct
<400> 5
   gagagtgaag accacaggca tt 22
<210> 6
   <211> 22
   <212> DNA
   <213> ARTIFICIAL
<220>
   <223> Synthetic Construct
<400> 6
   cgttctgatc tttggccacc ta 22
<210> 7
   <211> 22
   <212> DNA
   <213> ARTIFICIAL
<220>
   <223> Synthetic Construct
<400> 7
   gagaccaagt tccaggtgga ca 22
<210> 8
   <211> 20
   <212> DNA
   <213> ARTIFICIAL
<220>
   <223> Synthetic Construct
<400> 8
   gatccacttg cggcgttcat 20
<210> 9
   <211> 20
   <212> DNA
   <213> ARTIFICIAL
<220>
   <223> Synthetic Construct
<400> 9
   atcagaacat cttcacggcc 20
<210> 10
   <211> 20
   <212> DNA
   <213> ARTIFICIAL
<220>
   <223> Synthetic Construct
<400> 10
   aaagcagaca ccttctccac 20
<210> 11
   <211> 22
   <212> DNA
   <213> ARTIFICIAL
<220>
   <223> Synthetic Construct
<400> 11
   cggaatggat cacactgaga ag 22
<210> 12
   <211> 21
   <212> DNA
   <213> ARTIFICIAL
<220>
   <223> Synthetic Construct
<400> 12
   acataaggat ctgaaagccc g 21
<210> 13
   <211> 20
   <212> DNA
   <213> ARTIFICIAL
<220>
   <223> Synthetic Construct
<400> 13
   ttcccgatcc caaaagtgag 20
<210> 14
   <211> 23
   <212> DNA
   <213> ARTIFICIAL
<220>
   <223> Synthetic Construct
<400> 14
   gtcaaatccc aatcccaaat ctc 23
<210> 15
   <211> 23
   <212> DNA
   <213> ARTIFICIAL
<220>
   <223> Synthetic Construct
<400> 15
   gcttcaaggt tcacaactac atg 23
<210> 16
   <211> 20
   <212> DNA
   <213> ARTIFICIAL
<220>
   <223> Synthetic Construct
<400> 16
   accttctccc ggcatttatg 20
<210> 17
   <211> 20
   <212> DNA
   <213> ARTIFICIAL
<220>
   <223> Synthetic Construct
<400> 17
   cctaccttct gcgatcactg 20
<210> 18
   <211> 20
   <212> DNA
   <213> ARTIFICIAL
<220>
   <223> Synthetic Construct
<400> 18
   tactttggcg attcctctgg 20
<210> 19
   <211> 20
   <212> DNA
   <213> ARTIFICIAL
<220>
   <223> Synthetic Construct
<400> 19
   gtaaatgcgg tggaacttgc 20
<210> 20
   <211> 20
   <212> DNA
   <213> ARTIFICIAL
<220>
   <223> Synthetic Construct
<400> 20
   accccaatcc catttccttc 20
<210> 21
   <211> 21
   <212> DNA
   <213> ARTIFICIAL
<220>
   <223> Synthetic Construct
<400> 21
   gagaagcatg tgtttgagca g 21
<210> 22
   <211> 21
   <212> DNA
   <213> ARTIFICIAL
<220>
   <223> Synthetic Construct
<400> 22
   ggaagttttc tttgtcgctg c 21
<210> 23
   <211> 20
   <212> DNA
   <213> ARTIFICIAL
<220>
   <223> Synthetic Construct
<400> 23
   acgaagtcaa gagccacaag 20
<210> 24
   <211> 20
   <212> DNA
   <213> ARTIFICIAL
<220>
   <223> Synthetic Construct
<400> 24
   gtcgatgaac cacaaagctg 20
<210> 25
   <211> 21
   <212> DNA
   <213> ARTIFICIAL
<220>
   <223> Synthetic Construct
<400> 25
   cggattttgg aatgtgcaag g 21
<210> 26
   <211> 21
   <212> DNA
   <213> ARTIFICIAL
<220>
   <223> Synthetic Construct
<400> 26
   cataaaggag aaccccgaag g 21
<210> 27
   <211> 21
   <212> DNA
   <213> ARTIFICIAL
<220>
   <223> Synthetic Construct
<400> 27
   tgcttacatt tcctcatccc g 21
<210> 28
   <211> 20
   <212> DNA
   <213> ARTIFICIAL
<220>
   <223> Synthetic Construct
<400> 28
   cgcccgatga ctctgattag 20
<210> 29
   <211> 20
   <212> DNA
   <213> ARTIFICIAL
<220>
   <223> Synthetic Construct
<400> 29
   aatggagggc aaaggagatg 20
<210> 30
   <211> 21
   <212> DNA
   <213> ARTIFICIAL
<220>
   <223> Synthetic Construct
<400> 30
   aagatgtagg caatcactcc g 21
<210> 31
   <211> 21
   <212> DNA
   <213> ARTIFICIAL
<220>
   <223> Synthetic Construct
<400> 31
   cttgagaagg atccgaacga g 21
<210> 32
   <211> 20
   <212> DNA
   <213> ARTIFICIAL
<220>
   <223> Synthetic Construct
<400> 32
   ttgcctccac ttgctcttag 20
<210> 33
   <211> 22
   <212> DNA
   <213> ARTIFICIAL
<220>
   <223> Synthetic Construct
<400> 33
   cagttccagc gttcagttaa tg 22
<210> 34
   <211> 23
   <212> DNA
   <213> ARTIFICIAL
<220>
   <223> Synthetic Construct
<400> 34
   ttcgtgtagg actcaaaatc tcc 23
<210> 35
   <211> 22
   <212> DNA
   <213> ARTIFICIAL
<220>
   <223> Synthetic Construct
<400> 35
   ctctgacatc ctgaactctg tg 22
<210> 36
   <211> 22
   <212> DNA
   <213> ARTIFICIAL
<220>
   <223> Synthetic Construct
<400> 36
   ccgtggtctt aatgatctcc tg 22
<210> 37
   <211> 22
   <212> DNA
   <213> ARTIFICIAL
<220>
   <223> Synthetic Construct
<400> 37
   ggcacacctg gatatctttc tc 22
<210> 38
   <211> 21
   <212> DNA
   <213> ARTIFICIAL
<220>
   <223> Synthetic Construct
<400> 38
   agtctgtgtt ggtcttcatc c 21
<210> 39
   <211> 20
   <212> DNA
   <213> ARTIFICIAL
<220>
   <223> Synthetic Construct
<400> 39
   aaacagtctc gtaagcccag 20
<210> 40
   <211> 20
   <212> DNA
   <213> ARTIFICIAL
<220>
   <223> Synthetic Construct
<400> 40
   atcccatctg tagcgttgtg 20
<210> 41
   <211> 19
   <212> DNA
   <213> ARTIFICIAL
<220>
   <223> Synthetic Construct
<400> 41
   tcgcctctca catccaaac 19
<210> 42
   <211> 23
   <212> DNA
   <213> ARTIFICIAL
<220>
   <223> Synthetic Construct
<400> 42
   catctcgctc actgtaatat ccc 23
<210> 43
   <211> 21
   <212> DNA
   <213> ARTIFICIAL
<220>
   <223> Synthetic Construct
<400> 43
   aggacaccaa caacttcttc g 21
<210> 44
   <211> 20
   <212> DNA
   <213> ARTIFICIAL
<220>
   <223> Synthetic Construct
<400> 44
   ggtgccttgt cggtcatatt 20
<210> 45
   <211> 22
   <212> DNA
   <213> ARTIFICIAL
<220>
   <223> Synthetic Construct
<400> 45
   ctcagttcct ggagaaagat gg 22
<210> 46
   <211> 21
   <212> DNA
   <213> ARTIFICIAL
<220>
   <223> Synthetic Construct
<400> 46
   cccagtcaat tcatgtttgc c 21
<210> 47
   <211> 22
   <212> DNA
   <213> ARTIFICIAL
<220>
   <223> Synthetic Construct
<400> 47
   atggaatatg tgtctggagg tg 22
<210> 48
   <211> 21
   <212> DNA
   <213> ARTIFICIAL
<220>
   <223> Synthetic Construct
<400> 48
   tggtttcagg tctcgatgaa c 21
<210> 49
   <211> 21
   <212> DNA
   <213> ARTIFICIAL
<220>
   <223> Synthetic Construct
<400> 49
   gagtaaactt cccctcacca g 21
<210> 50
   <211> 20
   <212> DNA
   <213> ARTIFICIAL
<220>
   <223> Synthetic Construct
<400> 50
   tccactgacc atccacaaag 20
<210> 51
   <211> 20
   <212> DNA
   <213> ARTIFICIAL
<220>
   <223> Synthetic Construct
<400> 51
   cactgttatc cgctggtctg 20
<210> 52
   <211> 21
   <212> DNA
   <213> ARTIFICIAL
<220>
   <223> Synthetic Construct
<400> 52
   cttgtattgg tgctctccct c 21
<210> 53
   <211> 26
   <212> DNA
   <213> ARTIFICIAL
<220>
   <223> Synthetic Construct
<400> 53
   caaggacaac tcaaacttat acatgg 26
<210> 54
   <211> 22
   <212> DNA
   <213> ARTIFICIAL
<220>
   <223> Synthetic Construct
<400> 54
   cagatactca aaggtcagga cg 22
<210> 55
   <211> 21
   <212> DNA
   <213> ARTIFICIAL
<220>
   <223> Synthetic Construct
<400> 55
   cctttccttg ttcgactgga g 21
<210> 56
   <211> 19
   <212> DNA
   <213> ARTIFICIAL
<220>
   <223> Synthetic Construct
<400> 56
   tgagctgcat agaaccgtg 19
<210> 57
   <211> 22
   <212> DNA
   <213> ARTIFICIAL
<220>
   <223> Synthetic Construct
<400> 57
   ccggatctcc atcaatgaga ag 22
<210> 58
   <211> 20
   <212> DNA
   <213> ARTIFICIAL
<220>
   <223> Synthetic Construct
<400> 58
   ttcaatccaa ccctcccatc 20
<210> 59
   <211> 20
   <212> DNA
   <213> ARTIFICIAL
<220>
   <223> Synthetic Construct
<400> 59
   gcgcattctg aagttcctca 20
<210> 60
   <211> 21
   <212> DNA
   <213> ARTIFICIAL
<220>
   <223> Synthetic Construct
<400> 60
   aaaatcccca gagccacata g 21
<210> 61
   <211> 21
   <212> DNA
   <213> ARTIFICIAL
<220>
   <223> Synthetic Construct
<400> 61
   ttgcccgtta ttgaccctat c 21
<210> 62
   <211> 22
   <212> DNA
   <213> ARTIFICIAL
<220>
   <223> Synthetic Construct
<400> 62
   cgttcctatt ccaagctcat cc 22
<210> 63
   <211> 21
   <212> DNA
   <213> ARTIFICIAL
<220>
   <223> Synthetic Construct
<400> 63
   tgactgcact ggacatcttt g 21
<210> 64
   <211> 19
   <212> DNA
   <213> ARTIFICIAL
<220>
   <223> Synthetic Construct
<400> 64
   tggttgtagg tttgctggg 19
<210> 65
   <211> 23
   <212> DNA
   <213> ARTIFICIAL
<220>
   <223> Synthetic Construct
<400> 65
   tgtcggaggg aaatataaac tgg 23
<210> 66
   <211> 22
   <212> DNA
   <213> ARTIFICIAL
<220>
   <223> Synthetic Construct
<400> 66
   ggccttctga gattctagct tc 22
<210> 67
   <211> 18
   <212> DNA
   <213> ARTIFICIAL
<220>
   <223> Synthetic Construct
<400> 67
   aggtggaggt agtggagg 18
<210> 68
   <211> 22
   <212> DNA
   <213> ARTIFICIAL
<220>
   <223> Synthetic Construct
<400> 68
   gtacaattga gtcagagtcc cc 22
<210> 69
   <211> 21
   <212> DNA
   <213> ARTIFICIAL
<220>
   <223> Synthetic Construct
<400> 69
   gtgatgttct agccacagag g 21
<210> 70
   <211> 21
   <212> DNA
   <213> ARTIFICIAL
<220>
   <223> Synthetic Construct
<400> 70
   ccctttccct cctttcttgt c 21
<210> 71
   <211> 21
   <212> DNA
   <213> ARTIFICIAL
<220>
   <223> Synthetic Construct
<400> 71
   gttcaaatgc acccatcaca g 21
<210> 72
   <211> 21
   <212> DNA
   <213> ARTIFICIAL
<220>
   <223> Synthetic Construct
<400> 72
   agtaactccc caggatctgt c 21
<210> 73
   <211> 22
   <212> DNA
   <213> ARTIFICIAL
<220>
   <223> Synthetic Construct
<400> 73
   gtatgagatt ctgaaggccc tg 22
<210> 74
   <211> 22
   <212> DNA
   <213> ARTIFICIAL
<220>
   <223> Synthetic Construct
<400> 74
   ccaaacccca gtctattagt cg 22
<210> 75
   <211> 22
   <212> DNA
   <213> ARTIFICIAL
<220>
   <223> Synthetic Construct
<400> 75
   cgatacgacc atcaacagag ac 22
<210> 76
   <211> 20
   <212> DNA
   <213> ARTIFICIAL
<220>
   <223> Synthetic Construct
<400> 76
   tcgctttcca gtcttcatcg 20
<210> 77
   <211> 1065
   <212> DNA
   <213> Homo sapiens
<400> 77
   atgggctgca cgctgagcgc cgaggacaag gcggcggtgg agcggagtaa gatgatcgac 60
   cgcaacctcc gtgaggacgg cgagaaggcg gcgcgcgagg tcaagctgct gctgctcggt 120
   gctggtgaat ctggtaaaag tacaattgtg aagcagatga aaattatcca tgaagctggt 180
   tattcagaag aggagtgtaa acaatacaaa gcagtggtct acagtaacac catccagtca 240
   attattgcta tcattagggc tatggggagg ttgaagatag actttggtga ctcagcccgg 300
   gcggatgatg cacgccaact ctttgtgcta gctggagctg ctgaagaagg ctttatgact 360
   gcagaacttg ctggagttat aaagagattg tggaaagata gtggtgtaca agcctgtttc 420
   aacagatccc gagagtacca gcttaatgat tctgcagcat actatttgaa tgacttggac 480
   agaatagctc aaccaaatta catcccgact caacaagatg ttctcagaac tagagtgaaa 540
   actacaggaa ttgttgaaac ccattttact ttcaaagatc ttcattttaa aatgtttgat 600
   gtgggaggtc agagatctga gcggaagaag tggattcatt gcttcgaagg agtgacggcg 660
   atcatcttct gtgtagcact gagtgactac gacctggttc tagctgaaga tgaagaaatg 720
   aaccgaatgc atgaaagcat gaaattgttt gacagcatat gtaacaacaa gtggtttaca 780
   gatacatcca ttatactttt tctaaacaag aaggatctct ttgaagaaaa aatcaaaaag 840
   agccctctca ctatatgcta tccagaatat gcaggatcaa acacatatga agaggcagct 900
   gcatatattc aatgtcagtt tgaagacctc aataaaagaa aggacacaaa ggaaatatac 960
   acccacttca catgtgccac agatactaag aatgtgcagt ttgtttttga tgctgtaaca 1020
   gatgtcatca taaaaaataa tctaaaagat tgtggtctct tttaa 1065
<210> 78
   <211> 354
   <212> PRT
   <213> Homo sapiens
<400> 78
<210> 79
   <211> 909
   <212> DNA
   <213> Homo sapiens
<400> 79
   atgaaaatta tccatgaagc tggttattca gaagaggagt gtaaacaata caaagcagtg      60
   gtctacagta acaccatcca gtcaattatt gctatcatta gggctatggg gaggttgaag     120
   atagactttg gtgactcagc ccgggcggat gatgcacgcc aactctttgt gctagctgga     180
   gctgctgaag aaggctttat gactgcagaa cttgctggag ttataaagag attgtggaaa     240
   gatagtggtg tacaagcctg tttcaacaga tcccgagagt accagcttaa tgattctgca     300
   gcatactatt tgaatgactt ggacagaata gctcaaccaa attacatccc gactcaacaa     360
   gatgttctca gaactagagt gaaaactaca ggaattgttg aaacccattt tactttcaaa     420
   gatcttcatt ttaaaatgtt tgatgtggga ggtcagagat ctgagcggaa gaagtggatt     480
   cattgcttcg aaggagtgac ggcgatcatc ttctgtgtag cactgagtga ctacgacctg     540
   gttctagctg aagatgaaga aatgaaccga atgcatgaaa gcatgaaatt gtttgacagc     600
   atatgtaaca acaagtggtt tacagataca tccattatac tttttctaaa caagaaggat     660
   ctctttgaag aaaaaatcaa aaagagccct ctcactatat gctatccaga atatgcagga     720
   tcaaacacat atgaagaggc agctgcatat attcaatgtc agtttgaaga cctcaataaa     780
   agaaaggaca caaaggaaat atacacccac ttcacatgtg ccacagatac taagaatgtg     840
   cagtttgttt ttgatgctgt aacagatgtc atcataaaaa ataatctaaa agattgtggt     900
   ctcttttaa                                                             909
<210> 80
   <211> 302
   <212> PRT
   <213> Homo sapiens
<400> 80
   Met Lys Ile Ile His Glu Ala Gly Tyr Ser Glu Glu Glu Cys Lys Gln
   1               5                   10                  15
   Tyr Lys Ala Val Val Tyr Ser Asn Thr Ile Gln Ser Ile Ile Ala Ile
               20                  25                  30
   Ile Arg Ala Met Gly Arg Leu Lys Ile Asp Phe Gly Asp Ser Ala Arg
           35                  40                  45
   Ala Asp Asp Ala Arg Gln Leu Phe Val Leu Ala Gly Ala Ala Glu Glu
       50                  55                  60
   Gly Phe Met Thr Ala Glu Leu Ala Gly Val Ile Lys Arg Leu Trp Lys
   65                  70                  75                  80
   Asp Ser Gly Val Gln Ala Cys Phe Asn Arg Ser Arg Glu Tyr Gln Leu
                   85                  90                  95
   Asn Asp Ser Ala Ala Tyr Tyr Leu Asn Asp Leu Asp Arg Ile Ala Gln
               100                 105                 110
   Pro Asn Tyr Ile Pro Thr Gln Gln Asp Val Leu Arg Thr Arg Val Lys
           115                 120                 125
   Thr Thr Gly Ile Val Glu Thr His Phe Thr Phe Lys Asp Leu His Phe
       130                 135                 140
   Lys Met Phe Asp Val Gly Gly Gln Arg Ser Glu Arg Lys Lys Trp Ile
   145                 150                 155                 160
   His Cys Phe Glu Gly Val Thr Ala Ile Ile Phe Cys Val Ala Leu Ser
                   165                 170                 175
   Asp Tyr Asp Leu Val Leu Ala Glu Asp Glu Glu Met Asn Arg Met His
               180                 185                 190
   Glu Ser Met Lys Leu Phe Asp Ser Ile Cys Asn Asn Lys Trp Phe Thr
           195                 200                 205
   Asp Thr Ser Ile Ile Leu Phe Leu Asn Lys Lys Asp Leu Phe Glu Glu
       210                 215                 220
   Lys Ile Lys Lys Ser Pro Leu Thr Ile Cys Tyr Pro Glu Tyr Ala Gly
   225                 230                 235                 240
   Ser Asn Thr Tyr Glu Glu Ala Ala Ala Tyr Ile Gln Cys Gln Phe Glu
                   245                 250                 255
   Asp Leu Asn Lys Arg Lys Asp Thr Lys Glu Ile Tyr Thr His Phe Thr
               260                 265                 270
   Cys Ala Thr Asp Thr Lys Asn Val Gln Phe Val Phe Asp Ala Val Thr
           275                 280                 285
   Asp Val Ile Ile Lys Asn Asn Leu Lys Asp Cys Gly Leu Phe
       290                 295                 300
<210> 81
   <211> 1068
   <212> DNA
   <213> Homo sapiens
<400> 81
   atgggctgca ccgtgagcgc cgaggacaag gcggcggccg agcgctctaa gatgatcgac      60
   aagaacctgc gggaggacgg agagaaggcg gcgcgggagg tgaagttgct gctgttgggt     120
   gctggggagt cagggaagag caccatcgtc aagcagatga agatcatcca cgaggatggc     180
   tactccgagg aggaatgccg gcagtaccgg gcggttgtct acagcaacac catccagtcc     240
   atcatggcca ttgtcaaagc catgggcaac ctgcagatcg actttgccga cccctccaga     300
   gcggacgacg ccaggcagct atttgcactg tcctgcaccg ccgaggagca aggcgtgctc     360
   cctgatgacc tgtccggcgt catccggagg ctctgggctg accatggtgt gcaggcctgc     420
   tttggccgct caagggaata ccagctcaac gactcagctg cctactacct gaacgacctg     480
   gagcgtattg cacagagtga ctacatcccc acacagcaag atgtgctacg gacccgcgta     540
   aagaccacgg ggatcgtgga gacacacttc accttcaagg acctacactt caagatgttt     600
   gatgtgggtg gtcagcggtc tgagcggaag aagtggatcc actgctttga gggcgtcaca     660
   gccatcatct tctgcgtagc cttgagcgcc tatgacttgg tgctagctga ggacgaggag     720
   atgaaccgca tgcatgagag catgaagcta ttcgatagca tctgcaacaa caagtggttc     780
   acagacacgt ccatcatcct cttcctcaac aagaaggacc tgtttgagga gaagatcaca     840
   cacagtcccc tgaccatctg cttccctgag tacacagggg ccaacaaata tgatgaggca     900
   gccagctaca tccagagtaa gtttgaggac ctgaataagc gcaaagacac caaggagatc     960
   tacacgcact tcacgtgcgc caccgacacc aagaacgtgc agttcgtgtt tgacgccgtc    1020
   accgatgtca tcatcaagaa caacctgaag gactgcggcc tcttctga                 1068
<210> 82
   <211> 355
   <212> PRT
   <213> Homo sapiens
<400> 82
   Met Gly Cys Thr Val Ser Ala Glu Asp Lys Ala Ala Ala Glu Arg Ser
   1               5                   10                  15
   Lys Met Ile Asp Lys Asn Leu Arg Glu Asp Gly Glu Lys Ala Ala Arg
               20                  25                  30
   Glu Val Lys Leu Leu Leu Leu Gly Ala Gly Glu Ser Gly Lys Ser Thr
           35                  40                  45
   Ile Val Lys Gln Met Lys Ile Ile His Glu Asp Gly Tyr Ser Glu Glu
       50                  55                  60
   Glu Cys Arg Gln Tyr Arg Ala Val Val Tyr Ser Asn Thr Ile Gln Ser
   65                  70                  75                  80
   Ile Met Ala Ile Val Lys Ala Met Gly Asn Leu Gln Ile Asp Phe Ala
                   85                  90                  95
   Asp Pro Ser Arg Ala Asp Asp Ala Arg Gln Leu Phe Ala Leu Ser Cys
               100                 105                 110
   Thr Ala Glu Glu Gln Gly Val Leu Pro Asp Asp Leu Ser Gly Val Ile
           115                 120                 125
   Arg Arg Leu Trp Ala Asp His Gly Val Gln Ala Cys Phe Gly Arg Ser
       130                 135                 140
   Arg Glu Tyr Gln Leu Asn Asp Ser Ala Ala Tyr Tyr Leu Asn Asp Leu
   145                 150                 155                 160
   Glu Arg Ile Ala Gln Ser Asp Tyr Ile Pro Thr Gln Gln Asp Val Leu
                   165                 170                 175
   Arg Thr Arg Val Lys Thr Thr Gly Ile Val Glu Thr His Phe Thr Phe
               180                 185                 190
   Lys Asp Leu His Phe Lys Met Phe Asp Val Gly Gly Gln Arg Ser Glu
           195                 200                 205
   Arg Lys Lys Trp Ile His Cys Phe Glu Gly Val Thr Ala Ile Ile Phe
       210                 215                 220
   Cys Val Ala Leu Ser Ala Tyr Asp Leu Val Leu Ala Glu Asp Glu Glu
   225                 230                 235                 240
   Met Asn Arg Met His Glu Ser Met Lys Leu Phe Asp Ser Ile Cys Asn
                   245                 250                 255
   Asn Lys Trp Phe Thr Asp Thr Ser Ile Ile Leu Phe Leu Asn Lys Lys
               260                 265                 270
   Asp Leu Phe Glu Glu Lys Ile Thr His Ser Pro Leu Thr Ile Cys Phe
           275                 280                 285
   Pro Glu Tyr Thr Gly Ala Asn Lys Tyr Asp Glu Ala Ala Ser Tyr Ile
       290                 295                 300
   Gln Ser Lys Phe Glu Asp Leu Asn Lys Arg Lys Asp Thr Lys Glu Ile
   305 310 315 320
   Tyr Thr His Phe Thr Cys Ala Thr Asp Thr Lys Asn Val Gln Phe Val
   325 330 335
   Phe Asp Ala Val Thr Asp Val Ile Ile Lys Asn Asn Leu Lys Asp Cys
   340 345 350
   Gly Leu Phe
   355
<210> 83
   <211> 957
   <212> DNA
   <213> Homo sapiens
<400> 83
   atgagaggtg ctggggagtc agggaagagc accatcgtca agcagatgaa gatcatccac 60
   gaggatggct actccgagga ggaatgccgg cagtaccggg cggttgtcta cagcaacacc 120
   atccagtcca tcatggccat tgtcaaagcc atgggcaacc tgcagatcga ctttgccgac 180
   ccctccagag cggacgacgc caggcagcta tttgcactgt cctgcaccgc cgaggagcaa 240
   ggcgtgctcc ctgatgacct gtccggcgtc atccggaggc tctgggctga ccatggtgtg 300
   caggcctgct ttggccgctc aagggaatac cagctcaacg actcagctgc ctactacctg 360
   aacgacctgg agcgtattgc acagagtgac tacatcccca cacagcaaga tgtgctacgg 420
   acccgcgtaa agaccacggg gatcgtggag acacacttca ccttcaagga cctacacttc 480
   aagatgtttg atgtgggtgg tcagcggtct gagcggaaga agtggatcca ctgctttgag 540
   ggcgtcacag ccatcatctt ctgcgtagcc ttgagcgcct atgacttggt gctagctgag 600
   gacgaggaga tgaaccgcat gcatgagagc atgaagctat tcgatagcat ctgcaacaac 660
   aagtggttca cagacacgtc catcatcctc ttcctcaaca agaaggacct gtttgaggag 720
   aagatcacac acagtcccct gaccatctgc ttccctgagt acacaggggc caacaaatat 780
   gatgaggcag ccagctacat ccagagtaag tttgaggacc tgaataagcg caaagacacc 840
   aaggagatct acacgcactt cacgtgcgcc accgacacca agaacgtgca gttcgtgttt 900
   gacgccgtca ccgatgtcat catcaagaac aacctgaagg actgcggcct cttctga 957
<210> 84
   <211> 318
   <212> PRT
   <213> Homo sapiens
<400> 84
   Met Arg Gly Ala Gly Glu Ser Gly Lys Ser Thr Ile Val Lys Gln Met
   1               5                   10                  15
   Lys Ile Ile His Glu Asp Gly Tyr Ser Glu Glu Glu Cys Arg Gln Tyr
               20                  25                  30
   Arg Ala Val Val Tyr Ser Asn Thr Ile Gln Ser Ile Met Ala Ile Val
           35                  40                  45
   Lys Ala Met Gly Asn Leu Gln Ile Asp Phe Ala Asp Pro Ser Arg Ala
       50                  55                  60
   Asp Asp Ala Arg Gln Leu Phe Ala Leu Ser Cys Thr Ala Glu Glu Gln
   65                  70                  75                  80
   Gly Val Leu Pro Asp Asp Leu Ser Gly Val Ile Arg Arg Leu Trp Ala
                   85                  90                  95
   Asp His Gly Val Gln Ala Cys Phe Gly Arg Ser Arg Glu Tyr Gln Leu
               100                 105                 110
   Asn Asp Ser Ala Ala Tyr Tyr Leu Asn Asp Leu Glu Arg Ile Ala Gln
           115                 120                 125
   Ser Asp Tyr Ile Pro Thr Gln Gln Asp Val Leu Arg Thr Arg Val Lys
       130                 135                 140
   Thr Thr Gly Ile Val Glu Thr His Phe Thr Phe Lys Asp Leu His Phe
   145                 150                 155                 160
   Lys Met Phe Asp Val Gly Gly Gln Arg Ser Glu Arg Lys Lys Trp Ile
                   165                 170                 175
   His Cys Phe Glu Gly Val Thr Ala Ile Ile Phe Cys Val Ala Leu Ser
               180                 185                 190
   Ala Tyr Asp Leu Val Leu Ala Glu Asp Glu Glu Met Asn Arg Met His
           195                 200                 205
   Glu Ser Met Lys Leu Phe Asp Ser Ile Cys Asn Asn Lys Trp Phe Thr
       210                 215                 220
   Asp Thr Ser Ile Ile Leu Phe Leu Asn Lys Lys Asp Leu Phe Glu Glu
   225                 230                 235                 240
   Lys Ile Thr His Ser Pro Leu Thr Ile Cys Phe Pro Glu Tyr Thr Gly
                   245                 250                 255
   Ala Asn Lys Tyr Asp Glu Ala Ala Ser Tyr Ile Gln Ser Lys Phe Glu
               260                 265                 270
   Asp Leu Asn Lys Arg Lys Asp Thr Lys Glu Ile Tyr Thr His Phe Thr
           275                 280                 285
   Cys Ala Thr Asp Thr Lys Asn Val Gln Phe Val Phe Asp Ala Val Thr
       290                 295                 300
   Asp Val Ile Ile Lys Asn Asn Leu Lys Asp Cys Gly Leu Phe
   305                 310                 315
<210> 85
   <211> 912
   <212> DNA
   <213> Homo sapiens
<400> 85
   atgaagatca tccacgagga tggctactcc gaggaggaat gccggcagta ccgggcggtt      60
   gtctacagca acaccatcca gtccatcatg gccattgtca aagccatggg caacctgcag     120
   atcgactttg ccgacccctc cagagcggac gacgccaggc agctatttgc actgtcctgc     180
   accgccgagg agcaaggcgt gctccctgat gacctgtccg gcgtcatccg gaggctctgg     240
   gctgaccatg gtgtgcaggc ctgctttggc cgctcaaggg aataccagct caacgactca     300
   gctgcctact acctgaacga cctggagcgt attgcacaga gtgactacat ccccacacag     360
   caagatgtgc tacggacccg cgtaaagacc acggggatcg tggagacaca cttcaccttc     420
   aaggacctac acttcaagat gtttgatgtg ggtggtcagc ggtctgagcg gaagaagtgg     480
   atccactgct ttgagggcgt cacagccatc atcttctgcg tagccttgag cgcctatgac     540
   ttggtgctag ctgaggacga ggagatgaac cgcatgcatg agagcatgaa gctattcgat     600
   agcatctgca acaacaagtg gttcacagac acgtccatca tcctcttcct caacaagaag     660
   gacctgtttg aggagaagat cacacacagt cccctgacca tctgcttccc tgagtacaca     720
   ggggccaaca aatatgatga ggcagccagc tacatccaga gtaagtttga ggacctgaat     780
   aagcgcaaag acaccaagga gatctacacg cacttcacgt gcgccaccga caccaagaac     840
   gtgcagttcg tgtttgacgc cgtcaccgat gtcatcatca agaacaacct gaaggactgc     900
   ggcctcttct ga                                                         912
<210> 86
   <211> 303
   <212> PRT
   <213> Homo sapiens
<400> 86
   Met Lys Ile Ile His Glu Asp Gly Tyr Ser Glu Glu Glu Cys Arg Gln
   1               5                   10                  15
   Tyr Arg Ala Val Val Tyr Ser Asn Thr Ile Gln Ser Ile Met Ala Ile
               20                  25                  30
   Val Lys Ala Met Gly Asn Leu Gln Ile Asp Phe Ala Asp Pro Ser Arg
           35                  40                  45
   Ala Asp Asp Ala Arg Gln Leu Phe Ala Leu Ser Cys Thr Ala Glu Glu
       50                  55                  60
   Gln Gly Val Leu Pro Asp Asp Leu Ser Gly Val Ile Arg Arg Leu Trp
   65                  70                  75                  80
   Ala Asp His Gly Val Gln Ala Cys Phe Gly Arg Ser Arg Glu Tyr Gln
                   85                  90                  95
   Leu Asn Asp Ser Ala Ala Tyr Tyr Leu Asn Asp Leu Glu Arg Ile Ala
               100                 105                 110
   Gln Ser Asp Tyr Ile Pro Thr Gln Gln Asp Val Leu Arg Thr Arg Val
           115                 120                 125
   Lys Thr Thr Gly Ile Val Glu Thr His Phe Thr Phe Lys Asp Leu His
       130                 135                 140
   Phe Lys Met Phe Asp Val Gly Gly Gln Arg Ser Glu Arg Lys Lys Trp
   145                 150                 155                 160
   Ile His Cys Phe Glu Gly Val Thr Ala Ile Ile Phe Cys Val Ala Leu
                   165                 170                 175
   Ser Ala Tyr Asp Leu Val Leu Ala Glu Asp Glu Glu Met Asn Arg Met
               180                 185                 190
   His Glu Ser Met Lys Leu Phe Asp Ser Ile Cys Asn Asn Lys Trp Phe
           195                 200                 205
   Thr Asp Thr Ser Ile Ile Leu Phe Leu Asn Lys Lys Asp Leu Phe Glu
       210                 215                 220
   Glu Lys Ile Thr His Ser Pro Leu Thr Ile Cys Phe Pro Glu Tyr Thr
   225                 230                 235                 240
   Gly Ala Asn Lys Tyr Asp Glu Ala Ala Ser Tyr Ile Gln Ser Lys Phe
                   245                 250                 255
   Glu Asp Leu Asn Lys Arg Lys Asp Thr Lys Glu Ile Tyr Thr His Phe
   260 265 270
   Thr Cys Ala Thr Asp Thr Lys Asn Val Gln Phe Val Phe Asp Ala Val
   275 280 285
   Thr Asp Val Ile Ile Lys Asn Asn Leu Lys Asp Cys Gly Leu Phe
   290 295 300
<210> 87
   <211> 825
   <212> DNA
   <213> Homo sapiens
<400> 87
   atggccattg tcaaagccat gggcaacctg cagatcgact ttgccgaccc ctccagagcg 60
   gacgacgcca ggcagctatt tgcactgtcc tgcaccgccg aggagcaagg cgtgctccct 120
   gatgacctgt ccggcgtcat ccggaggctc tgggctgacc atggtgtgca ggcctgcttt 180
   ggccgctcaa gggaatacca gctcaacgac tcagctgcct actacctgaa cgacctggag 240
   cgtattgcac agagtgacta catccccaca cagcaagatg tgctacggac ccgcgtaaag 300
   accacgggga tcgtggagac acacttcacc ttcaaggacc tacacttcaa gatgtttgat 360
   gtgggtggtc agcggtctga gcggaagaag tggatccact gctttgaggg cgtcacagcc 420
   atcatcttct gcgtagcctt gagcgcctat gacttggtgc tagctgagga cgaggagatg 480
   aaccgcatgc atgagagcat gaagctattc gatagcatct gcaacaacaa gtggttcaca 540
   gacacgtcca tcatcctctt cctcaacaag aaggacctgt ttgaggagaa gatcacacac 600
   agtcccctga ccatctgctt ccctgagtac acaggggcca acaaatatga tgaggcagcc 660
   agctacatcc agagtaagtt tgaggacctg aataagcgca aagacaccaa ggagatctac 720
   acgcacttca cgtgcgccac cgacaccaag aacgtgcagt tcgtgtttga cgccgtcacc 780
   gatgtcatca tcaagaacaa cctgaaggac tgcggcctct tctga 825
<210> 88
   <211> 274
   <212> PRT
   <213> Homo sapiens
<400> 88
   Met Ala Ile Val Lys Ala Met Gly Asn Leu Gln Ile Asp Phe Ala Asp
   1 5 10 15
   Pro Ser Arg Ala Asp Asp Ala Arg Gln Leu Phe Ala Leu Ser Cys Thr
   20 25 30
   Ala Glu Glu Gln Gly Val Leu Pro Asp Asp Leu Ser Gly Val Ile Arg
           35                  40                  45
   Arg Leu Trp Ala Asp His Gly Val Gln Ala Cys Phe Gly Arg Ser Arg
       50                  55                  60
   Glu Tyr Gln Leu Asn Asp Ser Ala Ala Tyr Tyr Leu Asn Asp Leu Glu
   65                  70                  75                  80
   Arg Ile Ala Gln Ser Asp Tyr Ile Pro Thr Gln Gln Asp Val Leu Arg
                   85                  90                  95
   Thr Arg Val Lys Thr Thr Gly Ile Val Glu Thr His Phe Thr Phe Lys
               100                 105                 110
   Asp Leu His Phe Lys Met Phe Asp Val Gly Gly Gln Arg Ser Glu Arg
           115                 120                 125
   Lys Lys Trp Ile His Cys Phe Glu Gly Val Thr Ala Ile Ile Phe Cys
       130                 135                 140
   Val Ala Leu Ser Ala Tyr Asp Leu Val Leu Ala Glu Asp Glu Glu Met
   145                 150                 155                 160
   Asn Arg Met His Glu Ser Met Lys Leu Phe Asp Ser Ile Cys Asn Asn
                   165                 170                 175
   Lys Trp Phe Thr Asp Thr Ser Ile Ile Leu Phe Leu Asn Lys Lys Asp
               180                 185                 190
   Leu Phe Glu Glu Lys Ile Thr His Ser Pro Leu Thr Ile Cys Phe Pro
           195                 200                 205
   Glu Tyr Thr Gly Ala Asn Lys Tyr Asp Glu Ala Ala Ser Tyr Ile Gln
       210                 215                 220
   Ser Lys Phe Glu Asp Leu Asn Lys Arg Lys Asp Thr Lys Glu Ile Tyr
   225                 230                 235                 240
   Thr His Phe Thr Cys Ala Thr Asp Thr Lys Asn Val Gln Phe Val Phe
                   245                 250                 255
   Asp Ala Val Thr Asp Val Ile Ile Lys Asn Asn Leu Lys Asp Cys Gly
               260                 265                 270
   Leu Phe
<210> 89
   <211> 1020
   <212> DNA
   <213> Homo sapiens
<400> 89
   atggagtatg cagggcatct tcctgccagc tctgcccagg gcaccatatt ggcatgcacc      60
   agctgcacag gtgctgggga gtcagggaag agcaccatcg tcaagcagat gaagatcatc     120
   cacgaggatg gctactccga ggaggaatgc cggcagtacc gggcggttgt ctacagcaac     180
   accatccagt ccatcatggc cattgtcaaa gccatgggca acctgcagat cgactttgcc     240
   gacccctcca gagcggacga cgccaggcag ctatttgcac tgtcctgcac cgccgaggag     300
   caaggcgtgc tccctgatga cctgtccggc gtcatccgga ggctctgggc tgaccatggt     360
   gtgcaggcct gctttggccg ctcaagggaa taccagctca acgactcagc tgcctactac     420
   ctgaacgacc tggagcgtat tgcacagagt gactacatcc ccacacagca agatgtgcta     480
   cggacccgcg taaagaccac ggggatcgtg gagacacact tcaccttcaa ggacctacac     540
   ttcaagatgt ttgatgtggg tggtcagcgg tctgagcgga agaagtggat ccactgcttt     600
   gagggcgtca cagccatcat cttctgcgta gccttgagcg cctatgactt ggtgctagct     660
   gaggacgagg agatgaaccg catgcatgag agcatgaagc tattcgatag catctgcaac     720
   aacaagtggt tcacagacac gtccatcatc ctcttcctca acaagaagga cctgtttgag     780
   gagaagatca cacacagtcc cctgaccatc tgcttccctg agtacacagg ggccaacaaa     840
   tatgatgagg cagccagcta catccagagt aagtttgagg acctgaataa gcgcaaagac     900
   accaaggaga tctacacgca cttcacgtgc gccaccgaca ccaagaacgt gcagttcgtg     960
   tttgacgccg tcaccgatgt catcatcaag aacaacctga aggactgcgg cctcttctga    1020
<210> 90
   <211> 339
   <212> PRT
   <213> Homo sapiens
<400> 90
   Met Glu Tyr Ala Gly His Leu Pro Ala Ser Ser Ala Gln Gly Thr Ile
   1               5                   10                  15
   Leu Ala Cys Thr Ser Cys Thr Gly Ala Gly Glu Ser Gly Lys Ser Thr
               20                  25                  30
   Ile Val Lys Gln Met Lys Ile Ile His Glu Asp Gly Tyr Ser Glu Glu
           35                  40                  45
   Glu Cys Arg Gln Tyr Arg Ala Val Val Tyr Ser Asn Thr Ile Gln Ser
       50                  55                  60
   Ile Met Ala Ile Val Lys Ala Met Gly Asn Leu Gln Ile Asp Phe Ala
   65                  70                  75                  80
   Asp Pro Ser Arg Ala Asp Asp Ala Arg Gln Leu Phe Ala Leu Ser Cys
                   85                  90                  95
   Thr Ala Glu Glu Gln Gly Val Leu Pro Asp Asp Leu Ser Gly Val Ile
               100                 105                 110
   Arg Arg Leu Trp Ala Asp His Gly Val Gln Ala Cys Phe Gly Arg Ser
           115                 120                 125
   Arg Glu Tyr Gln Leu Asn Asp Ser Ala Ala Tyr Tyr Leu Asn Asp Leu
       130                 135                 140
   Glu Arg Ile Ala Gln Ser Asp Tyr Ile Pro Thr Gln Gln Asp Val Leu
   145                 150                 155                 160
   Arg Thr Arg Val Lys Thr Thr Gly Ile Val Glu Thr His Phe Thr Phe
                   165                 170                 175
   Lys Asp Leu His Phe Lys Met Phe Asp Val Gly Gly Gln Arg Ser Glu
               180                 185                 190
   Arg Lys Lys Trp Ile His Cys Phe Glu Gly Val Thr Ala Ile Ile Phe
           195                 200                 205
   Cys Val Ala Leu Ser Ala Tyr Asp Leu Val Leu Ala Glu Asp Glu Glu
       210                 215                 220
   Met Asn Arg Met His Glu Ser Met Lys Leu Phe Asp Ser Ile Cys Asn
   225                 230                 235                 240
   Asn Lys Trp Phe Thr Asp Thr Ser Ile Ile Leu Phe Leu Asn Lys Lys
                   245                 250                 255
   Asp Leu Phe Glu Glu Lys Ile Thr His Ser Pro Leu Thr Ile Cys Phe
               260                 265                 270
   Pro Glu Tyr Thr Gly Ala Asn Lys Tyr Asp Glu Ala Ala Ser Tyr Ile
           275                 280                 285
   Gln Ser Lys Phe Glu Asp Leu Asn Lys Arg Lys Asp Thr Lys Glu Ile
       290                 295                 300
   Tyr Thr His Phe Thr Cys Ala Thr Asp Thr Lys Asn Val Gln Phe Val
   305 310 315 320
   Phe Asp Ala Val Thr Asp Val Ile Ile Lys Asn Asn Leu Lys Asp Cys
   325 330 335
   Gly Leu Phe
<210> 91
   <211> 1020
   <212> DNA
   <213> Homo sapiens
<400> 91
   atgacggagg gtgtgaagac gctaggctgg acgaagcaga aaggcgggtg tcactgggga 60
   cgttctgagg gtgctgggga gtcagggaag agcaccatcg tcaagcagat gaagatcatc 120
   cacgaggatg gctactccga ggaggaatgc cggcagtacc gggcggttgt ctacagcaac 180
   accatccagt ccatcatggc cattgtcaaa gccatgggca acctgcagat cgactttgcc 240
   gacccctcca gagcggacga cgccaggcag ctatttgcac tgtcctgcac cgccgaggag 300
   caaggcgtgc tccctgatga cctgtccggc gtcatccgga ggctctgggc tgaccatggt 360
   gtgcaggcct gctttggccg ctcaagggaa taccagctca acgactcagc tgcctactac 420
   ctgaacgacc tggagcgtat tgcacagagt gactacatcc ccacacagca agatgtgcta 480
   cggacccgcg taaagaccac ggggatcgtg gagacacact tcaccttcaa ggacctacac 540
   ttcaagatgt ttgatgtggg tggtcagcgg tctgagcgga agaagtggat ccactgcttt 600
   gagggcgtca cagccatcat cttctgcgta gccttgagcg cctatgactt ggtgctagct 660
   gaggacgagg agatgaaccg catgcatgag agcatgaagc tattcgatag catctgcaac 720
   aacaagtggt tcacagacac gtccatcatc ctcttcctca acaagaagga cctgtttgag 780
   gagaagatca cacacagtcc cctgaccatc tgcttccctg agtacacagg ggccaacaaa 840
   tatgatgagg cagccagcta catccagagt aagtttgagg acctgaataa gcgcaaagac 900
   accaaggaga tctacacgca cttcacgtgc gccaccgaca ccaagaacgt gcagttcgtg 960
   tttgacgccg tcaccgatgt catcatcaag aacaacctga aggactgcgg cctcttctga 1020
<210> 92
   <211> 339
   <212> PRT
   <213> Homo sapiens
<400> 92
   Met Thr Glu Gly Val Lys Thr Leu Gly Trp Thr Lys Gln Lys Gly Gly
   1 5 10 15
   Cys His Trp Gly Arg Ser Glu Gly Ala Gly Glu Ser Gly Lys Ser Thr
               20                  25                  30
   Ile Val Lys Gln Met Lys Ile Ile His Glu Asp Gly Tyr Ser Glu Glu
           35                  40                  45
   Glu Cys Arg Gln Tyr Arg Ala Val Val Tyr Ser Asn Thr Ile Gln Ser
       50                  55                  60
   Ile Met Ala Ile Val Lys Ala Met Gly Asn Leu Gln Ile Asp Phe Ala
   65                  70                  75                  80
   Asp Pro Ser Arg Ala Asp Asp Ala Arg Gln Leu Phe Ala Leu Ser Cys
                   85                  90                  95
   Thr Ala Glu Glu Gln Gly Val Leu Pro Asp Asp Leu Ser Gly Val Ile
               100                 105                 110
   Arg Arg Leu Trp Ala Asp His Gly Val Gln Ala Cys Phe Gly Arg Ser
           115                 120                 125
   Arg Glu Tyr Gln Leu Asn Asp Ser Ala Ala Tyr Tyr Leu Asn Asp Leu
       130                 135                 140
   Glu Arg Ile Ala Gln Ser Asp Tyr Ile Pro Thr Gln Gln Asp Val Leu
   145                 150                 155                 160
   Arg Thr Arg Val Lys Thr Thr Gly Ile Val Glu Thr His Phe Thr Phe
                   165                 170                 175
   Lys Asp Leu His Phe Lys Met Phe Asp Val Gly Gly Gln Arg Ser Glu
               180                 185                 190
   Arg Lys Lys Trp Ile His Cys Phe Glu Gly Val Thr Ala Ile Ile Phe
           195                 200                 205
   Cys Val Ala Leu Ser Ala Tyr Asp Leu Val Leu Ala Glu Asp Glu Glu
       210                 215                 220
   Met Asn Arg Met His Glu Ser Met Lys Leu Phe Asp Ser Ile Cys Asn
   225                 230                 235                 240
   Asn Lys Trp Phe Thr Asp Thr Ser Ile Ile Leu Phe Leu Asn Lys Lys
                   245                 250                 255
   Asp Leu Phe Glu Glu Lys Ile Thr His Ser Pro Leu Thr Ile Cys Phe
               260                 265                 270
   Pro Glu Tyr Thr Gly Ala Asn Lys Tyr Asp Glu Ala Ala Ser Tyr Ile
   275 280 285
   Gln Ser Lys Phe Glu Asp Leu Asn Lys Arg Lys Asp Thr Lys Glu Ile
   290 295 300
   Tyr Thr His Phe Thr Cys Ala Thr Asp Thr Lys Asn Val Gln Phe Val
   305 310 315 320
   Phe Asp Ala Val Thr Asp Val Ile Ile Lys Asn Asn Leu Lys Asp Cys
   325 330 335
   Gly Leu Phe
<210> 93
   <211> 1065
   <212> DNA
   <213> Homo sapiens
<400> 93
   atgggctgca cgttgagcgc cgaagacaag gcggcagtgg agcgaagcaa gatgatcgac 60
   cgcaacttac gggaggacgg ggaaaaagcg gccaaagaag tgaagctgct gctactcggt 120
   gctggagaat ctggtaaaag caccattgtg aaacagatga aaatcattca tgaggatggc 180
   tattcagagg atgaatgtaa acaatataaa gtagttgtct acagcaatac tatacagtcc 240
   atcattgcaa tcataagagc catgggacgg ctaaagattg actttgggga agctgccagg 300
   gcagatgatg cccggcaatt atttgtttta gctggcagtg ctgaagaagg agtcatgact 360
   ccagaactag caggagtgat taaacggtta tggcgagatg gtggggtaca agcttgcttc 420
   agcagatcca gggaatatca gctcaatgat tctgcttcat attatctaaa tgatctggat 480
   agaatatccc agtctaacta cattccaact cagcaagatg ttcttcggac gagagtgaag 540
   accacaggca ttgtagaaac acatttcacc ttcaaagacc tatacttcaa gatgtttgat 600
   gtaggtggcc aaagatcaga acgaaaaaag tggattcact gttttgaggg agtgacagca 660
   attatcttct gtgtggccct cagtgattat gaccttgttc tggctgagga cgaggagatg 720
   aaccgaatgc atgaaagcat gaaactgttt gacagcattt gtaataacaa atggtttaca 780
   gaaacttcaa tcattctctt ccttaacaag aaagaccttt ttgaggaaaa aataaagagg 840
   agtccgttaa ctatctgtta tccagaatac acaggttcca atacatatga agaggcagct 900
   gcctatattc aatgccagtt tgaagatctg aacagaagaa aagataccaa ggagatctat 960
   actcacttca cctgtgccac agacacgaag aatgtgcagt ttgtttttga tgctgttaca 1020
   gatgtcatca ttaaaaacaa cttaaaggaa tgtggacttt attga 1065
<210> 94
   <211> 354
   <212> PRT
   <213> Homo sapiens
<400> 94
<210> 95
   <211> 1185
   <212> DNA
   <213> Homo sapiens
<400> 95
   atgggctgcc tcgggaacag taagaccgag gaccagcgca acgaggagaa ggcgcagcgt      60
   gaggccaaca aaaagatcga gaagcagctg cagaaggaca agcaggtcta ccgggccacg     120
   caccgcctgc tgctgctggg tgctggagaa tctggtaaaa gcaccattgt gaagcagatg     180
   aggatcctgc atgttaatgg gtttaatgga gagggcggcg aagaggaccc gcaggctgca     240
   aggagcaaca gcgatggtga gaaggcaacc aaagtgcagg acatcaaaaa caacctgaaa     300
   gaggcgattg aaaccattgt ggccgccatg agcaacctgg tgccccccgt ggagctggcc     360
   aaccccgaga accagttcag agtggactac atcctgagtg tgatgaacgt gcctgacttt     420
   gacttccctc ccgaattcta tgagcatgcc aaggctctgt gggaggatga aggagtgcgt     480
   gcctgctacg aacgctccaa cgagtaccag ctgattgact gtgcccagta cttcctggac     540
   aagatcgacg tgatcaagca ggctgactat gtgccgagcg atcaggacct gcttcgctgc     600
   cgtgtcctga cttctggaat ctttgagacc aagttccagg tggacaaagt caacttccac      660
   atgtttgacg tgggtggcca gcgcgatgaa cgccgcaagt ggatccagtg cttcaacgat      720
   gtgactgcca tcatcttcgt ggtggccagc agcagctaca acatggtcat ccgggaggac      780
   aaccagacca accgcctgca ggaggctctg aacctcttca agagcatctg gaacaacaga      840
   tggctgcgca ccatctctgt gatcctgttc ctcaacaagc aagatctgct cgctgagaaa      900
   gtccttgctg ggaaatcgaa gattgaggac tactttccag aatttgctcg ctacactact      960
   cctgaggatg ctactcccga gcccggagag gacccacgcg tgacccgggc caagtacttc     1020
   attcgagatg agtttctgag gatcagcact gccagtggag atgggcgtca ctactgctac     1080
   cctcatttca cctgcgctgt ggacactgag aacatccgcc gtgtgttcaa cgactgccgt     1140
   gacatcattc agcgcatgca ccttcgtcag tacgagctgc tctaa                     1185
<210> 96
   <211> 394
   <212> PRT
   <213> Homo sapiens
<400> 96
   Met Gly Cys Leu Gly Asn Ser Lys Thr Glu Asp Gln Arg Asn Glu Glu
   1               5                   10                  15
   Lys Ala Gln Arg Glu Ala Asn Lys Lys Ile Glu Lys Gln Leu Gln Lys
               20                  25                  30
   Asp Lys Gln Val Tyr Arg Ala Thr His Arg Leu Leu Leu Leu Gly Ala
           35                  40                  45
   Gly Glu Ser Gly Lys Ser Thr Ile Val Lys Gln Met Arg Ile Leu His
       50                  55                  60
   Val Asn Gly Phe Asn Gly Glu Gly Gly Glu Glu Asp Pro Gln Ala Ala
   65                  70                  75                  80
   Arg Ser Asn Ser Asp Gly Glu Lys Ala Thr Lys Val Gln Asp Ile Lys
                   85                  90                  95
   Asn Asn Leu Lys Glu Ala Ile Glu Thr Ile Val Ala Ala Met Ser Asn
               100                 105                 110
   Leu Val Pro Pro Val Glu Leu Ala Asn Pro Glu Asn Gln Phe Arg Val
           115                 120                 125
   Asp Tyr Ile Leu Ser Val Met Asn Val Pro Asp Phe Asp Phe Pro Pro
       130                 135                 140
   Glu Phe Tyr Glu His Ala Lys Ala Leu Trp Glu Asp Glu Gly Val Arg
   145                 150                 155                 160
   Ala Cys Tyr Glu Arg Ser Asn Glu Tyr Gln Leu Ile Asp Cys Ala Gln
                   165                 170                 175
   Tyr Phe Leu Asp Lys Ile Asp Val Ile Lys Gln Ala Asp Tyr Val Pro
               180                 185                 190
   Ser Asp Gln Asp Leu Leu Arg Cys Arg Val Leu Thr Ser Gly Ile Phe
           195                 200                 205
   Glu Thr Lys Phe Gln Val Asp Lys Val Asn Phe His Met Phe Asp Val
       210                 215                 220
   Gly Gly Gln Arg Asp Glu Arg Arg Lys Trp Ile Gln Cys Phe Asn Asp
   225                 230                 235                 240
   Val Thr Ala Ile Ile Phe Val Val Ala Ser Ser Ser Tyr Asn Met Val
                   245                 250                 255
   Ile Arg Glu Asp Asn Gln Thr Asn Arg Leu Gln Glu Ala Leu Asn Leu
               260                 265                 270
   Phe Lys Ser Ile Trp Asn Asn Arg Trp Leu Arg Thr Ile Ser Val Ile
           275                 280                 285
   Leu Phe Leu Asn Lys Gln Asp Leu Leu Ala Glu Lys Val Leu Ala Gly
       290                 295                 300
   Lys Ser Lys Ile Glu Asp Tyr Phe Pro Glu Phe Ala Arg Tyr Thr Thr
   305                 310                 315                 320
   Pro Glu Asp Ala Thr Pro Glu Pro Gly Glu Asp Pro Arg Val Thr Arg
                   325                 330                 335
   Ala Lys Tyr Phe Ile Arg Asp Glu Phe Leu Arg Ile Ser Thr Ala Ser
               340                 345                 350
   Gly Asp Gly Arg His Tyr Cys Tyr Pro His Phe Thr Cys Ala Val Asp
           355                 360                 365
   Thr Glu Asn Ile Arg Arg Val Phe Asn Asp Cys Arg Asp Ile Ile Gln
       370                 375                 380
   Arg Met His Leu Arg Gln Tyr Glu Leu Leu
   385                 390
<210> 97
   <211> 18
   <212> DNA
   <213> ARTIFICIAL
<220>
   <223> Synthetic Construct
<400> 97
   gagagggaaa gaagggga 18
<210> 98
   <211> 23
   <212> DNA
   <213> ARTIFICIAL
<220>
   <223> Synthetic Construct
<400> 98
   gaggaccgaa aagcgcaaag aca 23
<210> 99
   <211> 15
   <212> DNA
   <213> ARTIFICIAL
<220>
   <223> Synthetic Construct
<400> 99
   cagccaagac gccaa 15
<210> 100
   <211> 22
   <212> DNA
   <213> ARTIFICIAL
<220>
   <223> Synthetic Construct
<400> 100
   acaacaggca ccgggaggac aa 22

## Description

### CROSS REFERENCE TO RELATED APPLICATIONS

This application is a PCT application Serial No PCT/CA2014/* filed on June 16, 2014 and published in English under PCT Article 21(2), which itself claims benefit of U.S. provisional application Serial No. 61/835,839, filed on June 17, 2013.

### FIELD OF THE INVENTION

The present invention relates to markers of scoliosis and scoliosis progression. More particularly, it relates to Gi protein phosphorylation as marker for scoliosis and scoliosis progression and uses thereof to stratify scoliotic patients and predict the risk of developing scoliosis.

### REFERENCE TO SEQUENCE LISTING

Pursuant to 37 C.F.R. 1.821(c), a sequence listing is submitted herewith as an ASCII compliant text file named 14033_122_ST25.txt, that was created on June 16, 2014 and having a size of 57 kilobytes.

### BACKGROUND OF THE INVENTION

Idiopathic Scoliosis is a spine deformity of unknown cause generally defined as a lateral curvature greater than 10 degrees accompanied by a vertebral rotation ¹. Adolescent Idiopathic Scoliosis (AIS) is one of the most frequent childhood deformities worldwide, characterized by a 3D spinal deformity with unknown cause, and represents both an immediate medical challenge and a chronic condition affecting individuals throughout their lives. It is the most common orthopedic condition requiring surgery in adolescents and affects 4% of this population. This condition is most commonly diagnosed between the ages of 9 to 13 years ^{2,3,4}. The diagnosis is primarily of exclusion and is made only after ruling out other causes of spinal deformity such as vertebral malformation, neuromuscular or syndromic disorders. Traditionally, the trunkal asymmetry is revealed by Adams forward bending test and measured with scoliometer during physical examination ⁵. The diagnosis can then be confirmed by radiographic observation of the curve and the angle measurement using the Cobb method ⁶.

Once diagnosed, the primary concern for physicians in managing scoliotic children is whether the curve will progress. Indeed, the curve progression is often unpredictable and is more frequently observed among girls than in boys⁷. If untreated, the curve can progress dramatically, creating significant physical deformity and even cardiopulmonary problems. These manifestations become life threatening when the curve exceeds 70 degrees ^{8,9}. The current treatment options to prevent or stop curve progression include bracing and surgery. In general, bracing is recommended for curves between 25 and 40 degrees, while surgery is reserved for curves greater than 45 degrees or curves that are unresponsive to bracing. Today in the United States there are approximately one million children between ages 10 and 16 with some degree of idiopathic scoliosis (IS). Approximately 10% of children diagnosed with idiopathic scoliosis have curve progression requiring corrective surgery ¹⁰. About 29,000 scoliosis surgeries are done every year in North America, resulting in significant psychological and physical morbidity. (Goldberg MS, Mayo NE, Poitras B et al. The Ste-Justine Adolescent Idiopathic Scoliosis Cohort Study. Part I: Description of the study. Spine 1994;19:1551-61; Poitras B, Mayo NE, Goldberg MS et al. The Ste-Justine Adolescent Idiopathic Scoliosis Cohort Study. Part IV: Surgical correction and back pain. Spine 1994;19:1582-8).

Currently, there is no proven method or test available to identify subjects at risk of developing IS to predict which affected individuals require treatment to prevent or stop progression of the disease so that appropriate treatment can be early provided and prevent surgical complications and cardiac and/or respiratory problems. (Weinstein SL, Dolan LA, Cheng JC et al. Adolescent idiopathic scoliosis. Lancet 2008;371:1527-37).

Therefore, the application of current treatments, such as bracing or surgical correction, is delayed until a significant deformity is detected or until a significant progression is clearly demonstrated, resulting in a delayed, less than optimal treatment and often important psychological sequels (Society SR. Morbidity & Mortality Committee annual Report 1997).

Currently, in order to detect the deformity, diagnosed children are subjected to multiple radiographs over several years, usually until they reach skeletal maturity. It is estimated that the typical patients with scoliosis will have approximately 22 radiological examinations over a 3-year period ¹¹. There are potential risks in multiple radiographic examinations. For this reason also, alternative approaches that could allow performing the prognosis of idiopathic scoliosis are strongly desirable.

The major limitation in developing prognostic tests that could facilitate treatment choices for patients is the heterogeneous nature of AIS. At the clinical level, the heterogeneity of AIS is clearly illustrated by the variability of curve patterns, localisations and curve magnitude even in families with multiple affected members.

In absence of reliable AIS phenotypes, there is a need to understand better the molecular changes associated with disease onset and spinal deformity progression. Molecular definition of disease is rapidly replacing traditional pathology-based disease descriptions in part because of its utility in identifying the optimal treatment regimen for patients.

To this effect, the existence of a differential melatonin signaling dysfunction was reported among AIS patients leading to their stratification into three functional groups or biological endophenotypes (Moreau et al., 2004); (Azeddine et al., 2007); (Letellier et al., 2008) and WO2003/073102 to Moreau. More particularly, AIS patients were stratified into three functional groups (FG1, FG2 and FG3) representing distinct biological endophenotypes. With this approach, the scoliotic patients and children more at risk of developing scoliosis are less responsive to Gi protein stimulation when compared with healthy control subjects, and the classification is based on the percentage of degree of reduction relative to control group. The classification ranges were fixed between 10 and 40% for FG3, 40 and 60% for FG2 and 60 and 90% for FG1.

More recently, using the cellular dielectric spectrometry (CDS) technique, which is a label-free method for the functional evaluation of G proteins and endogenous receptors coupled to those proteins (Verdonk et al., 2006), it was found that the cellular response following melatonin receptor stimulation by melatonin was mainly Gi-dependent in normal osteoblasts and was reduced to different extents in osteoblasts derived from AIS patients (Akoume et al., 2010). Approximately 33% of asymptomatic children diagnosed with a defective Gi protein function have developed scoliosis many years later (Akoume et al., 2010).

Melatonin signalling dysfunction in AIS was described by Moreau et al. (Spine, Lippincott Williams & Wilkens, 2004) who teaches that melatonin signalling is differentially impaired in osteoblasts of AIS patients allowing their classification into 3 distinct groups based on cAMP production inhibition in response to melatonin or Gpp(NH)p. Moreau et al. discloses a general increase in phosphorylated Giα proteins coupled to MT2 receptors in AIS subjects as compared to control cells but does not disclose or suggest any specific differences in the phosphorylation pattern of Giα1, Giα2 and/or Giα3 isoforms between control or AIS or between AIS subjects.

Bagnall et al. (Scoliosis 2009) indicates that previous studies have led to the classification of AIS patients into 3 different functional groups depending on their response to melatonin and that molecular analysis has shown that melatonin signalling dysfunction is triggered by an increase in phosphorylation of Gi proteins inactivating their function. No additional details are provided concerning the use of these date for prognosis purpose.

Early detection/prognosis of scoliosis is not only critical to successful and less invasive clinical outcomes but broadens the range of treatment options for clinicians. Indeed, improving patients' stratification and disease staging represent key steps to select AIS patients for minimally invasive surgeries before their spinal deformity is too advanced.

### SUMMARY OF THE INVENTION

The present invention provides the clinical evidence that a differential disruption of Gi alpha subunits occurs in AIS and demonstrate that such impairment is caused by a serine phosphorylation of distinct Gi isoforms leading to the classification of AIS patients into three biological endophenotypes representing inheritable traits. Heritability was clearly demonstrated with the detection of the same endophenotype in all family members affected by scoliosis. Evaluation of the clinical outcomes of AIS patients according to their biological endophenotypes reveals in two distinct cohorts (Canadians and Italians) that AIS patients classified in FG2 endophenotype are more susceptible to developing severe scoliosis, while those in FG1 endophenotype present a much lower risk of disease progression.

In mechanistic *in vitro* studies, it was further observed that hypofunctionality of Gi proteins occurring in AIS led to (a) a systemic and generalized signaling defect perturbing all Gi-coupled receptors differentially in different cell types, (b) an enhancement of Gs-coupled receptor signaling while Gq-coupled receptor signal transduction was not compromised, and (c) pharmacological inhibition of selected kinases rescued or improved Gi-signaling impairment at the cellular level and such response varied in function of each biological endophenotype. The signaling defect is due to selective phosphorylation of Gi alpha subunit isoforms in each group involving distinct kinases, namely: FG3 has a Giα1 without phosphorylated serines and serine-phosphorylated Gia2 and Gia3; FG2 has a Gia3 without phosphorylated serines and serine-phosphorylated Gia1 and Gia2; and FG1 has serine-phosphorylated Gia1, Gia2 and Gia3.

These findings provide the evidence that a differential disruption of Gi-signaling underscores AIS pathogenesis and represents a rational basis for the development of innovative prognostic tools and pharmacological therapies.

More specifically, in accordance with the present invention, there is provided an *in vitro* method of stratifying a subject having adolescent idiopathic scoliosis (AIS) comprising: (i) providing a cell sample isolated from the subject; and (ii) (a) determining the serine phosphorylation of Giα1 in the cell sample; and/or (b) determining the serine phosphorylation of Giα3 in the cell sample or according to the description (c) determining the difference (Δ) between responses to Giα and Gsα protein stimulation in the cell sample; or (d) any combination of (a) to (c);whereby the results of the detecting step enables the stratification of the subject having AIS as belonging to an AIS subclass. The *in vitro* method of the invention also comprises (iii) stratifying said subject into a clinically useful AIS subclass based on the subject's Giα1 or Giα3 protein serine phosphorylation profile

In accordance with another aspect of the present invention, there is provided an *in vitro* method for predicting the risk for developing a severe scoliosis in a subject comprising: (i) providing a cell sample isolated from the subject; (ii) detecting or determining the serine phosphorylation of Giα3 protein in the cell sample; (iii) determining that the subject is at risk for developing a severe scoliosis when serine-phosphorylated Giα3 protein in the cell sample is not detected, or is not elevated as compared to levels corresponding to those of a control. According to the present description, this method may comprises a step of (b) determining the difference (Δ) between responses to Giα and Gsα protein stimulation in the cell sample; and/or (c) determining the GiPCR response to an agonist in the cell sample, wherein (a) an absence of serine phosphorylation in Giα3 protein; (b) a determination that the Giα/Gsα ratio is between about 0.5 and 1.5; and/or (c) a GiPCR response in the cell sample lower than that in a control sample by about 40 to 60%, is indicative that the subject is at risk for developing a severe scoliosis.

In a specific embodiment, the subject is a subject diagnosed with a scoliosis.

The subject is likely to develop a scoliosis. In another specific embodiment, the scoliosis is adolescent idiopathic scoliosis. In another specific embodiment, method of is *in vitro.* In another specific embodiment, said cell sample comprises osteoblasts, myoblasts and/or peripheral blood mononuclear cells (PBMC). In another specific embodiment, said cell sample comprises PBMCs. In another specific embodiment, said cell comprises lymphocytes.

There is also described a method of increasing GiPCR signaling in cells of a subject in need thereof (e.g., diagnosed with a scoliosis or a likely to develop scoliosis) comprising administering to the subject an effective amount of: (i) an inhibitor of PKA; or (ii) if the subject's cells have serine phosphorylated Gαi1, Gαi2 and Gαi3 proteins, an inhibitor of (a) PKC; (b) CaMK1 or 4; (c) CK; or (d) any combination of at least two of (a) to (c); (iii) if the subjects cells have an absence of serine phosphorylation on Gai3, an inhibitor of CaMK2; or (iv)if the subjects cells have an absence of serine phosphorylation on Gαi1, an inhibitor of CK, whereby the GiPCR signaling is increased in the subject's cells.

Moreover, there is described a use of (i) an inhibitor of PKA; or (ii) if the subject's cells have serine phosphorylated Gαi1, Gai2 and Gai3 proteins, an inhibitor of (a) PKC; (b) CaMK1 or 4; (c) CK; or (d) any combination of at least two of (a) to (c); (iii) if the subjects cells have an absence of serine phosphorylation on Gai3, an inhibitor of CaMK2; or (iv) if the subjects cells have an absence of serine phosphorylation on Gαi1, an inhibitor of CK, for increasing GiPCR signaling in cells of a subject in need thereof (e.g., diagnosed with a scoliosis or a likely to develop scoliosis) or for manufacturing a medicament for increasing GiPCR signaling in cells of a subject in need thereof (e.g., diagnosed with a scoliosis or a likely to develop scoliosis).

The present description also relates to a composition for use in increasing GiPCR signaling in cells of a subject in need thereof (e.g., diagnosed with a scoliosis or a likely to develop scoliosis) comprising: (i) an inhibitor of PKA; or (ii) if the subject's cells have serine phosphorylated Gαi1, Gai2 and Gai3 proteins, an inhibitor of (a) PKC; (b) CaMK1 or 4; (c) CK; or (d) any combination of at least two of (a) to (c); (iii) if the subjects cells have an absence of serine phosphorylation on Gai3, an inhibitor of CaMK2; or (iv) if the subjects cells have an absence of serine phosphorylation on Gαi1, an inhibitor of CK.

Another composition described herein may comprise : (i) an inhibitor of PKA; or (ii) if the subject's cells have serine phosphorylated Gαi1, Gai2 and Gai3 proteins, an inhibitor of (a) PKC; (b) CaMK1 or 4; (c) CK; or (d) any combination of at least two of (a) to (c); (iii) if the subjects cells have an absence of serine phosphorylation on Gai3, an inhibitor of CaMK2; or (iv) if the subjects cells have an absence of serine phosphorylation on Gαi1, an inhibitor of CK. In specific embodiments of the present invention, the compositions further comprise a pharmaceutically acceptable carrier.

A kit for stratifying a subject having adolescent idiopathic scoliosis (AIS) comprising: (a) a ligand for detecting an absence of serine phosphorylation on Gia1 in the cell sample; (b) a ligand for detecting an absence of serine phosphorylation on Gia3 in the cell sample; (c) ligands for detecting Gia and Gsa; or (d) any combination of (a) to (c) is also described herein.

Furthermore, a kit for predicting the risk for developing a severe scoliosis in a subject comprising: (a) a ligand for detecting an absence of serine phosphorylation on Gia3; (b) ligands for detecting Gia and Gsa; or (c) a combination of (a) and (b) is described herein.

Herein is also described a kit for increasing GiPCR signaling in cells of a subject in need thereof comprising: (a) an inhibitor of PKA; (b) an inhibitor of PKC; (c) an inhibitor of CaMK1 or 4; (d) an inhibitor of CK ; (e) an inhibitor of CaMK2; or (f) any combination of at least two of (a) and (e).

In the present description, the inhibitor of PKA is an inhibitor of PKA-y2. In another specific embodiment, the inhibitor of PKA is H89. The inhibitor of PKC may be an inhibitor of PKC-η or PKC-ε or. The inhibitor of CaMK1 is CaMk1δ. The inhibitor of CaMK1 or CaMK4 is also STO609. The inhibitor of CK is an inhibitor of CK2 or is D4476. The inhibitor of CaMK2 is STO609 or KN93. According to the description, the subject in need thereof is a subject diagnosed with a scoliosis or likely to develop a scoliosis., The scoliosis is adolescent idiopathic scoliosis.

There is also described a method of selecting an agent as a potential candidate for the reduction or prevention of scoliosis comprising contacting a candidate agent with a cell expressing (a) PKA; (b) PKC, (c) CaMK1, (d) CaMK4; (e) CK; (f) CaMK2; wherein when the expression or activity of any one of (a) to (f) is decreased, the candidate agent is selected.

As described herein, PKA is PKA-γ2,PKC is PKC-η or PKC-ε., CaMK1 is CaMK1δ and CK is a CK2.

In some aspects, the present invention relates to a method of stratifying a subject having or at risk for developing adolescent idiopathic scoliosis (AIS), the method comprising: (i) providing a cell sample isolated from the subject; (ii) detecting or determining from the cell sample the subject's Giα1 and/or Giα3 protein serine phosphorylation in the cell sample; and (iii) stratifying the subject into a clinically useful AIS subclass based on the subject's Giα1 or Giα3 protein serine phosphorylation profile .

In some embodiments, the present above mentioned method comprises: (a) detecting or determining the serine phosphorylation of Giα1 in the cell sample; and/or (b) detecting or determining the serine phosphorylation of Giα3 in the cell sample.

In some embodiments, the above mentioned method further comprises stratifying the subject as belonging to: (1) a first AIS subclass characterized by elevated levels of serine-phosphorylated Gia1 and Gia3 proteins as compared to levels corresponding to those of a control; (2) a second AIS subclass characterized by elevated levels of serine-phosphorylated Gia1 but not of serine-phosphorylated Gia3 protein, as compared to levels corresponding to that of a control; or (3) a third AIS subclass characterized by elevated levels of serine-phosphorylated Gia3 protein but not of serine-phosphorylated Gia1 protein as compared to levels corresponding to those of a control;. According to the present description, a first AIS subclass may be characterized by a Gia/Gsa response ratio below about 0.5, a second AIS subclass may be characterized by a Gia/Gsa response ratio between about 0.5 and 1.5; or a third AIS subclass characterized by a Gia/Gsa response ratio above about 1.5.

In some embodiments, the above mentioned method further comprises detecting or determining the serine phosphorylation of Giα2 protein in the cell sample, wherein elevated levels of serine-phosphorylated Giα2 protein are detected, as compared to levels corresponding to that of a control.

In some embodiments of the above mentioned methods, (1) subjects belonging to the first AIS subclass have a low risk of severe AIS progression; (2) subjects belonging to the second AIS subclass have a high risk for severe AIS progression; and (3) subjects belonging to the third AIS subclass have a moderate risk for severe AIS progression.

In some aspects, the present invention relates to an *in vitro* method for predicting the risk for developing a severe scoliosis in a subject having or at risk for developing scoliosis, the method comprising: (i) providing a cell sample isolated from the subject; (ii) detecting or determining the serine phosphorylation of Giα3 protein in the cell sample; ((iii) determining that the subject is at risk for developing a severe scoliosis when serine-phosphorylated Gia3 protein in the cell sample is not detected, or is not elevated as compared to levels corresponding to those of a control.lt is also described herein an *in vitro* method of predicting the risk for developing a severe scoliosis in a subject having or at risk for developing scoliosis, wherein in step (ii) is detecting or determining the responses to Gia and Gsa proteins stimulation in the cell sample; and/or (c) the GiPCR response to an agonist in the cell sample and in step (iii) is determining that the subject is at risk for developing a severe scoliosis when a ratio of the response to Gia protein stimulation to the response to Gsa protein stimulation (Giα/Gsα response ratio) in the cell sample is between about 0.5 and 1.5; and/or (c) a GiPCR response in the cell sample lower than that in a control sample by about 40 to 60% is detected.

In some embodiments of the above mentioned methods, step (ii) further comprises detecting or determining the serine phosphorylation of Giα1 and/or Giα2 protein in the cell sample, wherein the subject is at risk for developing a severe scoliosis when the level of the subject's serine-phosphorylated Giα1 or Giα2 protein is elevated as compared to levels corresponding to those of a control.

The present description further concerns a method for predicting the responsiveness of a subject having scoliosis to bracing treatment, the method comprising: (i) providing a cell sample isolated from the subject; and (ii) detecting or determining: (a) the serine phosphorylation of Gia1 in the cell sample; (b) the serine phosphorylation of Gia3 in the cell sample; (c) responses to Gia and Gsa proteins stimulation in the cell sample; or (d) any combination of (a) to (c); (iii) determining that the subject is likely to be responsive to bracing treatment when: (a) elevated levels of serine-phosphorylated Gia3 protein but not of serine-phosphorylated Gia1 protein is detected, as compared to levels corresponding to those of a control; and/or (b) a ratio of the response to Gia protein stimulation to the response to Gsa protein stimulation (Gia/Gsa response ratio) in the cell sample is above about 1.5.

In some embodiments, the above mentioned subject is a subject diagnosed with a scoliosis. In some embodiments, the scoliosis is adolescent idiopathic scoliosis (AIS).

In some embodiments, the above-mentioned cell sample comprises osteoblasts, myoblasts and/or peripheral blood mononuclear cells (PBMC). In some embodiments, the above mentioned cell sample comprises PBMCs. In some embodiments, the above mentioned PBMCs comprise lymphocytes.

In some embodiments, the above mentioned the detecting or determining the serine phosphorylation of Gia1 and/or Gia3 protein(s) in the cell sample comprises isolating Gia1 and/or Gia3 protein(s) from the cell sample and contacting the isolated Gia1 and/or Gia3 protein(s) with an anti-phosphoserine antibody.

It is also described a method of increasing GiPCR signaling in cells of a subject having or at risk for developing scoliosis, the method comprising administering to the subject an effective amount of: (i) an inhibitor of PKA; (ii) an inhibitor of (a) PKC; (b) CaMK1 or 4; (c) CK; or (d) any combination of at least two of (a) to (c), if the subject's cells have elevated levels of serine phosphorylated Gαi1, Gai2 and Gai3 proteins as compared to levels corresponding to those of a control; (iii) an inhibitor of CaMK2, if the subject's cells have levels of serine phosphorylated Gai3 protein that are not elevated as compared to levels corresponding to those of a control; or (iv) an inhibitor of CK, if the subject's cells have levels of serine phosphorylated Gαi1 protein that are not elevated as compared to levels corresponding to those of a control, whereby the GiPCR signaling is increased in the subject's cells.

The above mentioned inhibitor of PKA is an inhibitor of PKA-γ2 or is H89. The above mentioned inhibitor of PKC is an inhibitor of PKC-η or PKC-ε or is Gö6983. The above mentioned inhibitor of CaMK1 is CaMk1δ. The above mentioned inhibitor of CaMK1 or CaMK4 is STO609. The above mentioned inhibitor of CK is an inhibitor of CK2or is D4476. The above mentioned inhibitor of CaMK2 is STO609 or KN93.

In some embodiments, the above mentioned subject in need thereof is a subject diagnosed with a scoliosis. In some embodiments, the above mentioned subject in need thereof is likely to develop a scoliosis. In some embodiments, the above mentioned scoliosis is adolescent idiopathic scoliosis (AIS).

It is also described herein the use of an inhibitor as defined above for increasing GiPCR signaling, or in the preparation of a medicament for increasing GiPCR signaling, in cells of a subject having or at risk for developing scoliosis.

The above mentioned subject in need thereof is a subject diagnosed with a scoliosis or is likely to develop a scoliosis. The above mentioned the scoliosis is adolescent idiopathic scoliosis (AIS).
a kit for stratifying a subject having or at risk for developing adolescent idiopathic scoliosis (AIS) comprising: (a) a ligand for detecting the level of serine-phosphorylated Gia1 protein in a cell sample from the subject; (b) a ligand for detecting the level of serine-phosphorylated Giα3 protein in a cell sample from the subject; (c) ligands for detecting responses to Giα and Gsα proteins stimulation in the cell sample; or (d) any combination of (a) to (c) is also described herein.

The above mentioned kit further comprises a ligand for detecting the level of serine-phosphorylated Giα2 protein.

Furthermore, it is described a kit for predicting the risk for developing a severe scoliosis in a subject having or at risk for developing scoliosis, the kit comprising: (a) a ligand for detecting the level of serine-phosphorylated Giα3 protein in a cell sample from the subject; (b) ligands for detecting responses to Giα and Gsα proteins stimulation in a cell sample from the subject; or (c) a combination of (a) and (b).

A kit for increasing GiPCR signaling in cells of a subject in need thereof, the kit comprising: (a) an inhibitor of PKA; (b) an inhibitor of PKC; (c) an inhibitor of CaMK1 or 4; (d) an inhibitor of CK; (e) an inhibitor of CaMK2; or (f) any combination of at least two of (a) to (e) is also described.

The above mentioned inhibitor of PKA is an inhibitor of PKA-γ2 or is H89. The above mentioned inhibitor of PKC is an inhibitor of PKC-η or PKC-εor is Gö6983. The above mentioned inhibitor of CaMK1 is CaMk1δ. The above mentioned inhibitor of CaMK1 or CaMK4 is STO609. The above mentioned inhibitor of CK is an inhibitor of CK2 or is D4476. The above mentioned inhibitor of CaMK2 is STO609 or KN93.

It is also described herein a method of selecting an agent as a potential candidate for the treatment or prevention of scoliosis, the method comprising contacting a candidate agent with a cell expressing: (a) PKA; (b) PKC; (c) CaMK1; (d) CaMK4; (e) CK; or (f) CaMK2; and selecting the candidate agent when the expression or activity of any one of (a) to (f) is decreased. In some embodiments, the above mentioned PKA is PKA-y2. In some embodiments, the above mentioned PKC is PKC-η or PKC-ε. In some embodiments, the above mentioned CaMK1 is CaMK1δ. In some embodiments, the above mentioned CK is a CK2.

Other objects, advantages and features of the present invention will become more apparent upon reading of the following non-restrictive description of specific embodiments thereof, given by way of example only with reference to the accompanying drawings.

### BRIEF DESCRIPTION OF THE DRAWINGS

In the appended drawings:

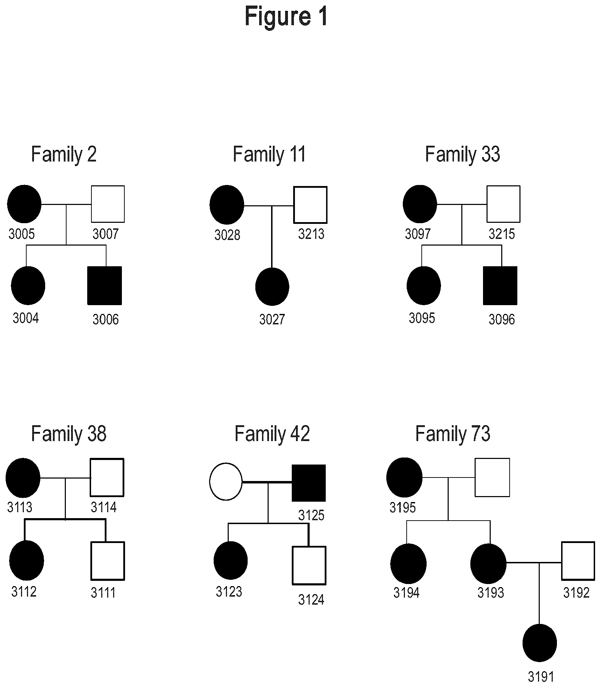

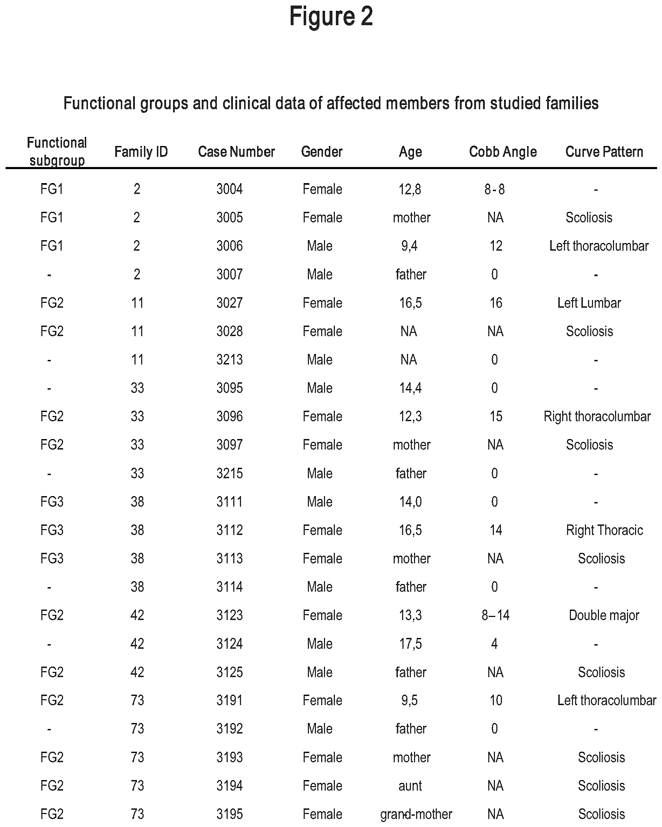

**Figure 1** **shows representative pedigrees of families with a positive history of AIS.** The circles represent females and the squares represent males. Filled symbols indicate affected individuals and the indicated numbers correspond to the individuals (case numbers) listed in **Figure 2****.**
**Figure 2** presents AIS subjects functional groups and clinical data of affected members from studied families.

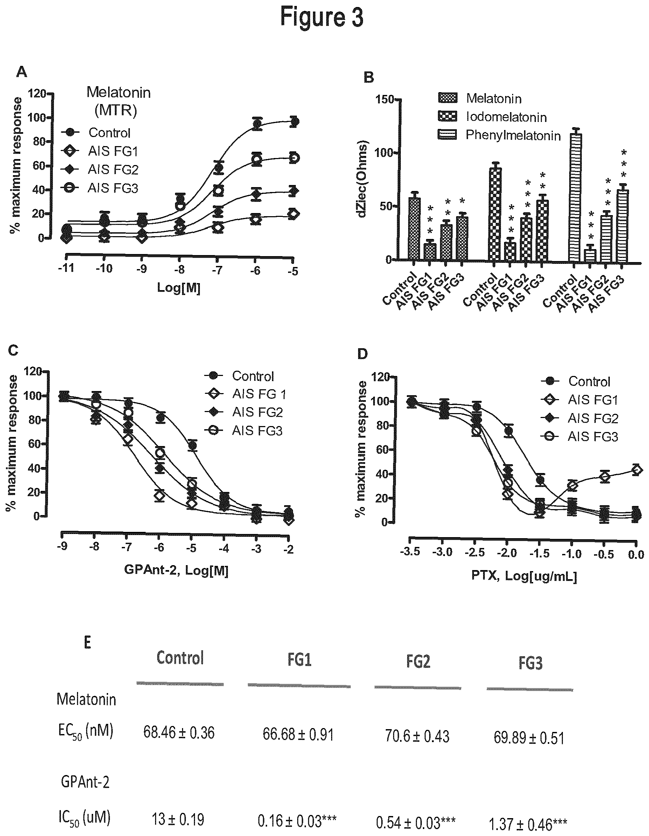

**Figure 3** **shows the functionality of melatonin receptor is not impaired in AIS. (A)** Comparison of concentration-response curves for melatonin between control and AIS functional groups. Data were normalised to maximal response in cells from control subjects. **(B)** Comparison of response to melatonin and its analogues. Cells were stimulated with the same concentration (1 µM) of melatonin, iodomelatonin or phenylmelatonin. The impedance represented in y-axis as dZiec, indicates the resistance (ohms) of the cells toward the electric current applied by the Cellkey™ apparatus and represents the integrated cellular response. **(C, D)** Inhibition curves for response to melatonin following a treatment of **(C)** 4h with G-Protein antagonist peptide (GPAnt-2) and **(D)** 16 hours with pertussis toxin. **(E)** EC₅₀ and IC₅₀ values of melatonin and GPAnt-2 in each functional group. Data are expressed as mean (± SE) of n = 12 patients per group. * P < 0.05, **P < 0.01, ***P < 0.001, vs control group.

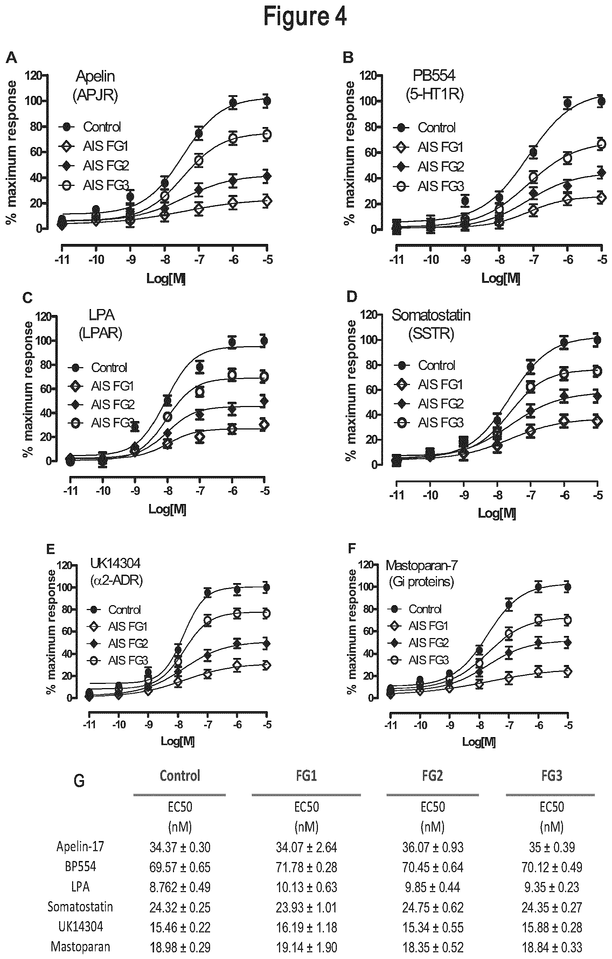

**Figure 4** **shows the AIS functional groups are distinguished by the degree of response to various specific agonists of Gi-coupled receptors in osteoblasts. (A, B, C, D, E, and F)** Agonists and targeted receptors are indicated in left corner of each panel. Data were normalised to maximal response in cells from control subjects. **(G)** EC₅₀ values of tested compounds in each functional group. Data are expressed as mean (± SE) of n = 12 patients per group. * P < 0.05, **P < 0.01, ***P < 0.001, vs control group.

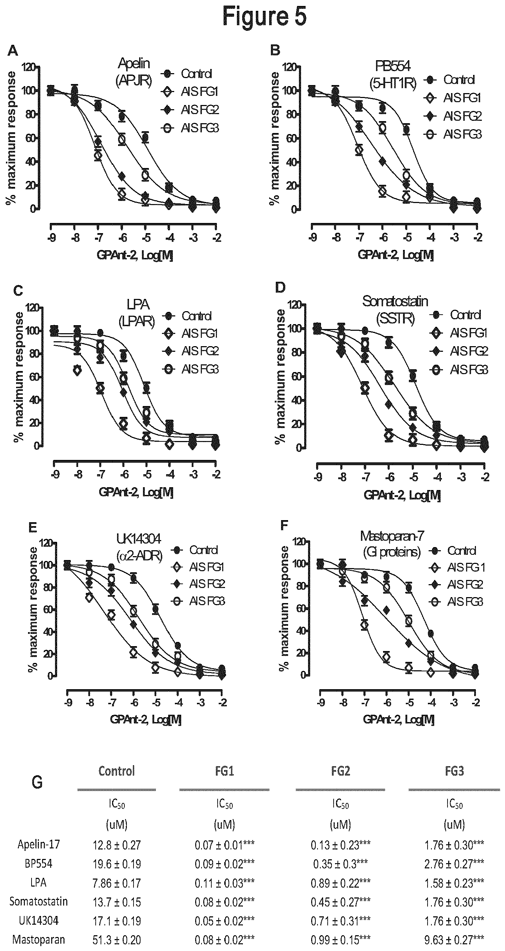

**Figure 5** **shows inhibition curves of GPAnt-2 on response to various selective agonists of Gi-coupled receptors. (A, B, C, D, E, and F)** Osteoblasts from control subjects or AIS patients of different groups were pre-incubated with varying concentrations of GPAnt-2 for 4h prior stimulation with 1 µM of specific synthetic agonist. The tested agonists and targeted receptors are indicated in left corner of each panel. Data were normalised to maximal response in cells from control subjects. **(G)** Table of IC₅₀ values GPAnt-2 for each tested compound in each functional group. Data are expressed as mean (± SE) of n = 12 patients per group. * P < 0.05, **P < 0.01, ***P < 0.001, vs control group.

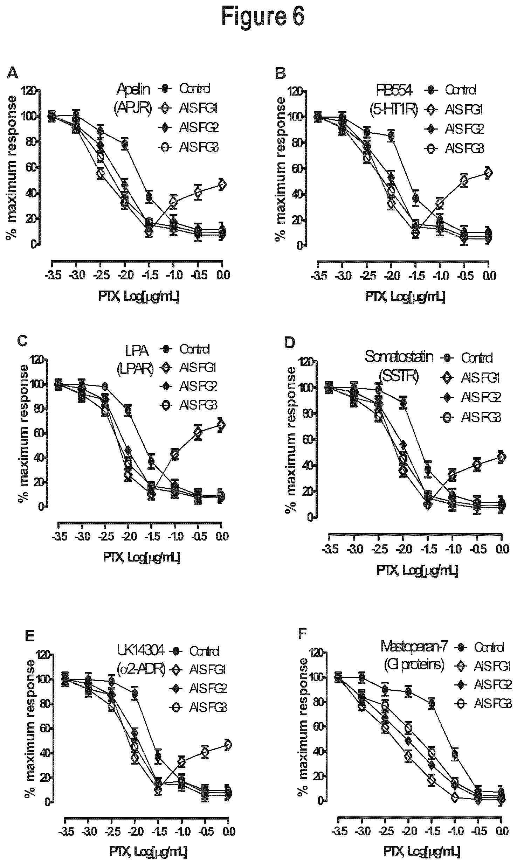

**Figure 6** **shows inhibition curves of PTX on response to various selective agonists of Gi-coupled receptors. (A, B, C, D, E, and F)** Osteoblasts from control subjects or AIS patients of different groups were pre-incubated with varying concentrations of PTX for 16h prior to stimulation with 1 µM of specific synthetic agonist. The tested agonists and targeted receptors are indicated in left corner of each panel. Data were normalised to maximal response in cells from control subjects. Data are expressed as mean ± SE of n = 12 patients per group. * P < 0.05, **P < 0.01, ***P < 0.001, vs control group.

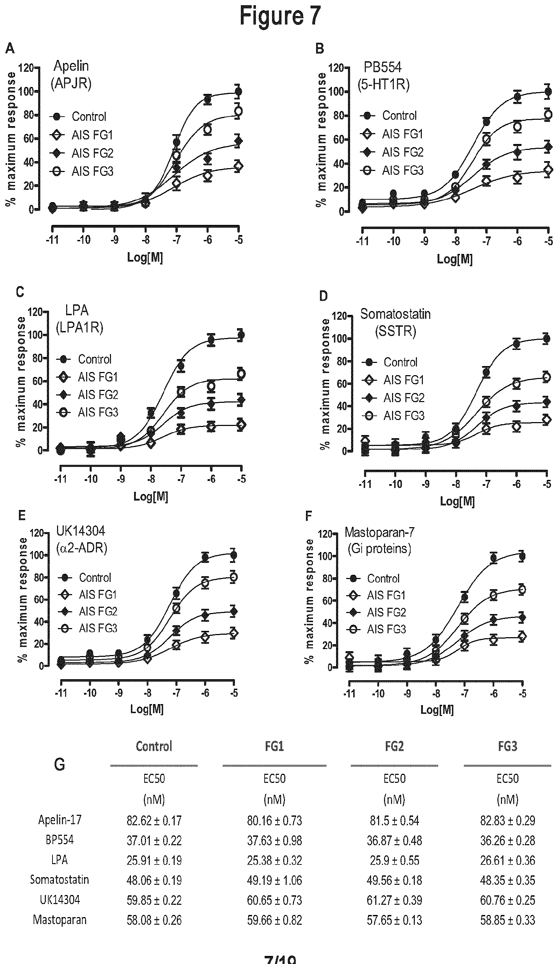

**Figure 7** **shows the AIS functional groups are distinguished by the degree of response to various specific agonists of Gi-coupled receptors in myoblasts. (A, B, C, D, E, and F)** Agonists and targeted receptors are indicated in left corner of each panel. Data were normalised to maximal response in cells from control subjects. **(G)** EC₅₀ values of tested compounds in each functional group. Data are expressed as mean (± SE) of n = 12 patients per group. * P < 0.05, **P < 0.01, ***P < 0.001, vs control group.

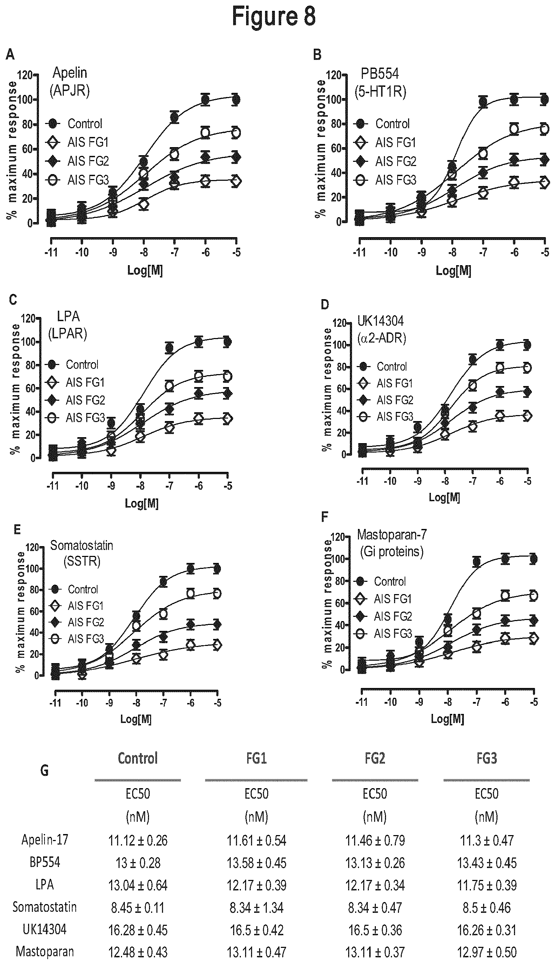

**Figure 8** **shows the AIS functional groups are distinguished by the degree of response to various specific agonists of Gi-coupled receptors in PBMCs. (A, B, C, D, E, and F)** Agonists and targeted receptors are indicated in left corner of each panel. Data were normalised to maximal response in cells from control subjects. **(G)** EC₅₀ values of tested compounds in each functional group. Data are expressed as mean (± SE) of n = 12 patients per group. * P < 0.05, **P < 0.01, ***P < 0.001, vs. control group.

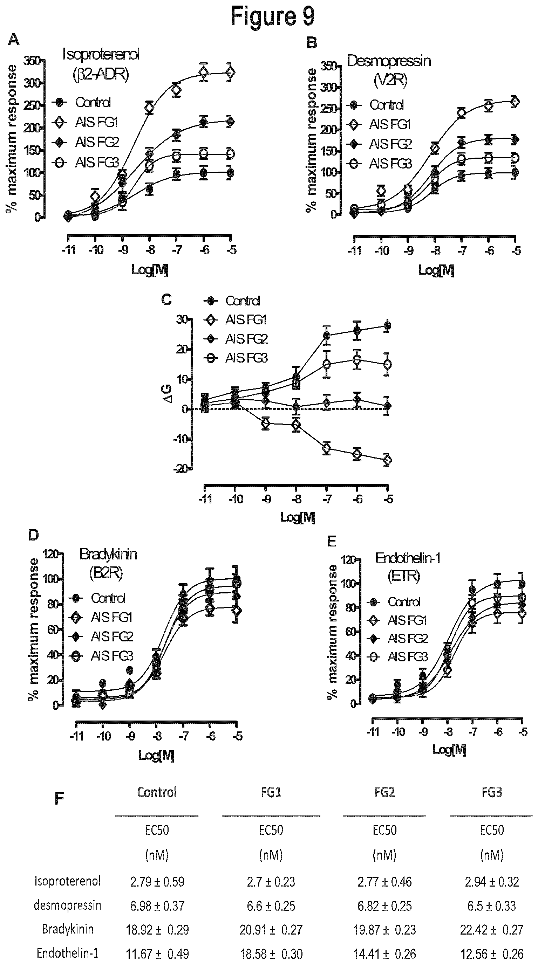

**Figure 9** **shows the functional status of Gs and Gq proteins in osteoblasts from control and AIS functional groups.** The functionality of Gs protein was evaluated by challenging cells with **(A)** Isoproterenol; or **(B)** Desmopressin. **(C)** The difference between response to Gi and Gs stimulation was calculated at various concentrations, and the functionality of Gq was assessed by challenging cells with Bradykinin **(D)** or Endothelin-1 **(E).** Receptor subtype targeted by the indicated agonist appears in parentheses. Data were normalised to maximal response in cells from control subjects. **(F)** EC₅₀ values of tested compounds in each functional group. Data are expressed as mean (± SE) of n = 12 patients per group. * P < 0.05, **P < 0.01, ***P < 0.001, vs control group.

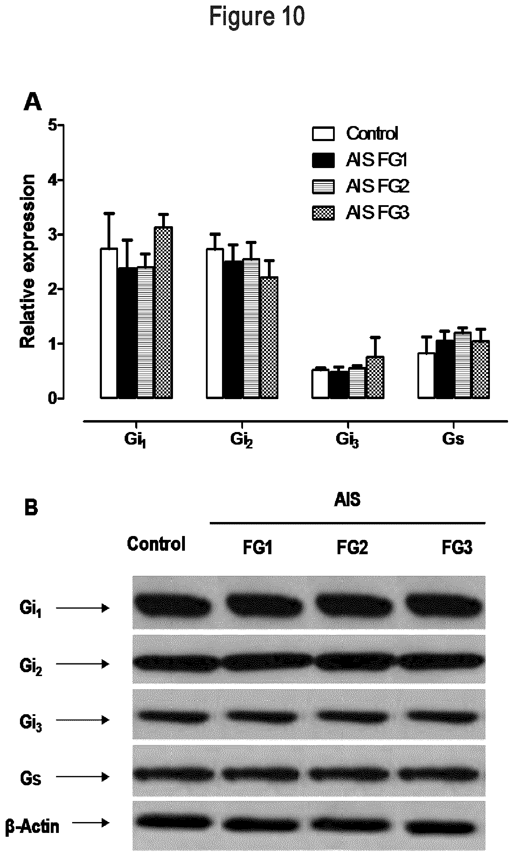

**Figure 10** **shows that expression of Gi and Gs proteins is similar between AIS functional groups. (A)** Total RNA were extracted from osteoblasts and qPCR was used to compare mRNA expression levels of Gi₁, Gi₂, Gi₃ and Gs genes in control relative to the AIS functional groups. β-actin was used as internal control. **(B)** Lysates were obtained from osteoblasts of each functional group. Equal amounts of proteins (40 µg) of each lysate were resolved by 10% SDS-PAGE and immunoblotted for Gi1, Gi2, Gi3 or Gs proteins. β-actin was used as internal control.

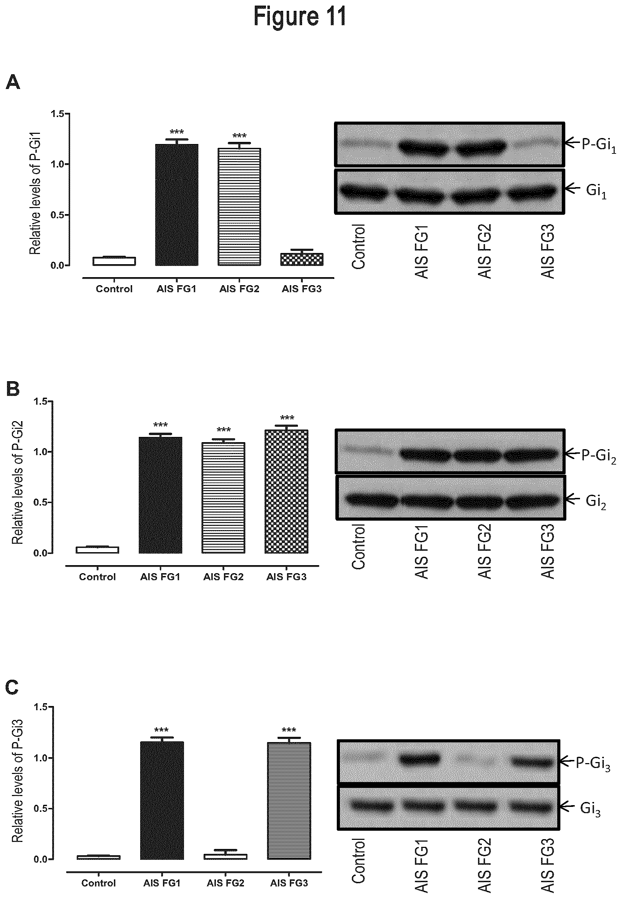

**Figure 11** **shows the differential phosphorylation patterns of Gi protein isoforms in AIS functional groups.** Whole osteoblast cells from control or AIS patients were subjected to immunoprecipitation with antibody recognizing **(A)** Gi₁, **(B)** Gi₂ or **(C)** Gi₃, and these precipitates were resolved by 10% SDS-PAGE and immunoblotted for phospho-serine/threonine specific antibody. Representative immunoblots from a single experiment are shown in the inserts. The bands were quantified by densitometric scanning. Values are expressed as mean ± SE, of n = 6 patients per group. * P < 0.05, **P < 0.01, ***P < 0.001, vs control group. Insert despites a typical immunodetectable phosphorylation profile of corresponding Gi protein isoforms in functional groups.

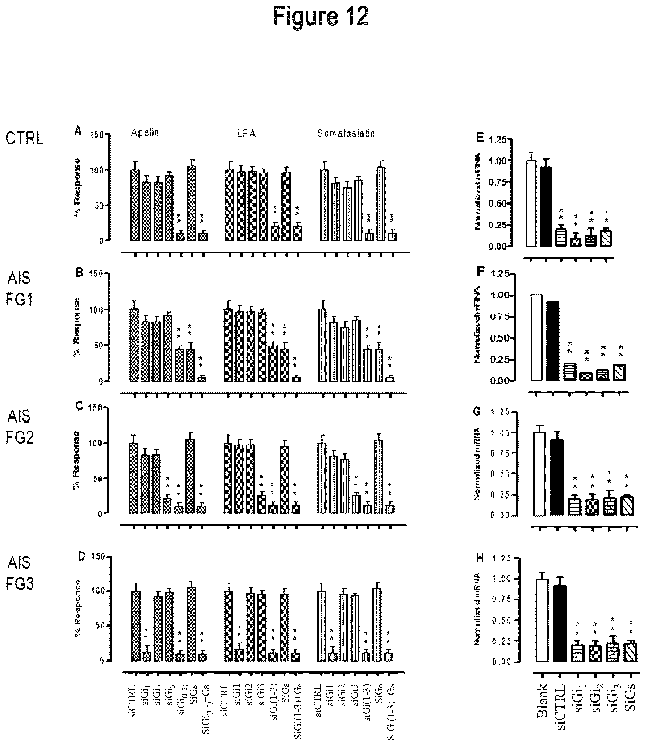

**Figure 12** **shows the differential effects of Gi and Gs siRNA on response to Gi stimulation in AIS functional groups.** Osteoblasts from **(A)** control subjects, **(B)** FG3, **(C)** FG2; and **(D)** FG1 were transfected with Gi₁, Gi₂, Gi₃ or Gs siRNA alone or in combination, or with scrambled siRNA, as indicated in **Example 1** - Materials and Methods. Efficiency of siRNA was verified with qPCR in each functional group **(E to H)** 48 hours after transfection, and response to Gi stimulation was evaluated by challenging cells with Apelin-17, LPA or Somatostatin. Results from a single representative subject for each group are presented. Data were normalised to response in cells transfected with scrambled siRNA, and are expressed as mean ± SE, of n = 6 patients in each functional group. * P < 0.05, **P < 0.01, ***P < 0.001, vs. control group.

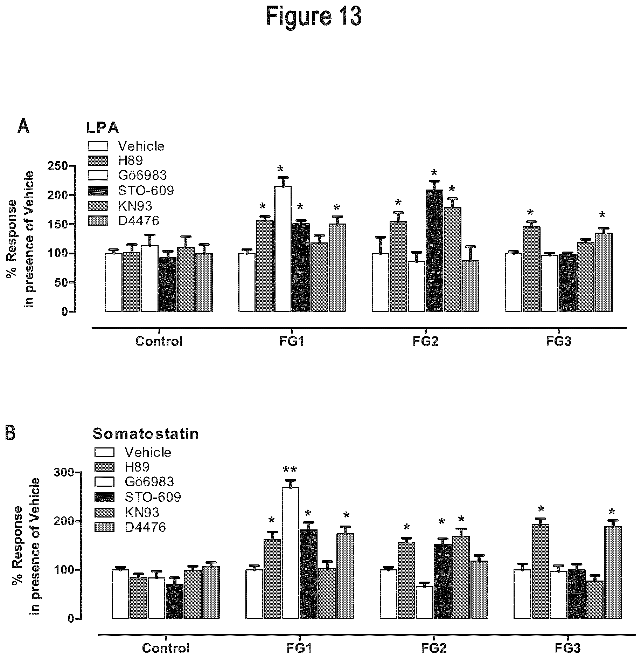

**Figure 13** **shows the effect of various serine/threonine kinase inhibitors on response to Gi stimulation in osteoblasts from control and AIS functional groups.** Cells were treated with Protein kinase A (PKA) inhibitor H89 (5 µM), Protein kinase C (PKC) inhibitor Gö6983 (5 µM), Ca²⁺ /calmodulin-dependent protein kinase (CaMK) (1,2,4) inhibitor STO-609 acetate (5 µM), Ca²⁺ /calmodulin-dependent protein kinase II (CaMK-2) inhibitor KN93 (5 µM), Casein kinase 1 (CK1) inhibitor D4476 (5 µM) or vehicle for 1 hour prior to the stimulation with **(A)** LPA (10⁻⁶ M) or **(B)** Somatostatin (10⁻⁶ M). Data were normalised to response in osteoblasts treated with vehicle, and are expressed as mean ± SE, of n = 12 patients in each functional group. * P < 0.05, **P < 0.01, ***P < 0.001, vs control group.

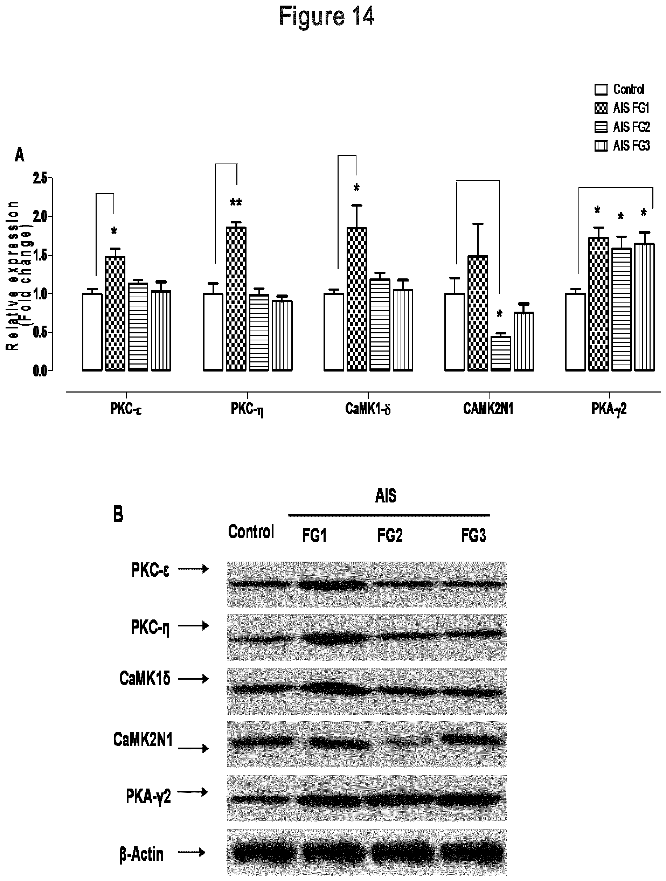

**Figure 14** **shows expression of various serine/threonine kinases in AIS. (A)** Total RNA were extracted from osteoblasts and qPCR was used to compare mRNA expression levels of PKC, PKA, CaMK and CK isoforms in control relative to the AIS functional groups. β-actin was used as internal control. **(B)** Lysates were obtained from osteoblasts of each functional group. Equal amounts of proteins (40 µg) of each lysate were resolved by 10% SDS-PAGE and immunoblotted for proteins of indicated kinase isoforms.

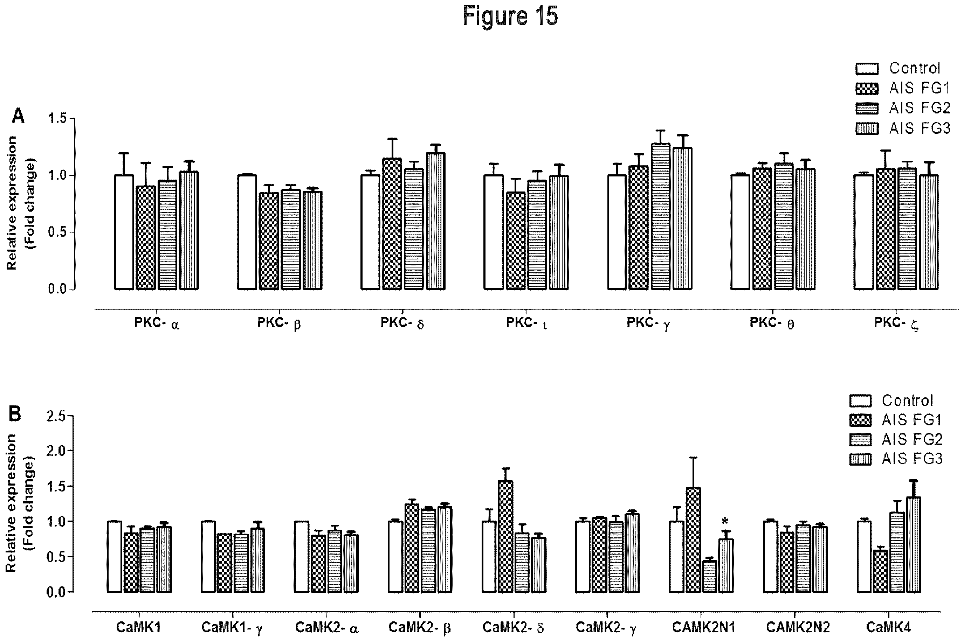

**Figure 15** **shows expression of various serine/threonine kinases in AIS.** Total RNA were extracted from osteoblasts and qPCR was used to compare mRNA expression levels of **(A)** various PKC isoforms (i.e., PKC-α, PKC-β, PKC-δ, PKC-I, PKC-γ, PKC-θ and PKC-ζ) and **(B)** various isoforms of CaMK (i.e., CaMK1, CaMK1γ, CaMK2α, CαMK2β, CαMK2δ, CaMK2γ, CaMK2N1, CaMK2N2 and CaMK4) in control relative to the AIS functional groups. β-actin was used as internal control.

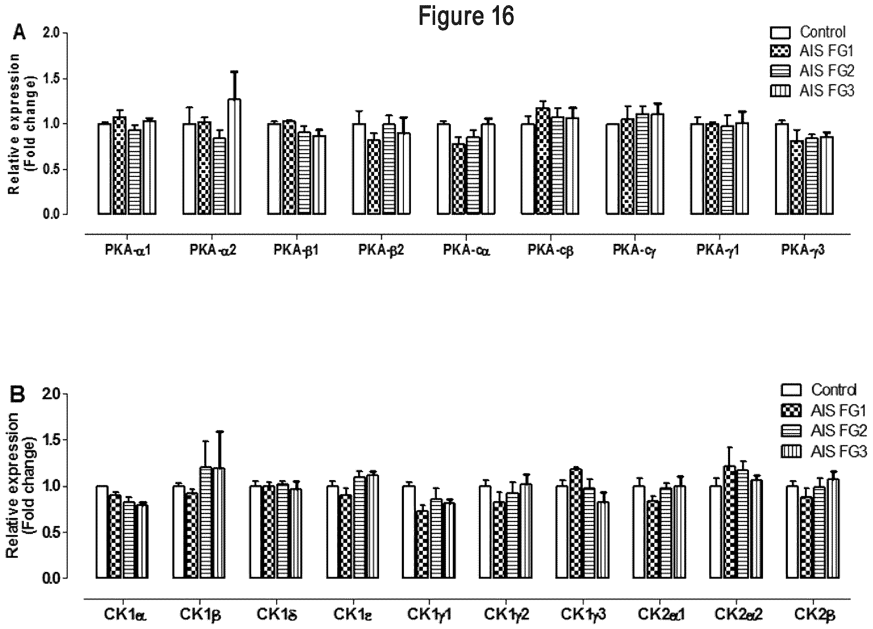

**Figure 16** **shows expression of various serine/threonine kinases in AIS.** Total RNA were extracted from osteoblasts and qPCR was used to compare mRNA expression levels of various isoforms of PKA and CK (i.e., **(A)** PKA-α1, PKA-α2, PKA-β1, PKAβ2, PKA-cβ, PKA-cγ, PKA-γ1 and PKA-γ3; and **(B)** CK1α, CK1β, CK1δ, CK1ε, CK1γ1, CK1γ2, CK1γ3, CK2α1, CK2α2, CK2β) in control relative to the AIS functional groups. β-actin was used as internal control.

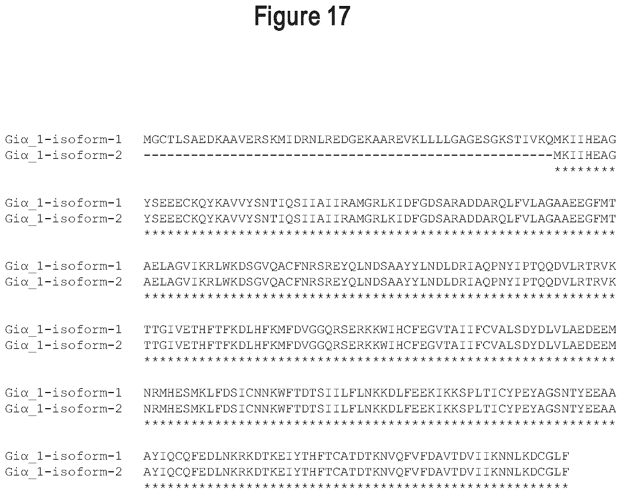

**Figure 17** shows a multiple sequence alignment between the amino acid sequences of Giα1 isoforms 1 and 2.

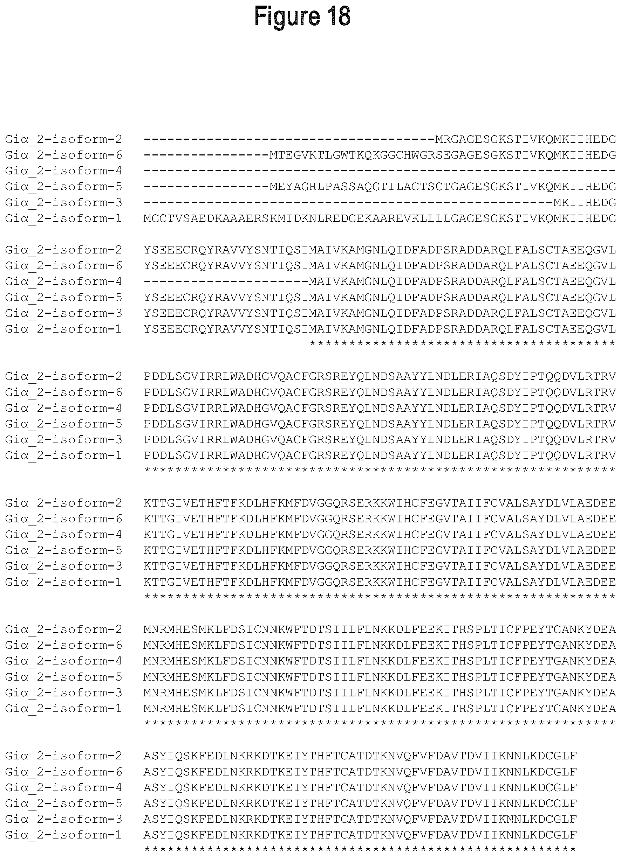

**Figure 18** shows a multiple sequence alignment between the amino acid sequences of Giα2 isoforms 1-6.

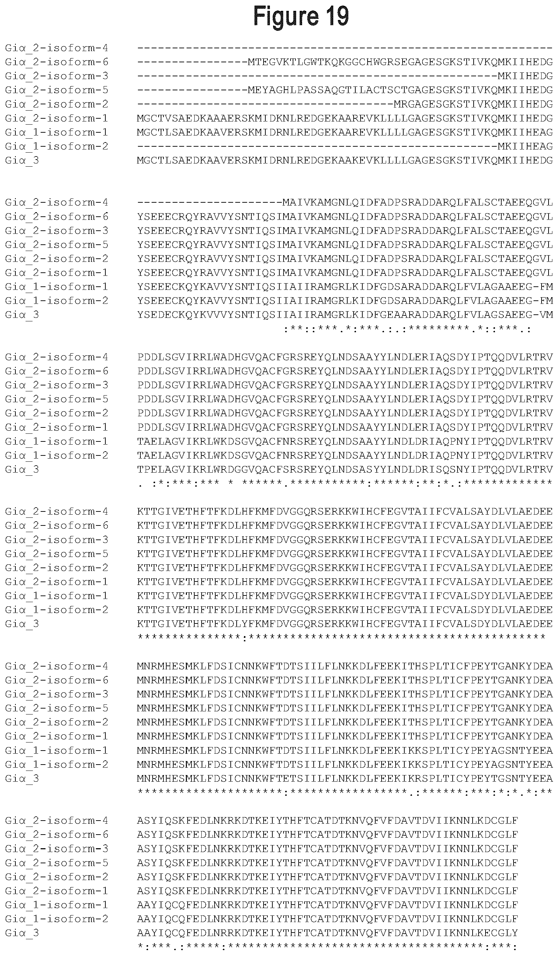

**Figure 19** shows a multiple sequence alignment between the amino acid sequences of Giα1 isoforms 1 and 2, Giα1 isoforms 1 and 2, and Giα3.

### DESCRIPTION OF ILLUSTRATIVE EMBODIMENTS

As used herein the terms **"risk of developing scoliosis"** refer to a genetic or metabolic predisposition of a subject to develop a scoliosis (i.e., spinal deformity) and/or to develop a more severe scoliosis at a future time (i.e., curve progression). For instance, an increase of the Cobb's angle of a subject (e.g., from 40° to 50°, or from 18° to 25°) is a "development" of scoliosis.

As used herein, the term **"severe progression"** is an increase of a subject's Cobb's angle to 45° or more, potentially at a younger age.

In accordance with the present invention, subjects having AIS can be stratified into at least three distinct AIS subclasses (FG1, FG2, or FG3) based on their Giα protein phosphorylation profiles and/or the degree of imbalance in the responses to Giα and Gsα protein stimulation. Subjects belonging to a first AIS subclass (FG1) generally have a relatively **"low risk"** of severe progression -- i.e., their risk of developing severe scoliosis is lower than that of subjects belonging to the second (FG2) and third AIS subclasses (FG3). Subjects belonging to a second AIS subclass (FG2) generally have a relatively **"high risk"** of severe progression -- i.e., their risk of developing severe scoliosis is higher than that of subjects belonging to the first (FG1) and third AIS subclasses (FG3). Subjects belonging to a third AIS subclass (FG3) generally have a **"moderate risk"** of severe progression -- i.e., their risk of developing severe scoliosis is higher than that of subjects belonging to the first (FG1) AIS subclass, but less than that of subjects belonging to the second AIS subclass (FG2). It has also been found that subjects belonging to this third AIS subclass (FG3) are more likely to respond to bracing treatment (US provisional application No. 61/879,314).

In an embodiment, the above-mentioned subject is a likely candidate for developing a scoliosis, such as idiopathic scoliosis (e.g., Infantile Idiopathic Scoliosis, Juvenile Idiopathic Scoliosis or Adolescent Idiopathic Scoliosis (AIS)). As used herein, the expressions **"likely candidate for developing scoliosis"** or "likely to develop scoliosis" include subjects (e.g., children) of which at least one parent has a scoliosis (e.g., adolescent idiopathic scoliosis). Among other factors, age (adolescence), gender and other family antecedents are factors that are known to contribute to the risk of developing a scoliosis and are used to a certain degree to assess the risk of developing a scoliosis. In certain subjects, scoliosis develops rapidly over a short period of time to the point of requiring a corrective surgery (often when the deformity reaches a Cobb's angle ≥ 50°). Current courses of action available from the moment a scoliosis such as AIS is diagnosed (when scoliosis is apparent) include observation (when Cobb's angle is around 10-25°), orthopedic devices (when Cobb's angle is around 25-30°), and surgery (over 45°). A more reliable determination of the risk of progression could enable to 1) select an appropriate diet to remove certain food products identified as contributors to scoliosis; 2) select the best therapeutic agent; and/or 3) select the least invasive available treatment such as postural exercises, orthopedic device, or less invasive surgeries or surgeries without fusions (a surgery that does not fuse vertebra and preserves column mobility). The present invention encompasses selecting the most efficient and least invasive known preventive actions or treatments in view of the determined risk of developing scoliosis.

As used herein the term **"subject"** is meant to refer to any mammal including human, mouse, rat, dog, chicken, cat, pig, monkey, horse, etc. In a particular embodiment, it refers to a human.

A **"subject in need thereof"** or a **"patient"** in the context of the present invention is intended to include any subject that will benefit or that is likely to benefit from an inhibitor of PKA (*e.g*., PKA-y2), PKC (*e.g*., PKC-ε, PKC-η), CaMK1 (*e.g*., CaMK1δ), CaMK4, CK (*e.g*., CK2), and/or CaMK2. In an embodiment, a subject in need thereof is a subject diagnosed with a scoliosis (*e.g*., AIS). In another embodiment, the subject is likely to develop a scoliosis (*e.g.,* AIS) or is at risk of developing scoliosis".

As used herein the terminology **"biological sample"** refers to any solid or liquid sample isolated from a living being. In a particular embodiment, it refers to any solid or liquid sample isolated from a human. Without being so limited, it includes a biopsy material, blood, tears, saliva, maternal milk, synovial fluid, urine, ear fluid, amniotic fluid and cerebrospinal fluid. In a specific embodiment it refers to a blood sample. As used herein, the terminology **"blood sample"** is meant to refer to blood, plasma or serum.

As used herein, the expression **"cell sample"** refers to a biological sample containing GiPCR-expressing cells obtained from a subject (e.g., a subject having, suspected of having, or at risk of developing AIS).

As used herein the terminology **"control sample"** is meant to refer to a corresponding sample that does not come from a subject known to have scoliosis or known to be a likely candidate for developing a scoliosis. In methods for determining the risk of developing scoliosis in a subject that is pre-diagnosed with scoliosis, the sample may however also come from the subject under scrutiny at an earlier stage of the disease or disorder.

As used herein the terminology **"control"** is meant to encompass "control sample". In certain embodiments, the term **"control"** also refers to the average or median value obtained following determination of Giα (*e.g*., 1, 2 and/or 3) protein phosphorylation (*e.g.,* serine phosphorylation) profiles and/or the degree of imbalance in the responses to Giα and Gsα proteins stimulation in a plurality of samples (*e.g*., samples obtained from several subjects not known to have scoliosis and not known to be a likely candidate for developing scoliosis).

As used herein the term **"treating"** or **"treatment"** in reference to scoliosis is meant to refer to at least one of a reduction of Cobb's angle in a preexisting spinal deformity, improvement of column mobility, preservation/maintenance of column mobility, improvement of equilibrium and balance in a specific plan; maintenance/preservation of equilibrium and balance in a specific plan; improvement of functionality in a specific plan, preservation/maintenance of functionality in a specific plan, cosmetic improvement, and combinations of any of the above.

As used herein the term **"preventing"** or **"prevention"** in reference to scoliosis is meant to refer to a at least one of a reduction in the progression of a Cobb's angle in a patient having a scoliosis or in an asymptomatic patient, a complete prevention of apparition of a spinal deformity, including changes affecting the rib cage and pelvis in 3D, and a combination of any of the above.

As used herein, the expression **"detecting or determining the serine phosphorylation"** of a Giα protein (e.g., Giα1, Giα2, and/or Giα3) in the cell sample relates to independently assessing the serine phosphorylation status of the Giα1 and/or Giα3 proteins in a cell sample from a subject. In some embodiments, this assessing can include determining the presence or absence of serine-phosphorylated Giα1, Giα2 and/or Giα3 proteins in the cell sample. In some embodiments, this assessing can include determining the level of expression of serine-phosphorylated Giα1, Giα2 and/or Giα3 proteins in the sample, or the proportion of total Giα1 and/or Giα3 proteins (e.g., both phosphorylated and unphosphorylated) in the cell sample which are serine-phosphorylated. In some embodiments, this assessing involves detecting each of the serine-phosphorylated Giα proteins independently (e.g., using specific anti-Giα1, anti-Giα2, and/or anti-Giα3 antibodies).

Guanine nucleotide binding proteins are heterotrimeric signal-transducing molecules consisting of alpha, beta, and gamma subunits. The alpha subunit binds guanine nucleotide, can hydrolyze GTP, and can interact with other proteins. As used herein, the expression **"Gia"** or **"Gia protein"** refers to the alpha subunit of members of the Gi-family of heterotrimeric G proteins. There are several types of Gi alpha subunits, including **"Gia1"** or **"Gia1 protein", "Gia2"** or **"Gia2 protein",** and **"Gia3"** or **"Gia3 protein".** As used herein, the expression **"Gs"** or **"Gs protein"** refers to the Gs subunit of members of the Gs-family of heterotrimeric G proteins. Examples of Gia and Gs nucleotide and amino acid sequences are shown in the Table below. Unless otherwise indicated, reference to a particular Gia protein (e.g., Gia1, Gia2, Gia3) includes all expressed isoforms of that particular Gia protein.

| Gene (GeneID) | Protein (accession No.) | cDNA sequence | Amino acid sequence |
|---|---|---|---|
| *GNAI1* (2770) | Giα1 isoform 1 (NP_002060) | SEQ ID NO: 77 | SEQ ID NO: 78 |
| *GNAI1* (2770) | Giα1 isoform 2 (NP_001243343) | SEQ ID NO: 79 | SEQ ID NO: 80 |
| *GNAI2* (2771) | Giα2 isoform 1 (NP_002061) | SEQ ID NO: 81 | SEQ ID NO: 82 |
| *GNAI2* (2771) | Giα2 isoform 2 (NP_001159897) | SEQ ID NO: 83 | SEQ ID NO: 84 |
| *GNAI2* (2771) | Giα2 isoform 3 (NP_001269546) | SEQ ID NO: 85 | SEQ ID NO: 86 |
| *GNAI2* (2771) | Giα2 isoform 4 (NP_001269547) | SEQ ID NO: 87 | SEQ ID NO: 88 |
| *GNAI2* (2771) | Giα2 isoform 5 (NP_001269548) | SEQ ID NO: 89 | SEQ ID NO: 90 |
| *GNAI2* (2771) | Giα2 isoform 6 (NP_001269549) | SEQ ID NO: 91 | SEQ ID NO: 92 |
| *GNAI3* (2773) | Giα3 (NP_006487) | SEQ ID NO: 93 | SEQ ID NO: 94 |
| *GNAS* (2778) | Gs (NM_000516) | SEQ ID NO: 95 | SEQ ID NO: 96 |

As used herein, the expression **"detecting or determining responses to Gia and Gsa protein stimulation in the cell sample"** relates to assessing the ability of a subject's Gia and Gsa proteins to mediate signal transduction upon stimulation (e.g., with an appropriate GPCR ligand or agonist), and thus relate to the activity and not to the level of expression of Gia and Gsa proteins. In some embodiments, this can be done using a CellKey™ apparatus, as previously described (Akoume et al., 2010 and WO 2010/040234, 2010 to Moreau et al.).

The terms **"suppressor", "inhibitor"** and **"antagonist"** are well known in the art and are used herein interchangeably. The expression inhibitors of PKA (*e.g*., PKA-γ2), inhibitors of PKC (*e.g.*, PKC-η or PKC-ε), inhibitors of CaMK1 (*e.g*., CaMK1δ), inhibitors of CaMK4, inhibitors of CK (*e.g*., CK2) and inhibitors of CaMK2 include any compound able to negatively affect the activity of PKA (*e.g*., PKA-γ2), PKC (*e.g*., PKC-η or PKC-ε), CaMK1 (*e.g*., CaMK1δ), CaMK4, CK (*e.g*., CK2) and CaMK2, respectively by reducing for example its expression (i.e., at the transcriptional and/or translational level), the level of PKA (*e.g.*, PKA-γ2), PKC (*e.g*., PKC-η or PKC-ε), CaMK1 (*e.g*., CaMK1δ), CaMK4, CK (*e.g*., CK2) and CaMK2 mRNA, respectively and/or protein, or an activity associated with PKA (*e.g*., PKA-γ2), PKC (*e.g.*, PKC-η or PKC-ε), CaMK1 (*e.g*., CaMK1δ), CaMK4, CK (*e.g*., CK2) and CaMK2, respectively. It includes intracellular as well as extracellular suppressors. Without being so limited, such suppressors include RNA interference agents (siRNA, shRNA, miRNA), antisense molecules, ribozymes, proteins (*e.g*., dominant negative, inactive variants), peptides, small molecules, antibodies, antibody fragments, etc. Without being limited, inhibitors of PKA (*e.g*., PKA-γ2) include H89 cGMP dependent kinase inhibitor peptide; KT 5720; PKA inhibitor fragment (6-22) amide; PKI 14-22 amide, myristoylated. Without being limited, inhibitors of PKC (*e.g.*, PKC-ε and PKC-η) include Gö6983, Gö6976; GF109203X; Dihydrosyphingosine; CID2858522; Chelerythrine chloride; CGP53353; Calphostin C; C-1; and Binsindolylmaleimide II. Without being limited, inhibitors of CaMK1 and CaMK4 (*e.g*., CaMK1δ) include STO609; NH 125; ML 9 hydrochloride; autocamtide-2-related inhibitory peptide; and arcyriaflavin A. Without being limited, inhibitors of CaMK2 include KN93, NH 125; ML 9 hydrochloride; autocamtide-2-related inhibitory peptide; and arcyriaflavin A. Without being limited, inhibitors of CK (*e.g.*, CK1, CK2) include (R)-DRF053 dihydrochloride inhibits CK1), Ellagic acid (Selective inhibitor of CK2), LH 846 (Selective casein kinase 1δ inhibitor), PF 4800567 hydrochloride (Selective casein kinase 1ε inhibitor), PF 670462 (Potent and selective CK1ε and CK1δ inhibitor), TBB (Selective cell-permeable CK2 inhibitor), TMCB (inhibits CK2) and D4476 (Selective CK1 inhibitor).

In an embodiment, the PKA (*e.g.*, PKA-γ2), PKC (*e.g*., PKC-η or PKC-ε), CaMK1 (*e.g*., CaMK1δ), CaMK4, CK (*e.g*., CK2) or CaMK2 inhibitor is a neutralizing antibody directed against (or specifically binding to) a human PKA (*e.g*., PKA-γ2), PKC (*e.g*., PKC-η or PKC-ε), CaMK1 (*e.g*., CaMK1δ), CaMK4, CK (*e.g*., CK2) and CaMK2 polypeptide, respectively. Antibodies are further described below.

The present invention also relates to methods for the determination of the level of expression (*i.e.*, transcript (RNA) or translation product (protein)) of Gαi1 (having phosphorylated and/or unphosphorylated serine residues), Gai2 (*e.g.*, having phosphorylated and/or unphosphorylated serine residues), Gai3 (*e.g*., having phosphorylated and/or unphosphorylated serine residues), and Gas. In specific embodiments, it also includes a method that comprises the determination of the level of expression of one or more other scoliosis markers. For example, it may include the determination of the level of expression (i.e., transcript or translation product) of OPN, sCD44, etc. as disclosed in co-pending WO 2008/119170 to Moreau. The present invention therefore encompasses any known method for such determination including Elisa (Enzyme Linked Immunosorbent Assay), RIA (Radioimmunoassay), real time PCR and competitive PCR, Northern blots, nuclease protection, plaque hybridization and slot blots.

### Antibodies

As used herein, the terms anti-PKA (e.g., PKA-γ2), PKC (e.g., PKC-η or PKC-ε), CaMK1 (e.g., CaMK1δ), CaMK4, CK (e.g., CK2), CaMK2, Giα1 (e.g., having phosphorylated and/or unphosphorylated serine residues), Giα2 (e.g., having phosphorylated and/or unphosphorylated serine residues), Giα3 (e.g., having phosphorylated and/or unphosphorylated serine residues) or Gsα antibody or "immunologically specific anti-PKA-γ2, PKC-ε, PKC-η, CaMK1δ, Gia1 (e.g., having phosphorylated and/or unphosphorylated serine residues), Giα2 (e.g., having phosphorylated and/or unphosphorylated serine residues), Giα3 (e.g., having phosphorylated and/or unphosphorylated serine residues), or Gsα antibody, refers to an antibody that specifically binds to (interacts with) a PKA (*e.g*., PKA-γ2), PKC (e.g., PKC-η or PKC-ε), CaMK1 (*e.g*., CaMK1δ), CaMK4, CK (*e.g*., CK2), CaMK2, Gia1 (e.g., having phosphorylated and/or unphosphorylated serine residues), Gia2 (e.g., having phosphorylated and/or unphosphorylated serine residues), Gia3 (e.g., having phosphorylated and/or unphosphorylated serine residues), or Gsα protein, respectively and displays no substantial binding to other naturally occurring proteins other than the ones sharing the same antigenic determinants as the PKA (*e.g.*, PKA-y2), PKC (*e.g*., PKC-η or PKC-ε), CaMK1 (*e.g*., CaMK1δ), CaMK4, CK (*e.g*., CK2), CaMK2, Gia1 (e.g., having phosphorylated and/or unphosphorylated serine residues), Gia2 (e.g., having phosphorylated and/or unphosphorylated serine residues), Gia3 (e.g., having phosphorylated and/or unphosphorylated serine residues), or Gsa protein. The term **"antibody"** or **"immunoglobulin"** is used in the broadest sense, and covers monoclonal antibodies (including full length monoclonal antibodies), polyclonal antibodies, multispecific antibodies, and antibody fragments so long as they exhibit the desired biological activity. Antibody fragments comprise a portion of a full length antibody, generally an antigen binding or variable region thereof. Examples of antibody fragments include Fab, Fab', F(ab')₂, and Fv fragments, diabodies, linear antibodies, single-chain antibody molecules, single domain antibodies (*e.g*., from camelids), shark NAR single domain antibodies, and multispecific antibodies formed from antibody fragments. Antibody fragments can also refer to binding moieties comprising CDRs or antigen binding domains including, but not limited to, VH regions (V_{H}, V_{H}-V_{H}), anticalins, PepBodies™, antibody-T-cell epitope fusions (Troybodies) or Peptibodies. Additionally, any secondary antibodies, either monoclonal or polyclonal, directed to the first antibodies would also be included within the scope of this invention. In an embodiment, the antibody is a monoclonal antibody. In another embodiment, the antibody is a humanized or CDR-grafted antibody.

Antibodies directed to PKA (e.g., PKA-y2), PKC (e.g., PKC-η or PKC-ε), CaMK1 (e.g., CaMK1δ), CaMK4, CK (e.g., CK2), CaMK2, Gia1 (e.g., having phosphorylated and/or unphosphorylated serine residues), Gia2 (e.g., having phosphorylated and/or unphosphorylated serine residues), Gia3 (e.g., having phosphorylated and/or unphosphorylated serine residues) and Gsα are also described and can be produced by well established procedures known to those of skill in the art. Additionally, any secondary antibodies, either monoclonal or polyclonal, directed to the first antibodies would also be described.

In general, techniques for preparing antibodies (including monoclonal antibodies and hybridomas) and for detecting antigens using antibodies are well known in the art (Campbell, 1984, In "Monoclonal Antibody Technology: Laboratory Techniques in Biochemistry and Molecular Biology", Elsevier Science Publisher, Amsterdam, The Netherlands) and in Harlow et al., 1988 (in: Antibody A Laboratory Manual, CSH Laboratories). The term antibody encompasses herein polyclonal, monoclonal antibodies and antibody variants such as single-chain antibodies, humanized antibodies, chimeric antibodies and immunologically active fragments of antibodies (e.g. Fab and Fab' fragments) which inhibit or neutralize their respective interaction domains in Hyphen and/or are specific thereto.

Polyclonal antibodies are preferably raised in animals by multiple subcutaneous (sc), intravenous (iv) or intraperitoneal (ip) injections of the relevant antigen with or without an adjuvant. It may be useful to conjugate the relevant antigen to a protein that is immunogenic in the species to be immunized, *e.g.,* keyhole limpet hemocyanin, serum albumin, bovine thyroglobulin, or soybean trypsin inhibitor using a bifunctional or derivatizing agent, for example, maleimidobenzoyl sulfosuccinimide ester (conjugation through cysteine residues), N-hydroxysuccinimide (through lysine residues), glutaraldehyde, succinic anhydride, SOCl₂, or R¹N=C=NR, where R and R¹ are different alkyl groups.

Animals may be immunized against the antigen, immunogenic conjugates, or derivatives by combining the antigen or conjugate (*e.g.,* 100 µg for rabbits or 5 µg for mice) with 3 volumes of Freund's complete adjuvant and injecting the solution intradermally at multiple sites. One month later the animals are boosted with the antigen or conjugate (*e.g.,* with 1/5 to 1/10 of the original amount used to immunize) in Freund's complete adjuvant by subcutaneous injection at multiple sites. Seven to 14 days later the animals are bled and the serum is assayed for antibody titer. Animals are boosted until the titer plateaus. Preferably, for conjugate immunizations, the animal is boosted with the conjugate of the same antigen, but conjugated to a different protein and/or through a different cross-linking reagent. Conjugates also can be made in recombinant cell culture as protein fusions. Also, aggregating agents such as alum are suitably used to enhance the immune response.

Monoclonal antibodies may be made using the hybridoma method first described by Kohler et al., Nature, 256: 495 (1975), or may be made by recombinant DNA methods (e.g., U.S. Patent No. 6,204,023). Monoclonal antibodies may also be made using the techniques described in U.S. Patent Nos. 6,025,155 and 6,077,677 as well as U.S. Patent Application Publication Nos. 2002/0160970 and 2003/0083293.

In the hybridoma method, a mouse or other appropriate host animal, such as a rat, hamster or monkey, is immunized (*e.g.,* as hereinabove described) to elicit lymphocytes that produce or are capable of producing antibodies that will specifically bind to the antigen used for immunization. Alternatively, lymphocytes may be immunized *in vitro.* Lymphocytes then are fused with myeloma cells using a suitable fusing agent, such as polyethylene glycol, to form a hybridoma cell.

The hybridoma cells thus prepared are seeded and grown in a suitable culture medium that preferably contains one or more substances that inhibit the growth or survival of the unfused, parental myeloma cells. For example, if the parental myeloma cells lack the enzyme hypoxanthine guanine phosphoribosyl transferase (HGPRT or HPRT), the culture medium for the hybridomas typically will include hypoxanthine, aminopterin, and thymidine (HAT medium), which substances prevent the growth of HGPRT-deficient cells.

As used herein, the term "purified" in the expression "purified antibody" is simply meant to distinguish man-made antibody from an antibody that may naturally be produced by an animal against its own antigens. Hence, raw serum and hybridoma culture medium containing anti-OPN antibody are "purified antibodies" within the meaning of the present invention.

The present description also concerns isolated **nucleic acid molecules** including probes and primers to detect PKA (*e.g.,* PKA-y2), PKC (e.g., PKC-η or PKC-ε), CaMK1 (*e.g.,* CaMK1δ), CaMK4, CK (*e.g.,* CK2), CaMK2, Gia1, Gia2, Gia3 or Gsa (and optionally other scoliosis markers (*e.g.,* OPN, sCD44, etc.). In specific embodiments, the isolated nucleic acid molecules have no more than 300, or no more than 200, or no more than 100, or no more than 90, or no more than 80, or no more than 70, or no more than 60, or no more than 50, or no more than 40 or no more than 30 nucleotides. In specific embodiments, the isolated nucleic acid molecules have at least 17, or at least 18, or at least 19, or at least 20, or at least 30, or at least 40 nucleotides. In other specific embodiments, the isolated nucleic acid molecules have at least 20 and no more than 300 nucleotides. In other specific embodiments, the isolated nucleic acid molecules have at least 20 and no more than 200 nucleotides. In other specific embodiments, the isolated nucleic acid molecules have at least 20 and no more than 100 nucleotides. In other specific embodiments, the isolated nucleic acid molecules have at least 20 and no more than 90 nucleotides. In other specific embodiments, the isolated nucleic acid molecules have at least 20 and no more than 80 nucleotides. In other specific embodiments, the isolated nucleic acid molecules have at least 20 and no more than 70 nucleotides. In other specific embodiments, the isolated nucleic acid molecules have at least 20 and no more than 60 nucleotides. In other specific embodiments, the isolated nucleic acid molecules have at least 20 and no more than 50 nucleotides. In other specific embodiments, the isolated nucleic acid molecules have at least 20 and no more than 40 nucleotides. In other specific embodiments, the isolated nucleic acid molecules have at least 17 and no more than 40 nucleotides. In other specific embodiments, the isolated nucleic acid molecules have at least 20 and no more than 30 nucleotides. In other specific embodiments, the isolated nucleic acid molecules have at least 17 and no more than 30 nucleotides. In other specific embodiments, the isolated nucleic acid molecules have at least 30 and no more than 300 nucleotides. In other specific embodiments, the isolated nucleic acid molecules have at least 30 and no more than 200 nucleotides. In other specific embodiments, the isolated nucleic acid molecules have at least 30 and no more than 100 nucleotides. In other specific embodiments, the isolated nucleic acid molecules have at least 30 and no more than 90 nucleotides. In other specific embodiments, the isolated nucleic acid molecules have at least 30 and no more than 80 nucleotides. In other specific embodiments, the isolated nucleic acid molecules have at least 30 and no more than 70 nucleotides. In other specific embodiments, the isolated nucleic acid molecules have at least 30 and no more than 60 nucleotides. In other specific embodiments, the isolated nucleic acid molecules have at least 30 and no more than 50 nucleotides. In other specific embodiments, the isolated nucleic acid molecules have at least 30 and no more than 40 nucleotides. It should be understood that in real-time PCR, primers also constitute probe without the traditional meaning of this term. Primers or probes appropriate to detect PKA (*e.g.,* PKA-γ2), PKC (*e.g.,* PKC-η or PKC-ε), CaMK1 (*e.g.,* CaMK1δ), CaMK4, CK (*e.g.,* CK2), CaMK2, Giα1, Giα2, Giα3 or Gsα in the methods of the present invention can be designed with known methods using sequences distributed across their respective nucleotide sequence.

Probes of the invention can be utilized with naturally occurring sugar-phosphate backbones as well as modified backbones including phosphorothioates, dithionates, alkyl phosphonates and α-nucleotides and the like. Modified sugar-phosphate backbones are generally known. Probes of the invention can be constructed of either ribonucleic acid (RNA) or deoxyribonucleic acid (DNA), and preferably of DNA.

The types of detection methods in which probes can be used include Southern blots (DNA detection), dot or slot blots (DNA, RNA), and Northern blots (RNA detection). Although less preferred, labeled proteins could also be used to detect a particular nucleic acid sequence to which it binds. Other detection methods include kits containing probes on a dipstick setup and the like.

As used herein the terms **"detectably labeled"** refer to a marking of a probe or an antibody in accordance with the presence invention that will allow the detection of PKA (*e.g.,* PKA-γ2), PKC (*e.g.,* PKC-η or PKC-ε), CaMK1 (*e.g*., CaMK1δ), CaMK4, CK (*e.g.,* CK2), CaMK2, Giα1, Giα2, Giα3 or Gsα (and optionally other scoliosis markers (*e.g*., OPN, sCD44, etc.). in accordance with the present invention. Although the present invention is not specifically dependent on the use of a label for the detection of a particular nucleic acid sequence, such a label might be beneficial, by increasing the sensitivity of the detection. Furthermore, it enables automation. Probes can be labeled according to numerous well known methods. Non-limiting examples of labels include 3H, 14C, 32P, and 35S. Non-limiting examples of detectable markers include ligands, fluorophores, chemiluminescent agents, enzymes, and antibodies. Other detectable markers for use with probes, which can enable an increase in sensitivity of the method of the invention, include biotin and radionucleotides. It will become evident to the person of ordinary skill that the choice of a particular label dictates the manner in which it is bound to the probe.

As commonly known, radioactive nucleotides can be incorporated into probes of the invention by several methods. Non-limiting examples thereof include kinasing the 5' ends of the probes using gamma 32P ATP and polynucleotide kinase, using the Klenow fragment of Pol I of *E. coli* in the presence of radioactive dNTP (e.g. uniformly labeled DNA probe using random oligonucleotide primers in low-melt gels), using the SP6/T7 system to transcribe a DNA segment in the presence of one or more radioactive NTP, and the like.

The present invention also relates to methods of selecting compounds. As used herein the term "compound" is meant to encompass natural, synthetic or semi-synthetic compounds, including without being so limited chemicals, macromolecules, cell or tissue extracts (from plants or animals), nucleic acid molecules, peptides, antibodies and proteins.

The present invention also relates to arrays. As used herein, an **"array"** is an intentionally created collection of molecules which can be prepared either synthetically or biosynthetically. The molecules in the array can be identical or different from each other. The array can assume a variety of formats, *e.g.,* libraries of soluble molecules; libraries of compounds tethered to resin beads, silica chips, or other solid supports.

As used herein **"array of nucleic acid molecules"** is an intentionally created collection of nucleic acids which can be prepared either synthetically or biosynthetically in a variety of different formats (*e.g.,* libraries of soluble molecules; and libraries of oligonucleotides tethered to resin beads, silica chips, or other solid supports). Additionally, the term "array" is meant to include those libraries of nucleic acids which can be prepared by spotting nucleic acids of essentially any length (*e.g.,* from 1 to about 1000 nucleotide monomers in length) onto a substrate. The term **"nucleic acid"** as used herein refers to a polymeric form of nucleotides of any length, either ribonucleotides, deoxyribonucleotides or peptide nucleic acids (PNAs), that comprise purine and pyrimidine bases, or other natural, chemically or biochemically modified, non-natural, or derivatized nucleotide bases. The backbone of the polynucleotide can comprise sugars and phosphate groups, as may typically be found in RNA or DNA, or modified or substituted sugar or phosphate groups. A polynucleotide may comprise modified nucleotides, such as methylated nucleotides and nucleotide analogs. The sequence of nucleotides may be interrupted by non-nucleotide components. Thus the terms nucleoside, nucleotide, deoxynucleoside and deoxynucleotide generally include analogs such as those described herein. These analogs are those molecules having some structural features in common with a naturally occurring nucleoside or nucleotide such that when incorporated into a nucleic acid or oligonucleotide sequence, they allow hybridization with a naturally occurring nucleic acid sequence in solution. Typically, these analogs are derived from naturally occurring nucleosides and nucleotides by replacing and/or modifying the base, the ribose or the phosphodiester moiety. The changes can be tailor made to stabilize or destabilize hybrid formation or enhance the specificity of hybridization with a complementary nucleic acid sequence as desired.

As used herein **"solid support", "support",** and **"substrate"** are used interchangeably and refer to a material or group of materials having a rigid or semi-rigid surface or surfaces. In many embodiments, at least one surface of the solid support will be substantially flat, although in some embodiments it may be desirable to physically separate synthesis regions for different compounds with, for example, wells, raised regions, pins, etched trenches, or the like. According to other embodiments, the solid support(s) will take the form of beads, resins, gels, microspheres, or other geometric configurations.

Any known nucleic acid arrays can be used in accordance with the present invention. For instance, such arrays include those based on short or longer oligonucleotide probes as well as cDNAs or polymerase chain reaction (PCR) products. Other methods include serial analysis of gene expression (SAGE), differential display, as well as subtractive hybridization methods, differential screening (DS), RNA arbitrarily primer (RAP)-PCR, restriction endonucleolytic analysis of differentially expressed sequences (READS), amplified restriction fragment-length polymorphisms (AFLP).

The present invention encompasses using antibodies for detecting or determining Gαi1 (e.g., having phosphorylated and/or unphosphorylated serine residues), Gαi2 (having e.g., having phosphorylated and/or unphosphorylated serine residues serine residues), Gai3 (e.g., having phosphorylated and/or unphosphorylated serine residues), Gαs levels for instance in the samples of a subject and for including in kits of the present invention. Antibodies that specifically bind to these biological markers can be produced routinely with methods further described above. The present invention also encompasses using antibodies commercially available. Without being so limited antibodies that specifically bind to Gαi1 (e.g., having phosphorylated and/or unphosphorylated serine residues), Gαi2 (e.g., having phosphorylated and/or unphosphorylated serine residues), Gαi3 (e.g., having phosphorylated and/or unphosphorylated serine residues), Gas include those listed in Table I below.

**Table I:**

| Commercially available antibodies for Gαi1 (e.g., having phosphorylated and/or unphosphorylated serine residues), Gαi2 (e.g., having phosphorylated and/or unphosphorylated serine residues), Gai3 (e.g., having phosphorylated and/or unphosphorylated serine residues), and Gas. | | | | | |
|---|---|---|---|---|---|
| **Description** | **Supplier** | **Catalogue Number** | **Reactivity** | **Application** | **Host** |
| G protein alpha inhibitor 1 antibody [7H7] | Abcam | ab136510 | Human, Rat | WB | Mouse |
| G protein alpha inhibitor 1 antibody [R4.5] | Abcam | ab140333 | Human, Mouse, Rat, Cow, Guinea pig | WB | Mouse |
| G protein alpha inhibitor 1 antibody | Abcam | ab102014 | Human | WB | Rabbit |
| G protein alpha inhibitor 1 antibody | Abcam | ab55103 | Human | IHC-P, WB | Mouse |
| G protein alpha inhibitor 1 antibody | Abcam | ab81447 | Human | ELISA, WB | Rabbit |
| G protein alpha inhibitor 1 antibody [SPM397] | Abcam | ab19932 | Human | WB | Mouse |
| G protein alpha inhibitor 1 antibody [2B8-2A5] | Abcam | ab118434 | Human | ELISA, IHC-P, WB | Mouse |
| G protein alpha inhibitor 1 antibody [EPR9441 (B)] | Abcam | ab140125 | Human, Mouse, Rat | Flow Cyt, ICC, IHC-P, WB | Rabbit |
| G protein alpha Inhibitor 2 antibody | Abcam | ab118578 | Human | IHC-P, WB | Rabbit |
| G protein alpha Inhibitor 2 antibody | Abcam | ab55117 | Human | WB | Mouse |
| G protein alpha Inhibitor 2 antibody | Abcam | ab81452 | Human | ELISA, WB | Rabbit |
| G protein alpha Inhibitor 2 antibody | Abcam | ab123427 | Human | WB | Rabbit |
| G Protein alpha Inhibitor 1+2 antibody | Abcam | ab3522 | Human, Mouse, Rat | IHC-P, WB | Rabbit |
| G protein alpha Inhibitor 2 antibody | Abcam | ab154155 | Human | ICC/IF, WB | Rabbit |
| G protein alpha Inhibitor 2 antibody [EPR9469] | Abcam | ab157204 | Human, Mouse, Rat | Flow Cyt, ICC/IF, IHC-P, WB | Rabbit |
| G protein alpha Inhibitor 2 antibody [EPR9468] | Abcam | ab137050 | Human, Mouse, Rat | Flow Cyt, WB | Rabbit |
| G protein alpha Inhibitor 2 antibody | Abcam | ab20392 | Human, Rat | ICC/IF, IP, WB | Rabbit |
| G protein alpha Inhibitor 2 antibody [L5] | Abcam | ab78193 | Human, Mouse, Rat, Cow, Guinea pig | WB | Mouse |
| GNAl1 Antibody (monoclonal) (M01) | Abgent | AT2225a | Human | WB,IHC,E | Mouse |
| GNAl1 polyclonal antibody (A01) | Abnova CorpoRation | H00002770-A01 | Human | ELISA, WB | Mouse |
| GNAl1 monoclonal antibody (M01), clone 2B8-2A5 | Abnova CorpoRation | H00002770-M01 | Human | WB-Tr,WB-Ti,S-ELISA,ELISA,WB-Re,IHC-P | Mouse |
| anti G protein alpha inhibitor 1 | Acris Antibodies GmbH | AM05302PU-N | Cow, Guinea Pig, Human, Mouse, Rat | WB | Mouse |
| anti G protein alpha inhibitor 1 (+ alpha 2) | Acris Antibodies GmbH | SP5158P | Human, Mouse, Rat | WB | Rabbit |
| anti G protein alpha inhibitor 1 | Acris Antibodies GmbH | AM12136PU-N | Cow, Guinea pig, Human, Mouse, Pig, Rat, Rat | P, WB | Mouse |
| anti G protein alpha inhibitor 1 | Acris Antibodies GmbH | AM20888PU-N | Human | E, P, WB | Mouse |
| anti G protein alpha inhibitor 1 (+ GNAI2) | Acris Antibodies GmbH | AP05163PU-N | Cow, Human, Mouse, Rat | WB | Rabbit |
| anti G protein alpha inhibitor 1 (+ GNAI2) | Acris Antibodies GmbH | AP09041PU-N | Cow, Human, Rat, Mouse | P, WB | Rabbit |
| anti G protein alpha inhibitor 1 | Acris Antibodies GmbH | AM32641SU-N | Cow, Guinea pig, Human, Mouse, Pig, Rat, Rat | WB | Mouse |
| anti G protein alpha inhibitor 1 (110-123) | Acris Antibodies GmbH | AM32537PU-N | VeRateb Rates | WB | Mouse |
| anti-G Protein Alpha Inhibitor 1/2 (GNAI1/GNAI2) (AA 345-354) antibody | antibodies-online | ABIN609015 | Human | WB, IHC | Rabbit |
| anti-Guanine Nucleotide Binding Protein (G Protein), alpha Inhibiting Activity Polypeptide 1 (GNAI1) antibody | antibodies-online | ABIN406409 | Cow (Cow),Human,Mouse (Murine),Rat (Rattus),Fruit Fly (Drosophila melanogaster),Pig (Porcine),Xenopus laevis,Chicken | WB | Rabbit |
| anti-Guanine Nucleotide Binding Protein (G Protein), alpha Inhibiting Activity Polypeptide 1 (GNAI1) antibody | antibodies-online | ABIN393287 | Human | ELISA, WB, IHC | Mouse |
| anti-Guanine Nucleotide Binding Protein (G Protein), alpha Inhibiting Activity Polypeptide 1 (GNAI1) (AA 345-354) antibody | antibodies-online | ABIN203567 | Human | ELISA | Rabbit |
| anti-Guanine Nucleotide Binding Protein (G Protein), alpha Inhibiting Activity Polypeptide 1 (GNAI1) antibody | antibodies-online | ABIN574258 | Human | ELISA, WB, IHC | Mouse |
| anti-Guanine Nucleotide Binding Protein (G Protein), alpha Inhibiting Activity Polypeptide 1 (GNAI1) (AA 110-123) antibody | antibodies-online | ABIN211172 | Human | WB | Mouse |
| anti-Guanine Nucleotide Binding Protein (G Protein), alpha Inhibiting Activity Polypeptide 3 (GNAI3) (C-Term) antibody | antibodies-online | ABIN470853 | Human | WB, IP | Rabbit |
| anti-Guanine Nucleotide Binding Protein (G Protein), alpha Inhibiting Activity Polypeptide 1 (GNAI1) antibody | antibodies-online | ABIN1010195 | Human,Rat,Mouse,Cow,C at ,Dog, Chicken | ELISA, WB, IHC | Rabbit |
| anti-Guanine Nucleotide Binding Protein (G Protein), alpha Inhibiting Activity Polypeptide 1 (GNAI1) (Internal Region) antibody | antibodies-online | ABIN322115 | Human | WB, ELISA | Rabbit |
| anti-G Protein Alpha Inhibitor 1/2 (GNAI1/GNAI2) (AA 346-355) antibody | antibodies-online | ABIN472931 | Human | WB | Rabbit |
| GNAl1 antibody - middle region (ARP54630_P050) | Aviva SysteMouse Biology | ARP54630_P0 50 | Human, Mouse, Rat, Drosophila, Pig, Cow, Fruit fly, African clawed frog, Chicken | WB | Rabbit |
| Rabbit Anti-G Protein alpha Inhibitor 1 Polyclonal Antibody | Bioss Inc. | bs-8556R | Human,Mouse,Rat, Cow, Dog | WB, ELISA, IHC-P, IHC-F | Rabbit |
| Rabbit Anti-G protein alpha Inhibitor 2 Polyclonal Antibody | Bioss Inc. | bs-9920R | Human,Mouse,Rat, Dog, Pig, Chicken, Cow, Sheep | WB,ELISA,IHC-P,IHC-F | Rabbit |
| Gα(i) Antibody | Cell Signaling Technology | 5290S | Human, Mouse, Rat | WB | Rabbit |
| Anti-G Protein Gialpha-1, clone R4 | EMD Millipore | MAB3075 | Cow, Guinea pig, Human, Mouse, Pig, Rat, Rat | WB | |
| Anti-Gialpha-2-Subunit, Internal (85-100) Rabbit pAb | EMD Millipore | 371727-50UL | Human, Mouse, Rat | ELISA, WB | Rabbit |
| Anti-G Protein Gialpha-2, clone L5 | EMD Millipore | MAB3077 | Cow, Guinea pig, Human, Mouse, Pig, Rat, Rat | WB | Mouse |
| G-Protein Subunit Antibody Set | EMD Millipore | 371770-1ST | N/A | WB | Rabbit |
| GNAl1 antibody | Fitzgerald Industries International | 70R-2047 | Human,Mouse, Rat, Droso phila | WB | Rabbit |
| Chicken Anti-Gai3 (G protein Gi alpha subunit 3) | GenWay Biotech, Inc. | 15-288-22489 | Human, Mouse, Rat | ICC, WB | Chicken |
| GNAI1 antibody - middle region | GenWay Biotech, Inc. | GWB-MT472D | Human Mouse Rat Drosophila Pig Cow Fruit fly African clawed frog Chicken | WB | Rabbit |
| Anti-Gi / GNAI1 | LifeSpan BioSciences | LS-B4333-50 | Human | ELISA, IHC-P, WB | Mouse |
| Anti-Gi / GNAI1 | LifeSpan BioSciences | LS-C26697-100 | Cow, Human, Rat | WB | Mouse |
| Anti-Gi / GNAI1 | LifeSpan BioSciences | LS-C87973-200 | Cow, Guinea pig, Human, Mouse, Pig, Rat, Rat | WB | Rat |
| Anti-Gi / GNAI1 | LifeSpan BioSciences | LS-B2546-50 | Rat, Cow, Guinea pig, Human, Mouse, Rat | IHC-P, WB | Mouse |
| Anti-Gi / GNAI1 | LifeSpan BioSciences | LS-C3237-100 | Human | ELISA | Rabbit |
| Anti-Gi / GNAI2 | LifeSpan BioSciences | LS-C87972-100 | Cow, Guinea pig, Human, Mouse, Pig, Rat, Rat | WB | |
| Anti-Gi / GNAI3 | LifeSpan BioSciences | LS-C23979-100 | Cow, Guinea pig, Mouse, Rat, Cow | WB | Mouse |
| Anti-Gi / GNAl1 | LifeSpan BioSciences | LS-C81891-50 | Cow, Chicken, Drosophila, Xenopus, Human, Mouse, Pig, Rat, Human | WB | |
| Anti-Gi / GNAl1 | LifeSpan BioSciences | LS-C23978-200 | Human | ELISA, WB | Mouse |
| Anti-G Protein Alpha Inhibitor 1/2 (GNAI1/GNAI2) | LifeSpan BioSciences | LS-C121228-100 | Human, Cow, Human, Mouse, Rat | IHC-P, WB | Rabbit |
| Anti-GNAI3 | LifeSpan BioSciences | LS-C23813-50 | Hamster, Human, Mouse, Rat | IP, WB | Rabbit |
| Anti-G Protein Gialpha-1, clone R4 | Merck Millipore | MAB3075 | Cow, Guinea pig, Human, Mouse, Pig, Rat, Rat | WB | |
| Anti-G Protein Gialpha 1/2 | Merck Millipore | 07-1500 | Human, Mouse, Rat, Cow | WB, IH(P) | |
| Anti-Gial pha-2-Subunit, Internal (85-100) Rabbit pAb | Merck Millipore | 371727-50UL | Human, Mouse, Rat | ELISA, WB | Rabbit |
| Rabbit anti-Human guanine nucleotide binding protein (G protein), alpha inhibiting activity polypeptide 1 polyclonal Antibody | MyBioSource. com | MBS716824 | Human, Mouse, Rat. | ELISA, WB, IHC | Rabbit |
| G protein alpha inhibitor 1 Antibody | Novus Biologicals | NBP1-52926 | Human, Mouse, Rat, Cow, Chicken, Drosophila, Porcine, Xenopus | WB | Rabbit |
| G protein alpha inhibitor 1 Antibody | Novus Biologicals | H00002770-A01 | Human | WB, ELISA | Mouse |
| G protein alpha inhibitor 1 Antibody | Novus Biologicals | NBP1-31601 | Human | WB | Rabbit |
| G protein alpha inhibitor 1 Antibody (2B8-2A5) | Novus Biologicals | H00002770-M01 | Human | WB, ELISA, IHC | Mouse |
| G protein alpha inhibitor 1 Antibody | Novus Biologicals | NBP2-16558 | Human | WB, IHC | Rabbit |
| G protein alpha inhibitor 1 Antibody (R4.5) | Novus Biologicals | NBP1-40247 | Human, Mouse, Rat, Cow, Guinea Pig | WB, IHC | Mouse |
| G protein alpha inhibitor 1/2 Antibody | Novus Biologicals | NB120-3522 | Human, Mouse, Rat | WB, IP | Rabbit |
| GNAI1 | Proteintech Group Inc | 12617-1-AP | Human, Mouse, Rat | ELISA, WB, IHC | Rabbit |
| Galpha i-1 (SPM397) | Santa Cruz Biotechnology, Inc. | sc-56536 | Mouse, Rat, Human, cow | WB, IP | Mouse |
| Galpha i-1 (R4) | Santa Cruz Biotechnology, Inc. | sc-13533 | Mouse, Rat, Human, cow | WB, IP, IF, IHC(P) | Mouse |
| Galpha i-1/3 (I-18) | Santa Cruz Biotechnology, Inc. | sc-26762 | Mouse, Rat, Human | WB, IF, ELISA | Goat |
| Galpha i-1 (I-20) | Santa Cruz Biotechnology, Inc. | sc-391 | Mouse, Rat, Human | WB, IF, ELISA | Rabbit |
| Galpha i-3 (C-10) | Santa Cruz Biotechnology, Inc. | sc-262 | Mouse, Rat, Human | WB, IP, IF, ELISA | Rabbit |
| Galpha i-3 (H-7) | Santa Cruz Biotechnology, Inc. | sc-365422 | Mouse, Rat, Human | WB, IP, IF, ELISA | Mouse |
| Galpha i-1/2/3 (N-20) | Santa Cruz Biotechnology, Inc. | sc-26761 | Mouse, Rat, Human | WB, IP, IF, ELISA | Goat |
| Galpha i/o/t/z/gust (H-300) | Santa Cruz Biotechnology, Inc. | sc-28586 | Mouse, Rat, Human | WB, IP, IF, ELISA | Rabbit |
| Galpha i/o/t/z (D-15) | Santa Cruz Biotechnology, Inc. | sc-12798 | Mouse, Rat, Human | WB, IP, IF, ELISA | Goat |
| G protein alpha Inhibitor 1 Antibody | Thermo Scientific Pierce Antibodies | PA5-30043 | Human | IHC (P), WB | Rabbit |
| G protein alpha inhibitor 1 Antibody | Thermo Scientific Pierce Antibodies | PA5-28223 | Human | WB | Rabbit |
| Gia-1 Antibody | Thermo Scientific Pierce Antibodies | MA1-12610 | Cow, Guinea pig, Human, Mouse, Pig, Rat, Rat | WB | Mouse |
| Galphai1 G-Protein Antibody | Thermo Scientific Pierce Antibodies | MA5-12800 | Cow, Guinea pig, Human, Mouse, Pig, Rat, Rat | WB | Mouse |
| G protein alpha Inhibitor 2 Antibody | Thermo Scientific Pierce Antibodies | PA5-27496 | Human, Mouse | WB | Rabbit |
| G protein alpha Inhibitor 2 Antibody | Thermo Scientific Pierce Antibodies | PA5-27520 | Human | IF, WB | Rabbit |
| | | | | | |
| G protein alpha S antibody | Abcam | ab97629 | Human | WB | Rabbit |
| G protein alpha S antibody | Abcam | ab83735 | Human, Mouse | IHC-P, WB | Rabbit |
| G protein alpha S antibody | Abcam | ab101736 | Human, Mouse, Rat | WB | Goat |
| G protein alpha S antibody | Abcam | ab97663 | Human, Mouse | IHC-P, WB | Rabbit |
| GNAS Antibody (Center) | Abgent | AP6865c | Human | WB, ELISA | Rabbit |
| GNAS Antibody (C-term) | Abgent | AP13065b | Human | WB,IHC,E | Rabbit |
| GNAS purified MaxPab rabbit polyclonal antibody (D01P) | Abnova Corporation | H00002778-D01P | Human | PLA-Ce,Det Ab,WB-Tr | Rabbit |
| GNAS monoclonal antibody | Abnova Corporation | H00002778-M | Human | WB,ELISA | Mouse |
| GNAS polyclonal antibody | Abnova Corporation | PAB18557 | Human | ELISA,WB-Ce | Goat |
| anti GNAS / GSP | Acris Antibodies GmbH | AP20123PU-N | Human, Cow, Mouse, Rat | WB, ELISA | Goat |
| anti-Galpha S (AA 11-21) antibody | antibodies-online | ABIN968909 | Human | WB | Mouse |
| anti-GNAS Complex Locus (GNAS) antibody | antibodies-online | ABIN571171 | Human | ELISA, WB | Goat |
| anti-GNAS Complex Locus (GNAS) antibody | antibodies-online | ABIN1086769 | Human | WB, ELISA | Rabbit |
| anti-GNAS Complex Locus (GNAS) antibody | antibodies-online | ABIN604875 | Human | WB, IHC | Rabbit |
| anti-GNAS Complex Locus (GNAS) (Transcript Variant 1) antibody | antibodies-online | ABIN1035879 | Human, Rat, Mouse | WB, IHC, ELISA | Rabbit |
| anti-GNAS Complex Locus (GNAS) (AA 385-394) antibody | antibodies-online | ABIN213649 | Human | ELISA, IH | Rabbit |
| anti-GNAS Complex Locus (GNAS) (C-Term) antibody | antibodies-online | ABIN656989 | Human | WB, ELISA | Rabbit |
| anti-GNAS Complex Locus (GNAS) (N-Term) antibody | antibodies-online | ABIN203445 | Human | IH, WB | Rabbit |
| GNAS antibody - N-terminal region (ARP42693_P050) | Aviva Systems Biology | ARP42693_PO50 | Human, Mouse, Rat, Dog, Bovine, Pig | WB, IHC | Rabbit |
| GNAS antibody - N-terminal region (ARP41764 _ T100) | Aviva Systems Biology | ARP41764_T1 | Human, Dog, Mouse, Rat, Pig, Rabbit, Cow | WB | Rabbit |
| Anti-GNAS | Biomatik | CAE21174 | Human | Pep-ELISA, WB | Goat |
| G protein alpha S antibody | Biorbyt | orb6103 | Human, Mouse, Rat | P-ELISA, WB, IHC-P | Rabbit |
| GNAS antibody | Biorbyt | orb31105 | Human | WB, ELISA | Rabbit |
| GNAS antibody | Biorbyt | orb37122 | Human | WB, ELISA | Rabbit |
| GNAS antibody | Biorbyt | orb20551 | Human, Mouse, Rat, Cow | ELISA, WB | Goat |
| Rabbit Anti-G protein alpha S Polyclonal Antibody | Bioss Inc. | bs-3939R | Human,Mouse,Rat, Dog, Pig, Horse, Chicken, Cow, Rabbit | WB, ELISA, IP,IHC-P,IHC-F,IF | Rabbit |
| Anti-Gsalpha | EMD Millipore | 06-237 | Cow, Human, Mouse | IC, IP, WB | Rabbit |
| GNAS antibody | Fitzgerald Industries International | 70R-12940 | Human, Mouse | IHC-P, WB | Rabbit |
| GNAS antibody | Fitzgerald Industries International | 70R-1651 | Human, Mouse, Rat, Dog | WB | Rabbit |
| GNAS antibody | Fitzgerald Industries International | 70R-1652 | Human | WB, IHC | Rabbit |
| GNAS antibody | Fitzgerald Industries International | 70R-5756 | Human, Mouse, Rat, Dog | WB | Rabbit |
| GNAS antibody | Fitzgerald Industries International | 70R-1643 | Human,Dog | WB | Rabbit |
| GNAS antibody [C1C3] | GeneTex | GTX113338 | NA | WB-Ag | Rabbit |
| Rabbit Anti-Human GNAS Polyclonal Antibody, Unconjugated | GenWay Biotech, Inc | 18-003-43749 | Human | Immunohistochemistry-P, Western Blot | Rabbit |
| GNAS antibody - N-terminal region | GenWay Biotech, Inc. | GWB-MP242H | Human, Mouse, Rat, Dog, Cow, Pig | WB | Rabbit |
| GNAS antibody - N-terminal region | GenWay Biotech, Inc. | GWB-MN943G | Human, Dog, Mouse, Rat, Pig, Rabbit, Cow | WB | Rabbit |
| Rabbit Anti-GNAS Polyclonal Antibody, Unconjugated | GenWay Biotech, Inc. | 18-003-44268 | Human, Mouse, Rat, Dog | WB | Rabbit |
| Anti-GNAS | LifeSpan BioSciences | LS-B102-50 | Human, Primate, Human | ELISA, IHC-P | Rabbit |
| Anti-GNAS | LifeSpan BioSciences | LS-B4790-50 | Human, Primate, Human | ELISA, IHC-P | Rabbit |
| Anti-GNAS | LifeSpan BioSciences | LS-B4007-50 | Human, Bovine, Dog, Human, Mouse, Pig, Rat | IHC-P, WB | Rabbit |
| Anti-GNAS | LifeSpan BioSciences | LS-C31914-100 | Bovine, Human, Mouse, Pig, Rabbit, Rat, Human | IHC, WB | Rabbit |
| Anti-GNAS | LifeSpan BioSciences | LS-C113002-100 | Human, Mouse, Rat, Human | ELISA, WB | Goat |
| Anti-GNAS | LifeSpan BioSciences | LS-C80456-100 | Bovine, Dog, Human, Mouse, Pig, Rabbit, Rat, Human | WB | Rabbit |
| Anti-Gs protein, alpha subunit, clone N192/12 | Merck Millipore | MABN543 | Human, Rat | WB | Mouse |
| GNAS Antibody | ProSci, Inc | 42-594 | Human | ELISA, WB | Goat |
| GNAS Antibody | ProSci, Inc | 29-639 | Human, Dog | WB, ELISA | Rabbit |
| GNAS antibody | ProSci, Inc | 25-797 | Human, Mouse, Rat, Dog | ELISA, Western Blot | Rabbit |
| GNAS Antibody | ProSci, Inc | 29-791 | Human | ELISA, Western Blot, Immunohistochemistry | Rabbit |
| GNAS Antibody | ProSci, Inc | 29-640 | Human, Mouse, Rat, Dog | ELISA, Western Blot | Rabbit |
| Galpha s (C-19) | Santa Cruz Biotechnology, Inc. | sc-46976 | Mouse, Rat, Human | WB, IF, ELISA | Goat |
| Galpha s (12) | Santa Cruz Biotechnology, Inc. | sc-135914 | Mouse, Rat, Human | WB, IP | Mouse |
| Galpha 12 (S-20) | Santa Cruz Biotechnology, Inc. | sc-409 | Mouse, Rat, Human | WB , IP, IF, IHC(P), ELISA | Rabbit |
| Galpha s (C-17) | Santa Cruz Biotechnology, Inc. | sc-46975 | Mouse, Rat, Human | WB , IP, IF, IHC(P), ELISA | Goat |
| Galpha s (A-16) | Santa Cruz Biotechnology, Inc. | sc-26766 | Mouse, Rat, Human | WB, IP, IF, ELISA | Goat |
| Galpha s (K-20) | Santa Cruz Biotechnology, Inc. | sc-823 | Mouse, Rat, Human | WB, IP, IF, IHC(P), ELISA | Rabbit |
| Galpha s/olf (E-7) | Santa Cruz Biotechnology, Inc. | sc-55546 | Mouse, Rat, Human | WB, IP, IF, IHC(P), ELISA | Mouse |
| Galpha s/olf (A-5) | Santa Cruz Biotechnology, Inc. | sc-55545 | Mouse, Rat, Human | WB, IP, IF, IHC(P), ELISA | Mouse |
| Galpha s/olf (C-18) | Santa Cruz Biotechnology, Inc. | sc-383 | Mouse, Rat, Human | WB, IP, IF, ELISA | Rabbit |
| Galpha s/olf (G-10) | Santa Cruz Biotechnology, Inc. | sc-365855 | Mouse, Rat, Human | WB, IP, IF, ELISA | Mouse |
| Galpha s/olf (H-300) | Santa Cruz Biotechnology, Inc. | sc-28585 | Mouse, Rat, Human | WB, IP, IF, ELISA | Rabbit |
| Galpha s/olf (C-10) | Santa Cruz Biotechnology, Inc. | sc-377435 | Mouse, Rat, Human | WB, IP, IF, ELISA | Mouse |
| GNAS Antibody | Thermo Scientific Pierce Antibodies | PA5-19315 | Human | WB | Goat |

The present description also describes arrays to detect and/or quantify the translation products of PKA (*e.g.,* PKA-γ2), PKC (*e.g.,* PKC-η or PKC-ε), CaMK1 (*e.g.,* CaMK1δ), CaMK4, CK (*e.g.,* CK2), CaMK2, Giα1, Giα2, Giα3 or Gsα. Such arrays include protein micro- or macroarrays, gel technologies including high-resolution 2D-gel methodologies, possibly coupled with mass spectrometry imaging system at the cellular level such as microscopy combined with a fluorescent labeling system.

The present description also describes methods to screen/select for potential useful therapeutic agents using whole cells assays, the therapeutic compound being able to decrease the transcription and/or synthesis and/or activity (*e.g.,* phosphorylation of Gi proteins) of PKA (*e.g.,* PKA-γ2), PKC (*e.g.,* PKC-η or PKC-ε), CaMK1 (*e.g.,* CaMK1δ), CaMK4, CK (*e.g.,* CK2), CaMK2, Giα1, Giα2, Giα3 or Gsα. Cells for use in such methods includes cells of any source (including in house or commercially available cell lines) and type (any tissue). In house cell lines could be made for instance by immortalizing cells from AIS subjects. In specific embodiments, methods of screening of the invention seek to identify agents that inhibit the PKA (*e.g.,* PKA-γ2), PKC (*e.g.,* PKC-η or PKC-ε), CaMK1 (*e.g.,* CaMK1δ), CaMK4, CK (*e.g.,* CK2), CaMK2, Giα1, Giα2, Giα3 or Gsα expression (transcription and/or translation). Useful cell lines for these embodiments, not part of the invention, include those producing high levels of PKA (*e.g.,* PKA-γ2), PKC (*e.g.,* PKC-η or PKC-ε), CaMK1 (*e.g.,* CaMK1δ), CaMK4, CK(*e.g*., CK2), CaMK2, Giα1, Giα2, Giα3 or Gsα. They include osteoblasts, PMBcs and myoblasts.

It includes cells of any cell type derived from a scoliotic patient (whole cell assay). In specific embodiments, not part of the invention, it includes osteoblasts, chondrocytes, myoblasts or blood cells including PBMCs including lymphocytes. As used herein, the term **"cell derived from a scoliotic patient"** refers to cells isolated directly from scoliotic patients, or immortalized cell lines originating from cells isolated directly from scoliotic patients. In specific embodiments, the cells are paraspinal muscle cells. Such cells may be isolated by a subject through needle biopsies for instance.

Pharmaceutical compositions can also be administered by routes such as nasally, intravenously, intramuscularly, subcutaneously, sublingually, intrathecally, or intradermally. The route of administration can depend on a variety of factors, such as the environment and therapeutic goals.

### Dosage

Any amount of a pharmaceutical and/or nutraceutical and/or dietary supplement compositions can be administered to a subject. The dosages will depend on many factors including the mode of administration. Typically, the amount of anti-scoliosis composition (e.g., agent that decrease the transcription and/or synthesis and/or activity (*e.g.,* phosphorylation of Gi proteins) and/or stability of PKA (*e.g.,* PKA-γ2), PKC (*e.g.,* PKC-η or PKC-ε), CaMK1 (*e.g.,* CaMK1δ), CaMK4, CK (*e.g.,* CK2), or CaMK2 contained within a single dose will be an amount that effectively prevents, delays or reduces scoliosis without inducing significant toxicity "therapeutically effective amount".

The effective amount of the agent that agent that decrease the transcription and/or synthesis and/or activity (*e.g.,* phosphorylation of Gi proteins) and/or stability of PKA (*e.g.,* PKA-γ2), PKC (*e.g.,* PKC-η or PKC-ε), CaMK1 (*e.g.,* CaMK1δ), CaMK4, CK (*e.g.,* CK2), or CaMK2 may also be measured directly. The effective amount may be given daily or weekly or fractions thereof. Typically, a pharmaceutical and/or nutraceutical and/or dietary supplement composition of the invention can be administered in an amount from about 0.001 mg up to about 500 mg per kg of body weight per day (e.g., 10 mg, 50 mg, 100 mg, or 250 mg). Dosages may be provided in either a single or multiple dosage regimen. For example, in some embodiments the effective amount is a dose that ranges from about 1 mg to about 25 grams of the anti-scoliosis preparation per day, about 50 mg to about 10 grams of the anti- scoliosis preparation per day, from about 100 mg to about 5 grams of the anti- scoliosis preparation per day, about 1 gram of the anti- scoliosis preparation per day, about 1 mg to about 25 grams of the anti- scoliosis preparation per week, about 50 mg to about 10 grams of the anti- scoliosis preparation per week, about 100 mg to about 5 grams of the anti- scoliosis preparation every other day, and about 1 gram of the anti- scoliosis preparation once a week.

By way of example, a pharmaceutical (e.g., containing an agent that decreases the transcription and/or synthesis and/or activity (*e.g.,* phosphorylation of Gi proteins) and/or stability of PKA (*e.g.,* PKA-γ2), PKC (*e.g.,* PKC-η or PKC-ε), CaMK1 (*e.g.,* CaMK1δ), CaMK4, CK (*e.g.,* CK2), or CaMK2 composition of the invention can be in the form of a liquid, solution, suspension, pill, capsule, tablet, gelcap, powder, gel, ointment, cream, nebulae, mist, atomized vapor, aerosol, or phytosome. For oral administration, tablets or capsules can be prepared by conventional means with at least one pharmaceutically acceptable excipient such as binding agents, fillers, lubricants, disintegrants, or wetting agents. The tablets can be coated by methods known in the art. Liquid preparations for oral administration can take the form of, for example, solutions, syrups, or suspension, or they can be presented as a dry product for constitution with saline or other suitable liquid vehicle before use. Preparations for oral administration also can be suitably formulated to give controlled release of the active ingredients.

In addition, a pharmaceutical (e.g., containing an agent that decreases the transcription and/or synthesis and/or activity (*e.g.,* phosphorylation of Gi proteins) and/or stability of PKA (*e.g.,* PKA-γ2), PKC (*e.g.,* PKC-η or PKC-ε), CaMK1 (*e.g.,* CaMK1δ), CaMK4, CK (*e.g.,* CK2), or CaMK2 composition of the invention can contain a pharmaceutically acceptable carrier for administration to a mammal, including, without limitation, sterile aqueous or non-aqueous solutions, suspensions, and emulsions. Examples of non-aqueous solvents include, without limitation, propylene glycol, polyethylene glycol, vegetable oils, and injectable organic esters. Aqueous carriers include, without limitation, water, alcohol, saline, and buffered solutions. Pharmaceutically acceptable carriers also can include physiologically acceptable aqueous vehicles (*e.g.,* physiological saline) or other known carriers appropriate to specific routes of administration.

An agent that decreases the transcription and/or synthesis and/or stability and/or activity (*e.g.,* phosphorylation of Gi proteins) and/or stability of PKA (*e.g.,* PKA-γ2), PKC (*e.g.,* PKC-η or PKC-ε), CaMK1 (*e.g.,* CaMK1δ), CaMK4, CK (*e.g.,* CK2), or CaMK2 may be incorporated into dosage forms in conjunction with any of the vehicles which are commonly employed in pharmaceutical preparations, e.g. talc, gum arabic, lactose, starch, magnesium stearate, cocoa butter, aqueous or non-aqueous solvents, oils, paraffin derivatives or glycols. Emulsions such as those described in U.S. Pat. No. 5,434,183, may also be used in which vegetable oil (e.g., soybean oil or safflower oil), emulsifying agent (e.g., egg yolk phospholipid) and water are combined with glycerol. Methods for preparing appropriate formulations are well known in the art (see e.g., Remington's Pharmaceutical Sciences, 16th Ed., 1980, A. Oslo Ed., Easton, Pa.).

In cases where parenteral administration is elected as the route of administration, preparations containing an agent that decreases the transcription and/or synthesis and/or activity (*e.g.,* phosphorylation of Gi proteins) and/or stability of PKA (*e.g.,* PKA-γ2), PKC (*e.g.,* PKC-η or PKC-ε), CaMK1 (*e.g.,* CaMK1δ), CaMK4, CK (*e.g.,* CK2), or CaMK2 may be provided to patients in combination with pharmaceutically acceptable sterile aqueous or non-aqueous solvents, suspensions or emulsions. Examples of non-aqueous solvents are propylene glycol, polyethylene glycol, vegetable oil, fish oil, and injectable organic esters. Aqueous carriers include water, water-alcohol solutions, emulsions or suspensions, including saline and buffered medical parenteral vehicles including sodium chloride solution, Ringer's dextrose solution, dextrose plus sodium chloride solution, Ringer's solution containing lactose, or fixed oils. Intravenous vehicles may include fluid and nutrient replenishers, electrolyte replenishers, such as those based upon Ringer's dextrose, and the like.

These are simply guidelines since the actual dose must be carefully selected and titrated by the attending physician based upon clinical factors unique to each patient or by a nutritionist. The optimal daily dose will be determined by methods known in the art and will be influenced by factors such as the age of the patient and other clinically relevant factors. In addition, patients may be taking medications for other diseases or conditions. The other medications may be continued during the time that the agent that decreases the transcription and/or synthesis and/or activity (*e.g.,* phosphorylation of Gi proteins) and/or stability of PKA (*e.g.,* PKA-γ2), PKC (*e.g.,* PKC-η or PKC-ε), CaMK1 (*e.g.,* CaMK1δ), CaMK4, CK (*e.g.,* CK2), or CaMK2 is given to the patient, but it is particularly advisable in such cases to begin with low doses to determine if adverse side effects are experienced.

The present description also relates to kits for stratifying scoliotic subjects and/or predicting whether a subject is at risk of developing a scoliosis comprising an isolated nucleic acid, a protein or a ligand such as an antibody in accordance with the present invention as described above. For example, a compartmentalized kit in accordance with the present invention includes any kit in which reagents are contained in separate containers. Such containers include small glass containers, plastic containers or strips of plastic or paper. Such containers allow the efficient transfer of reagents from one compartment to another compartment such that the samples and reagents are not cross-contaminated and the agents or solutions of each container can be added in a quantitative fashion from one compartment to another. Such containers will include a container which will accept the subject sample (DNA genomic nucleic acid, cell sample or blood samples), a container which contains in some kits of the present description, the probes used in the methods of the present invention, containers which contain enzymes, containers which contain wash reagents, and containers which contain the reagents used to detect the extension products. Kits of the present description may also contain instructions to use these probes and or antibodies to stratify scoliotic subjects or predict whether a subject is at risk of developing a scoliosis.

The articles **"a," "an"** and **"the"** are used herein to refer to one or to more than one (i.e., to at least one) of the grammatical object of the article.

The term **"including"** and **"comprising"** are used herein to mean, and are used interchangeably with, the phrases "including but not limited to" and "comprising but not limited to".

The terms **"such as"** are used herein to mean, and is used interchangeably with, the phrase "such as but not limited to".

The term **"about"** is used to indicate that a value includes the standard deviation of error for the device or method being employed to determine the value. In general, the terminology "about" is meant to designate a possible variation of up to 10%. Therefore, a variation of 1, 2, 3, 4, 5, 6, 7, 8, 9 and 10% of a value is included in the term "about".

As used herein, the term **"purified"** or **"isolated"** refers to a molecule (e.g., polynucleotide or polypeptide) having been separated from a component of the composition in which it was originally present. Thus, for example, an "isolated polynucleotide" or "isolated polypeptide" has been purified to a level not found in nature. A **"substantially pure"** molecule is a molecule that is lacking in most other components (e.g., 30, 40, 50, 60, 70, 75, 80, 85, 90, 95, 96, 97, 98, 99, 100% free of contaminants). By opposition, the term **"crude"** means molecules that have not been separated from the components of the original composition in which it was present. For the sake of brevity, the units (e.g., 66, 67...81, 82, 83, 84, 85,...91, 92%....) have not been specifically recited but are considered nevertheless within the scope of the present invention.

The present invention is illustrated in further details by the following non-limiting examples.

### EXAMPLE 1

### Materials and Methods

### French-Canadian patients (Montreal's cohort)

This study was approved by the institutional review boards of The Sainte-Justine University Hospital, The Montreal Children's Hospital, and The Shriners Hospital for Children. Three populations including children with AIS, families of children with AIS and control subjects were enrolled in the study. Healthy children recruited in Montreal's elementary schools and Trauma cases were used as controls. The recruitment was approved by the Montreal English school Board, The Affluent School Board and all institutional review Board mentioned above. Parents or legal guardians of all participants with or without AIS gave their informed written consent, and minors gave their assent. All participants were examined by one of the seven orthopedic surgeons (H.L., B.P., C-H.R., G.G., J.O., M.B-B., S.P.) participating in this study.

### Italian patients (Milano's cohort)

A total of 139 consecutive AIS patients and 103 controls subjects were enrolled at the IRCCS Istituto Ortopedico Galeazzi (Milano, Italy) and represent a replication cohort. This replication study was approved by the institutional review board of IRSCC and The Sainte-Justine University Hospital. All patients were examined by one orthopedic surgeon (M.B-B).

### Cell preparation and culture

Osteoblasts were isolated from bone specimens obtained intraoperatively from vertebras (varying from T3 to L4 according to the surgical procedure performed) and from other anatomical sites (tibia or femur) in AIS patients and trauma control cases, respectively as previously described (Moreau et al., 2004).

Myoblasts were isolated from biopsy specimen of skeletal muscle obtained from AIS and trauma control patients. Each biopsy specimen was cleared of remains fatty and connective tissue prior to being cut into smaller pieces. The tissue pieces were then transferred into PBS solution containing 0.01% collagenase, and digested for 45 min at 37 °C. After dilution with culture media (1:1), the solution was filtered in sterile conditions through a nylon filter of 45 µm prior to the centrifugation at 280 x g for 5 min at room temperature. The pallet containing myoblasts was suspended in culture media (alpha-MEM) supplemented with 20% foetal bovine serum (certified FBS; Invitrogen, Burlington, ON, Canada), and 1% penicillin/streptomycin (Invitrogen). After two weeks, culture media was replaced by fresh culture media supplemented with 10% FBS and 1% penicillin/ streptomycin and myoblasts were allowed to grow until confluence.

Peripheral blood mononuclear cells (PBMC) were extracted from blood obtained from patients and control groups, as previously described (Akoume et al., 2010).

### Functional classification

Patients were classified by evaluating the functional status of melatonin signaling in PBMC with cellular dielectric spectroscopy (CDS), using CellKey™ apparatus, as previously described (Akoume et al., 2010 and WO 2010/040234, 2010 to Moreau et al.). The impedance that reflects the cellular changes resulting from ligand/receptor interaction was measured for 15 minutes to monitor the cellular response to 14⁻⁴M iodomelatonin stimulation. The classification was achieved according to the following value ranges of impedance: between 0 and 40 ohms for FG1, between 40 and 80 ohms for FG2 and between 80 and 120 ohms for FG3. All control cases exhibiting a response extent of less than 120 ohms were excluded from the study.

### Functional evaluation of G proteins

The functionality of Gi, Gs and Gq proteins was evaluated by CDS assay in osteoblasts, and the functionality of Gi was also evaluated by CDS assay in myoblasts and PBMCs from the same individuals, using CellKey™ apparatus, as previously described (Akoume et al., 2010 and WO 2010/040234, 2010 to Moreau et al.).

### RNA interference

All Gi₁, Gi₂, Gi₃, Gs and scrambled siRNA were obtained from Ambion (Ambion USA). The sequences used for gene silencing are shown in **Table II** below. Osteoblasts from control subjects and AIS patients were transiently transfected in serum-free medium, using Lipofectamine RNAiMAX™ reagent (Invitrogen) according to the manufacturer's instructions and functional experiments were performed 48h post transfection. The gene knockdown was evaluated by quantitative real-time PCR (qPCR).

**Table II: SiRNA sequences for Gi₁, Gi₂, Gi₃, Gs**

| **Gene Symbol** | **SiRNA sequence** | **Cat. Number** |
|---|---|---|
| GNAi1 | GAGAUUGUGGAAAGAUAGUGGUGUA (SEQ ID NO: 97) | 1299003 |
| GNAi2 | GAGGACCUGAAUAAGCGCAAAGACA (SEQ ID NO: | 1299003 |
| GNAi3 | UCAGCUCAAUGAUUCUGCUUCAUAU (SEQ ID NO: 99) | 1299003 |
| GNAS | ACAACAUGGUCAUCCGGGAGGACAA (SEQ ID NO: 100) | 1299003 |

### Quantitative real-time PCR

RNA was isolated from osteoblasts using TRIzol™ reagent (Invitrogen) according to the manufacturer's protocol. Total RNA (1 µg) was reverse-transcribed into cDNA using Tetro™ cDNA synthesis Kit (Bioline). Following cDNA synthesis, qPCR was performed using a PCR master mix containing QuantiTect™ SYBR Green PCR Master Mix (QIAGEN, Ontario, Canada). Transcript expression was assessed with the Stratagene™ Mx3000P (Agilent Technologies, La Jolla, CA) and calculations were performed according to the ΔΔCT method using β-actin as internal control. The sequences of the forward and reverse primers used for identification of human mRNA of our interest genes are shown in **Table III.**

**Table III: List of the forward and reverse sequences**

| | **Forwards** | **Reverses** |
|---|---|---|
| Gi1 | AGGGCTATGGGGAGGTTGAAGAT (SEQ ID NO : 1) | ACTCCAGCAAGTTCTGCAGTCA (SEQ ID NO : 2) |
| Gi2 | AGGGAATACCAGCTCAACGACTCA (SEQ ID NO : 3) | TGTGTGGGGATGTAGTCACTCTGT (SEQ ID NO : 4) |
| Gi3 | GAGAGTGAAGACCACAGGCATT (SEQ ID NO : 5) | CGTTCTGATCTTTGGCCACCTA (SEQ ID NO : 6) |
| Gs | GAGACCAAGTTCCAGGTGGACA (SEQ ID NO : 7) | GATCCACTTGCGGCGTTCAT (SEQ ID NO : 8) |
| Gq | ATCAGAACATCTTCACGGCC (SEQ ID NO : 9) | AAAGCAGACACCTTCTCCAC (SEQ ID NO : 10) |
| PKC-α (PKCA) | CGGAATGGATCACACTGAGAAG (SEQ ID NO: 11) | ACATAAGGATCTGAAAGCCCG (SEQ ID NO: 12) |
| PKC-β (PKCB) | TTCCCGATCCCAAAAGTGAG (SEQ ID NO : 13) | GTCAAATCCCAATCCCAAATCTC (SEQ ID NO : 14) |
| PKC-δ (PKCD) | GCTTCAAGGTTCACAACTACATG (SEQ ID NO : 15) | ACCTTCTCCCGGCATTTATG (SEQ ID NO : 16) |
| PKC-ε (PKCE) | CCTACCTTCTGCGATCACTG (SEQ ID NO : 17) | TACTTTGGCGATTCCTCTGG (SEQ ID NO: 18) |
| PKC-η (PKCHt) | GTAAATGCGGTGGAACTTGC (SEQ ID NO: 19) | ACCCCAATCCCATTTCCTTC (SEQ ID NO : 20) |
| PKC-I (PKCI) | GAGAAGCATGTGTTTGAGCAG (SEQ ID NO: 21) | GGAAGTTTTCTTTGTCGCTGC (SEQ ID NO : 22) |
| PKC-γ (Gamma) | ACGAAGTCAAGAGCCACAAG (SEQ ID NO : 23) | GTCGATGAACCACAAAGCTG (SEQ ID NO : 24) |
| PKC-θ (PKCQ) | CGGATTTTGGAATGTGCAAGG (SEQ ID NO : 25) | CATAAAGGAGAACCCCGAAGG (SEQ ID NO : 26) |
| PKC-ζ (PKCZ) | TGCTTACATTTCCTCATCCCG (SEQ ID NO : 27) | CGCCCGATGACTCTGATTAG (SEQ ID NO : 28) |
| CaMK1-δ (CaMK1D) | AATGGAGGGCAAAGGAGATG (SEQ ID NO : 29) | AAGATGTAGGCAATCACTCCG (SEQ ID NO : 30) |
| CaMK1-γ (CaMK1G) | CTTGAGAAGGATCCGAACGAG (SEQ ID NO: 31) | TTGCCTCCACTTGCTCTTAG (SEQ ID NO : 32) |
| CaMK2-α (CaMK2A) | CAGTTCCAGCGTTCAGTTAATG (SEQ ID NO : 33) | TTCGTGTAGGACTCAAAATCTCC (SEQ ID NO : 34) |
| CaMK2-β (CaMK2B) | CTCTGACATCCTGAACTCTGTG (SEQ ID NO : 35) | CCGTGGTCTTAATGATCTCCTG (SEQ ID NO : 36) |
| CaMK2-δ (CaMK2D | GGCACACCTGGATATCTTTCTC (SEQ ID NO : 37) | AGTCTGTGTTGGTCTTCATCC (SEQ ID NO : 38) |
| CaMK2-γ (CaMK2G) | AAACAGTCTCGTAAGCCCAG (SEQ ID NO : 39) | ATCCCATCTGTAGCGTTGTG (SEQ ID NO : 40) |
| CaMK4 | TCGCCTCTCACATCCAAAC (SEQ ID NO: 41) | CATCTCGCTCACTGTAATATCCC (SEQ ID NO : 42) |
| CaMK2n1 | AGGACACCAACAACTTCTTCG (SEQ ID NO : 43) | GGTGCCTTGTCGGTCATATT (SEQ ID NO : 44) |
| PKA-α1 | CTCAGTTCCTGGAGAAAGATGG (SEQ ID NO : 45) | CCCAGTCAATTCATGTTTGCC (SEQ ID NO : 46) |
| PKA-α2 | ATGGAATATGTGTCTGGAGGTG (SEQ ID NO : 47) | TGGTTTCAGGTCTCGATGAAC (SEQ ID NO : 48) |
| PKA-β1 | GAGTAAACTTCCCCTCACCAG (SEQ ID NO : 49) | TCCACTGACCATCCACAAAG (SEQ ID NO : 50) |
| PKA-β2 | CACTGTTATCCGCTGGTCTG (SEQ ID NO: 51) | CTTGTATTGGTGCTCTCCCTC (SEQ ID NO : 52) |
| PKA-cα | CAAGGACAACTCAAACTTATACATGG (SEQ ID NO : 53) | CAGATACTCAAAGGTCAGGACG (SEQ ID NO : 54) |
| PKA-cβ | CCTTTCCTTGTTCGACTGGAG (SEQ ID NO : 55) | TGAGCTGCATAGAACCGTG (SEQ ID NO : 56) |
| PKA-cγ | CCGGATCTCCATCAATGAGAAG (SEQ ID NO : 57) | TTCAATCCAACCCTCCCATC (SEQ ID NO : 58) |
| PKA-γ1 | GCGCATTCTGAAGTTCCTCA (SEQ ID NO : 59) | AAAATCCCCAGAGCCACATAG (SEQ ID NO : 60) |
| PKA-γ2 | TTGCCCGTTATTGACCCTATC (SEQ ID NO: 61) | CGTTCCTATTCCAAGCTCATCC (SEQ ID NO : 62) |
| PKA-γ13 | TGACTGCACTGGACATCTTTG (SEQ ID NO : 63) | TGGTTGTAGGTTTGCTGGG (SEQ ID NO : 64) |
| CK-1α | TGTCGGAGGGAAATATAAACTGG (SEQ ID NO : 65) | GGCCTTCTGAGATTCTAGCTTC (SEQ ID NO : 66) |
| CK-1γ1 | AGGTGGAGGTAGTGGAGG (SEQ ID NO : 67) | GTACAATTGAGTCAGAGTCCCC (SEQ ID NO : 68) |
| CK-1γ2 | GTGATGTTCTAGCCACAGAGG (SEQ ID NO : 69) | CCCTTTCCCTCCTTTCTTGTC (SEQ ID NO : 70) |
| CK-1γ3 | GTTCAAATGCACCCATCACAG (SEQ ID NO: 71) | AGTAACTCCCCAGGATCTGTC (SEQ ID NO : 72) |
| CK-2α1 | GTATGAGATTCTGAAGGCCCTG (SEQ ID NO : 73) | CCAAACCCCAGTCTATTAGTCG (SEQ ID NO : 74) |
| CK-2α2 | CGATACGACCATCAACAGAGAC (SEQ ID NO : 75) | TCGCTTTCCAGTCTTCATCG (SEQ ID NO : 76) |

### G protein expression in osteoblasts

Expression levels of Gi proteins isoforms and Gs protein were determined using standard western blotting technique. In brief, osteoblasts from AIS patients and trauma control cases were lysed in RIPA buffer (25 mM Tris-HCl, pH 7.4, 150 mM NaCl, 1% NP-40, 1% sodium deoxycholate, 0.1% SDS) added with 5 mM NaVO₄ and protease inhibitor cocktail (Roche molecular Biochemicals, Mannheim, Germany). Immunoblots were performed with primary antibodies directed specifically against Gi₁, Gi₂, Gi₃ or Gs (Santa Cruz Biotechnology, Santa Cruz, CA) and peroxidase-conjugated secondary antibody. Bands were then visualized using SuperSignal™ chemilunescent substrate (Pierce, Rockford, IL).

### Assessment of phosphorylation of Gi protein isoforms in osteoblasts

Comparative studies were performed to examine the level of phosphorylation and to identify the phosphorylated Gi isoform with immunoprecipitated proteins using isoform specific antibodies and, subsequently, probing the level of phosphorylation with a phospho-serine/threonine specific antibody (Santa Cruz Biotechnology). Whole cell proteins (1 mg) were incubated with anti-Gii, anti-Gi₂ or anti-Gi₃ (Santa Cruz Biotechnology), plus protein G beads for immunoprecipitation (IP) at 4°C overnight. Purified proteins were loaded on 10% gel after IP, then processed by transferring to nitrocellulose membrane and 5% BSA blocking. Membranes were exposed to phospho-serine/threonine specific antibody at 4°C overnight and, subsequently, treated with secondary antibody at room temperature for 1 h. Bands were visualized using SuperSignal cheminescence™, and quantified by densitometric scanning.

### Statistical analysis

Data are presented as mean ± SE, and were analyzed by ANOVA or Student's t test using GraphPad™ Prism 4.0 software. Multiple comparisons of means were performed with one-way ANOVA followed by a post-hoc test of Dunnett. Only P values < 0.05 were considered significant.

*Demographic and clinical characteristics of French-Canadian and Italian cohorts.* The French-Canadian cohorts consisted of 956 consecutive (i.e. without selection: the first 956 subjects having accepted to participate in the study) adolescents with AIS and 240 aged-matched controls without a family history of scoliosis. The absence of spine abnormalities was confirmed in all control subjects, while a total of 162 AIS patients exhibited curvatures greater than 45° and 794 AIS patients had curvature between 10° and 44°. In the Italian cohort, the moderate curves (Cobb angle 10° - 44°) were apparent in 61 AIS patients and the severe curves (Cobb angle > 45°) in 78 AIS patients. All AIS patients were age-matched with control subjects in both Canadian and Italian cohorts (see **Table IV** below).

**Table IV: Demographic data of AIS and healthy control subjects**

| | **Canadian cohort** | | **Italian cohort** | |
|---|---|---|---|---|
| | **N** | **Mean age (years)** | **N** | **Mean age (years)** |
| **Healthy control subjects** | 240 | 12.4 ± 3.2 | 103 | 11.0 ± 1.6 |

| **AIS patients** | | | | |
|---|---|---|---|---|
| Cobb Angle < 44% | 794 | 13.0 ± 2.6 | 78 | 13.6 ± 2.8 |
| Cobb Angle > 45% | 162 | 15.1 ± 2.1 | 61 | 13.8 ± 2.2 |

### EXAMPLE 2

### Clinical outcomes of AIS patients according to their functional classification

Patients were classified according to the response degree of their PBMCs to iodomelatonin stimulation as indicated in **Example 1.** Of 956 AIS patients from the Canadian cohort, 243 were classified in functional group 1 (FG1), 353 in functional group 2 (FG2) and 360 in functional group 3 (FG3). The prevalence of all three functional groups was comparable among low to moderate cases (Cobb angle 10° - 44°). However, the FG2 was predominant among severe cases (Cobb angle > 45°) with a proportion of 56% compared to 31% and 13% for FG3 and FG1, respectively. See **Table V** below.

Similar profile of distribution was observed in the Italian cohort in whom surgery was required for 61% of FG2, 36% of FG3 and 3% of FG1 AIS patients. Collectively, these results strengthen the view that clinical outcomes vary among AIS patients and suggest that the risk of severe progression is higher for FG2, moderate for FG3 and low for FG1. See **Table VI** below.

**Table V: Clinical data of AIS patients classified into functional groups**

| **Canadian Cohort** | | | | | | | |
|---|---|---|---|---|---|---|---|
| | **Cobb Angle < 44°** | | **Cobb Angle > 45°** | | **Curve Type** | | |
| | N (%) | Cobb Angle (°) | N (%) | Cobb Angle (°) | Single N (%) | Double N (%) | Triple N (%) |
| **FG1** | 222 (28) | 20.0 ± 10.1 | 21 (13) | 60.8 ± 12.4 | 124 (51) | 109 (45) | 10 (4) |
| **FG2** | 262 (33) | 21.5 ± 9.5 | 91 (56) | 60.0 ± 10.8 | 170 (48) | 162 (46) | 21 (6) |
| **FG3** | 310 (39) | 18.9 ± 9.4 | 50 (31) | 60.7 ± 12.6 | 194 (54) | 148 (41) | 18 (5) |

**Table VI: Clinical data of AIS patients classified into functional groups**

| **Italian Cohort** | | | | | | | |
|---|---|---|---|---|---|---|---|
| | **Cobb Angle < 44°** | | **Cobb Angle > 45°** | | **Curve Type** | | |
| | N (%) | Cobb Angle (°) | N (%) | Cobb Angle (°) | Single N (%) | Double N (%) | Triple N (%) |
| **FG1** | - | - | 1(4) | 62.0 | 1(2) | - | - |
| **FG2** | 92 (95) | 24.3 ± 10.0 | 15(65) | 53.5±13.4 | 58(91) | 48(87) | - |
| **FG3** | 5 (5) | 17.6 ±5.7 | 7(30) | 60.6±12.9 | 5(8) | 7(13) | - |

### EXAMPLE 3

### Each functional group represents a potential hereditary trait

Since the hereditary or genetic basis of AIS has consistently been claimed (Riseborough and Wynne-Davies, 1973); (Blank et al., 1999); (Roach, 1999), the possibility that the biological defect characterizing each functional group may be a hereditary condition was tested. For this purpose, 25 individuals from 6 unrelated families were examined. Pedigrees are shown in **Figure 1****.** At least two individuals were affected in each family. The classification has revealed that all affected family members belonged to the same functional group and so displayed similar biological defect (**Figure 2**). However, neither pattern nor severity of curve was group specific (**Figure 2**). This suggests that each functional group represents a biological endophenotype that co-segregates within families independently of curve type and magnitude of spinal deformity.

### EXAMPLE 4

### Affinity of melatonin receptor for its agonist is similar between functional groups

It was tested whether the melatonin signaling dysfunction in AIS was due to changes affecting the melatonin receptor/Gi protein coupling. First was examined whether the affinity of melatonin for its receptors varies among AIS patients groups. For this purpose, concentration-response curves were generated for melatonin using osteoblasts from control subjects and compared with those of AIS patients of each biological endophenotype. As illustrated in **Figure 3A****,** melatonin caused a concentration-dependent increase of response in all control and AIS groups reaching a plateau at 1 µM, with the higher magnitude in control group and lower but at varying degree within the three AIS groups. The order of magnitude of the maximum response from highest to lowest responses was control group, FG3, FG2 and FG1. Despite this apparent discrepancy in the maximum response between groups, the half-maximum response was observed at similar concentrations in control and AIS groups. These observations suggest that the affinity of melatonin receptor for its agonist is preserved in AIS.

### EXAMPLE 5

### Activity of melatonin receptor in response to different agonists is comparable between control subjects and AIS patients

Then was examined the possibility of changes in the activity of melatonin receptor by testing three agonists that have different efficacy to activate melatonin receptor. The response produced with their maximum concentration (10 µM) in osteoblasts from control subjects and AIS patients of each functional group was compared. Results illustrated in **Figure 3B** show that the three agonists, melatonin, idomelatonin and phenylmelatonin evoked various degrees of response in osteoblasts from control and AIS subjects. The response to each agonist was lower in all AIS groups compared to the control group. However, the magnitude and the reduction due to AIS in each group reflected the efficacy of agonists, the order of agonist potency being phenylmelatonin > iodomelatonin > melatonin, as previously reported by other investigators using different methods (Nonno et al., 1999). This suggests that the activity of melatonin receptor is comparable between control subjects and AIS patients and underscore that deficit is beyond the melatonin receptor.

### EXAMPLE 6

### Ability of agonist/receptor complex to activate Gi proteins is decreased in AIS patients vs. in controls

The possibility that the deficit in melatonin signaling could be related to a decreased ability of agonist/receptor complex to activate Gi proteins was tested. The G protein antagonist GPAnt-2, a hydrophobic peptide, has been shown to inhibit receptor/Gi protein coupling by competing with the activated receptor for interaction with G proteins (Mukai et al., 1992). Results illustrated in **Figure 3C** show that GPAnt-2 inhibited the response to melatonin stimulation in a concentration-dependent manner in control and AIS functional groups. At maximal concentration, the extent of reduction was similar in all groups, but at submaximal concentrations, the pattern is clearly different. Concentration-response curves of all three AIS functional groups exhibited a left-shift compared to that of the control group, and the IC50 values were significantly decreased among AIS groups when compared to controls. Taking into account that GPAnt-2 competes with receptors on various G proteins, the amount of Gi proteins was further selectively decreased by incubating osteoblasts with increasing concentration of pertussis toxin (PTX). It was found that PTX treatment reduced the cellular response to melatonin in FG2 and FG3 osteoblasts exhibiting a pattern similar to the one obtained with GPAnt-2. In contrast, PTX treatment enhanced the response to melatonin in FG1 osteoblasts at maximal concentration (**Figure 3D**). These data show that the reduced melatonin signaling in AIS is most likely due to a decrease in the sensibility of Gi proteins for melatonin receptors, and indicate that the melatonin receptors may also interact with Gs in AIS patients classified in FG1.

### EXAMPLE 7

### AIS subjects have a systemic and generalized impairment of Gi protein-mediated receptor signaling

To determine if the signaling dysfunction through Gi proteins as measured in AIS osteoblasts was restricted to melatonin receptors, a comparative study was performed with various synthetic compounds activating selectively other receptors coupled to Gi proteins. Five compounds were used including apelin-17, PB554 maleate, lysophosphatidic acid (LPA), UK14304 and somatostatin to activate endogenous APJ, serotonin 5-HT_{1A}, LPA₁, alpha2-adrenergic and somatostatin (sst) receptors, respectively. As illustrated in **Figure 4****,** all tested agonists caused a concentration-dependent increase of the cellular response, reaching a plateau at the same concentration in all control and AIS functional groups. In each case, the magnitude of the signaling response was lower in all AIS groups when compared with the control group, but the EC50 values were almost identical in all groups indicating that the affinity of all tested agonists for their respective receptors is not affected in AIS (**Figure 4G**). Interestingly, inhibition curves of GPAnt-2 (**Figure 5**) or PTX (**Figure 6**) generated with any of the five synthetic agonists tested revealed curve patterns similar to those obtained with melatonin. In each case, GPAnt-2 reduced the IC50 values in AIS groups when compared to control group (**Figure 5G**). Similar responses were also observed when GPAnt-2 was competed with mastoparan-7 (**Figure 5F**), which directly activates Gi proteins by mimicking agonist-activated receptor (Higashijima et al., 1990). Moreover, response to mastoparan was almost abolished in all AIS and control groups following treatment with high concentrations of PTX (**Figure 6F**), further pointing to an abnormality at the level of Gi proteins. Collectively, these data strengthen the concept that agonist/receptor interaction is not affected in AIS, and reveal that AIS patients can be functionally stratified with any compounds that activate Gi protein-mediated signaling pathways.

The analysis was extended to other cell types, namely skeletal myoblasts and peripheral blood mononuclear cells (PBMCs) from the same set of controls and AIS patients. A pattern of response similar to that obtained in osteoblasts for each agonist tested was obtained in these cell types (**Figures 7** and **8**). Overall, these findings are strongly indicative of a systemic and generalized impairment of Gi protein-mediated receptor signaling.

### EXAMPLE 8

### Reduction in Gi protein function selectively influences Gs protein function in AIS

Osteoblasts from control and AIS patients were screened for their response to isoproterenol and desmopressin, which activate beta-adrenergic and vasopressin (V2) receptors, respectively. Both receptors mediate signal transduction through Gs proteins. Results illustrated in **Figure 9** show that cellular responses initiated by both agonists were significantly enhanced in osteoblasts from AIS patients when compared with control osteoblasts. For each functional group, increased response to Gs stimulation inversely mirrored the reduced response induced after Gi protein stimulation, suggesting a functional imbalance between Gi and Gs proteins. To further illustrate this divergence, values of the responses to melatonin and isoproterenol were reported as differences (Δ) between response to Gi and Gs protein stimulation. As illustrated in **Figure 9C****,** response to Gi stimulation predominated in a concentration-dependent manner in control group (i.e., a Gi/Gs ratio of more than about 1.5). A similar pattern was observed in the FG3 group (i.e., a Gi/Gs ratio of more than about 1.5), while no apparent imbalance was observed in the FG2 group (i.e., a Gi/Gs ratio of between about 0.5 and 1.5). In contrast, the FG1 group exhibited predominance for response to Gs stimulation (i.e., a Gi/Gs ratio of less than about 0.5). These data indicate that Gs protein is functionally affected in AIS according to the aberration degree of Gi protein function, revealing a profile of imbalance between Gi and Gs protein function specific to each AIS group.

Results presented reveal a relationship between reduced Gi and increased Gs protein functions in AIS. The profile of the functional imbalance between Gi and Gs protein was specific to each AIS group, indicating that AIS patients can be clearly classified with respect to the profile of imbalance between response to Gi and Gs protein stimulation. Such an approach advantageously eliminates the necessity to use control subjects and allows the monitoring patient responses over time.

The functional status of Gq protein was then studied. Osteoblasts were stimulated with bradykinin and endothelin-1. Both agonists elicited similar responses at various concentrations in osteoblasts derived from control subjects and AIS patients, demonstrating that receptor signaling through Gq protein remains largely intact in AIS (**Figures 9D-E**). It appears that reduction in Gi protein function in AIS exclusively influences Gs protein function.

### EXAMPLE 9

### Differences in the degree of response characterizing the three AIS biological endophenotypes are not due to differential expression of Gi proteins

The three isoforms of Gi proteins, termed Gi₁, Gi₂ and Gi₃ share the same proprieties and most membranous receptors that interact with Gi proteins are able to initiate signals via each of these isoforms. However, the amplitude of the response depends on the capacity of Gi isoforms to mediate the signal transduction while some Gi isoforms seem more efficient than others.

It was tested whether the differences in the degree of response characterizing the three AIS biological endophenotypes is due to the differential alteration of Gi proteins isoforms. First was examined whether observed functional changes are due to changes in the expression of G proteins. The qPCR analysis revealed no significant change in expression of any isoform of Gi proteins (Gi₁, Gi₂ and Gi₃) and Gs protein between control and AIS osteoblasts (**Figure 10A**). Assuming that PCR-amplification of the different isoforms was equally efficient, it appears that Gi₁ and Gi₂ were the most abundant isoforms of Gi proteins in control and AIS osteoblasts, while the expression level of Gi₃ isoform was less abundant and similar to that of Gs protein. At the protein level, these isoforms have also revealed no difference between control and any AIS group (**Figure 10B**). These results indicate that the functional changes observed in osteoblasts from AIS patients were unlikely due to aberration in the expression levels of Gi protein isoforms.

### EXAMPLE 10

### Differential phosphorylation patterns affect Gi protein isoforms in AIS functional groups

Activity of Gi proteins is acutely regulated by phosphorylation, a process that limits their ability to transduce signals (Casey et al., 1995); (Katada et al., 1985); (Kozasa and Gilman, 1996); (Lounsbury et al., 1991); (Lounsbury et al., 1993); (Morishita et al. 1,995); (Morris et al., 1995); (Yatomi et al., 1992). An increased phosphorylation of serine residues of the three Gi₁, Gi₂ and Gi₃ isoforms of Gi proteins in osteoblasts from AIS patients was previously reported (Moreau et al., 2004). To examine whether the functional disruption in Gi signaling occurring in AIS can be related to the differential patterns of Gi phosphorylated isoforms, Gi isoforms from control subjects and AIS patients from each biological endophenotype were immunoprecipitated and probed with anti-phospho-serine antibody (**Figure 11**). Compared with control group, only Gi₂ and Gi₃ isoforms were phosphorylated in the FG3 group, while Gi₁ and Gi₂ only were phosphorylated in the FG2 group. However, the three Gi₁, Gi₂ and Gi₃ isoforms were phosphorylated in FG1 group. These data provide evidence for a relationship between the phosphoprylation pattern of Gi isoforms and the heterogeneous defect of Gi proteins in AIS and are indicative of a difference in the functional status of Gi protein isoforms among AIS groups.

### EXAMPLE 11

### Differential phosphorylation patterns affect Gi protein isoforms in AIS functional groups

The identity of Gi isoforms responsible for the residual response in each AIS group was tested using a small interference RNA (siRNA) approach to knockdown individually or in combination the expression of Gi₁, Gi₂ Gi₃ and Gs prior to stimulating osteoblasts with melatonin, LPA or somatostatin. Silencing of each gene reduced by 75-85 % the expression of the corresponding mRNA in osteoblasts from control and the three AIS groups (**Figure 12E-H**). In the control group, the response to any tested agonist was not significantly affected by Gi₁, Gi₂ Gi₃ siRNA when transfected alone, but was almost abolished when transfected together (**Figure 12A**). The response to each tested agonist was reduced by at least 75% by silencing Gi₃ alone in FG2 osteoblasts (**Figure 12C**), and by 90% by silencing Gi₁ alone in FG3 osteoblasts (**Figure 12D**) confirming that the residual response to Gi stimulation is mediated by Gi₃ and Gi₁ isoforms in FG2 and FG3 groups, respectively. In FG1 osteoblasts, response to each agonist was reduced by 50% following the depletion of all Gi isoforms by siRNA, and was not affected by the knockdown of individual Gi isoforms (**Figure 12B**). In contrast, the depletion of Gs alone reduced the cellular response to any tested agonist by 50% in the FG1 AIS group, and was devoid of effect in the control, FG2 and FG3 AIS groups. This indicates that the residual response to Gi stimulation in the FG1 functional group is an additive effect of Gi-receptors coupling simultaneously to Gs and Gi proteins.

The detection of unphosphorylated Gi₃ and Gi₁ isoforms respectively in FG2 and FG3 groups could explain their higher Gi signaling activity when compared to FG1 group. Nevertheless, FG2 osteoblasts exhibit a much weaker residual Gi signaling activity when compared to FG3 osteoblasts, which could be explained by the fact that Gi₃ isoform is less abundant in human osteoblasts as demonstrated in Example 9 (**Figure 10**). The selective depletion of Gi₃ and Gi₁ isoforms in FG2 and FG3 osteoblasts, respectively almost abolished their cellular responses to three distinct Gi-coupled receptors in a similar manner as demonstrated in **Figure 12****,** further confirming the functional status of Gi₃ and Gi₁ isoforms in these two AIS groups. It is tempting to speculate that the loss of function of other Gi isoforms may be compensated by Gi₃ isoform in FG2 and by Gi₁ in FG3 group, which is consistent with the concept that each Gi isoform can partially rescue Gi signaling (Hurst et al., 2008).

Without being bound by this hypothesis, these findings, together with results from the experiments with high PTX concentrations, suggest the presence of a compensatory mechanism involving other G proteins in FG1 osteoblasts. Conceptually, it is possible that phosphorylation of all Gi isoforms allows or facilitates the coupling of Gs proteins to Gi-coupled receptors in FG1 osteoblasts. Such possibility was clearly demonstrated by the simultaneous deletion of Gs and all Gi isoforms, which almost abrogated completely the signaling activity in FG1 osteoblasts. On the other hand, the three GPCRs tested did not activate Gs mediated signaling in FG2 and FG3 osteoblasts, which strongly suggests that Gs protein access to these receptors is limited by the presence of Gi₃ and Gi₁ isoforms, respectively, which are not phosphorylated in these groups. Of note, depletion of Gs proteins alone did not abolish the signaling activity in FG1 osteoblasts despite the fact that all three Gi isoforms were phosphorylated suggesting a possible heterogeneity in the number and position of serine residues phosphorylated among AIS functional groups. It is conceivable that phosphorylated Gi proteins in FG1 osteoblasts still exhibit some residual activity as opposed to phosphorylated Gi isoforms in FG2 and FG3 groups.

### EXAMPLE 12

### Identification of serine/threonine kinases contributing to Gi proteins hypofunctionality in AIS

In order to identify putative serine/threonine kinases phosphorylating Gi isoforms in AIS, control and AIS osteoblasts were treated with a panel of serine/threonine kinase inhibitors prior to their stimulation with two distinct agonists (LPA and somatostatin). Of note, Gi stimulation induced via LPA or somatostatin receptor activation by their agonists in control osteoblasts was not affected by any kinase inhibitor (**Figure 13**). In contrast, signaling responses was significantly increased in FG1 osteoblasts by Gö8963, which inhibits several isoforms of PKC and also by STO-609 acetate, which inhibits three isoforms of calmodulin kinase (CaMK1, CaMK2, CaMK4), but was unaffected by KN93 that inhibits selectively CaMK2. However, both CaMK inhibitors STO-609 acetate and KN93 increased the signalling response in osteoblasts from FG2 group, while Gö8963 was devoid of significant effect in this AIS group. Cellular response in FG3 osteoblasts was not improved by inhibition of neither of PKC nor CaMK. In contrast, the inhibition of Casein Kinase 2 (CK2) with D4476 increased response in osteoblasts from FG1 and FG3 groups, but not in FG2 osteoblasts, while the inhibition of PKA with H89 increased the signalling response in all AIS groups.

Expression analysis has revealed a selective increase in the expression levels of PKCε, PKCη and CaMK1-δ in FG1 osteoblasts when compared to control group (**Figure 14**). FG2 and FG3 osteoblasts have exhibited high expression levels of PKAγ2 only. In contrast, the expression level of CaMK2n1, a natural CaMK2 inhibitor, was significantly decreased in FG2 osteoblasts, suggesting that the regulatory system of CaMK2 activity is affected in this AIS group (**Figure 14**). The expression levels of other analysed kinases did not show any significant selective increase or decrease (**Figures 15-16**) Collectively, these results show that selective functional alteration of Gi protein isoforms in AIS involves distinct kinases.

### REFERENCES

1. Akoume, M.-Y., B. Azeddine, et al. (2010). "Cell-based screening test for idiopathic scoliosis using cellular dielectric spectroscopy." Spine 35(13): E601.
2. Anand-Srivastava, M. B. (1989). "Amiloride interacts with guanine nucleotide regulatory proteins and attenuates the hormonal inhibition of adenylate cyclase." J Biol Chem 264(16): 9491-9496.
3. Anand-Srivastava, M. B., A. K. Srivastava, et al. (1987). "Pertussis toxin attenuates atrial natriuretic factor-mediated inhibition of adenylate cyclase. Involvement of inhibitory guanine nucleotide regulatory protein." J Biol Chem 262(11): 4931-4934.
4. Azeddine, B., K. Letellier, et al. (2007). "Molecular determinants of melatonin signaling dysfunction in adolescent idiopathic scoliosis." Clin Orthop Relat Res 462: 45-52.
5. Blank, R. D., C. L. Raggio, et al. (1999). "A genomic approach to scoliosis pathogenesis." Lupus 8(5): 356-360.
6. Bushfield, M., S. L. Griffiths, et al. (1990). "Diabetes-induced alterations in the expression, functioning and phosphorylation state of the inhibitory guanine nucleotide regulatory protein Gi-2 in hepatocytes." Biochem J 271(2): 365-372.
7. Bushfield, M., B. E. Lavan, et al. (1991). "Okadaic acid identifies a phosphorylation/dephosphorylation cycle controlling the inhibitory guanine-nucleotide-binding regulatory protein Gi2." Biochem J 274 (Pt 2): 317-321.
8. Casey, J. H., C. J. McLean, et al. (1995). "Driving, glaucoma, and the law. Diabetic patients must also satisfy regulations." BMJ 310(6971): 56.
9. Chen, C. A. and D. R. Manning (2001). "Regulation of G proteins by covalent modification." Oncogene 20(13): 1643-1652.
10. Fazal, M. A. and M. Edgar (2006). "Detection of adolescent idiopathic scoliosis." Acta orthopaedica belgica 72(2): 184.
11. Gawler, D., G. Milligan, et al. (1987). "Abolition of the expression of inhibitory guanine nucleotide regulatory protein Gi activity in diabetes." Nature 327(6119): 229-232.
12. Hardeland, R. (2009). "Melatonin: signaling mechanisms of a pleiotropic agent." Biofactors 35(2): 183-192.
13. Higashijima, T., J. Burnier, et al. (1990). "Regulation of Gi and Go by mastoparan, related amphiphilic peptides, and hydrophobic amines. Mechanism and structural determinants of activity." J Biol Chem 265(24): 14176-14186.
14. Houslay, M. D. and G. Milligan (1997). "Tailoring cAMP-signalling responses through isoform multiplicity." Trends Biochem Sci 22(6): 217-224.
15. Itoh, H., F. Okajima, et al. (1984). "Conversion of adrenergic mechanism from an alpha- to a beta-type during primary culture of rat hepatocytes. Accompanying decreases in the function of the inhibitory guanine nucleotide regulatory component of adenylate cyclase identified as the substrate of islet-activating protein." J Biol Chem 259(24): 15464-15473.
16. Kane, W. J. (1977). "Scoliosis prevalence: a call for a statement of terms." Clin Orthop Relat Res(126): 43-46.
17. Katada, T., A. G. Gilman, et al. (1985). "Protein kinase C phosphorylates the inhibitory guanine-nucleotide-binding regulatory component and apparently suppresses its function in hormonal inhibition of adenylate cyclase." Eur J Biochem 151(2): 431-437.
18. Kozasa, T. and A. G. Gilman (1996). "Protein kinase C phosphorylates G12 alpha and inhibits its interaction with G beta gamma." J Biol Chem 271(21): 12562-12567.
19. Letellier, K., B. Azeddine, et al. (2008). "Estrogen cross-talk with the melatonin signaling pathway in human osteoblasts derived from adolescent idiopathic scoliosis patients." Journal of pineal research 45(4): 383-393.
20. Lonstein, J. (1994). "Adolescent idiopathic scoliosis." The lancet 344(8934): 1407-1412.
21. Lounsbury, K. M., P. J. Casey, et al. (1991). "Phosphorylation of Gz in human platelets. Selectivity and site of modification." J Biol Chem 266(32): 22051-22056.
22. Lounsbury, K. M., B. Schlegel, et al. (1993). "Analysis of Gz alpha by site-directed mutagenesis. Sites and specificity of protein kinase C-dependent phosphorylation." J Biol Chem 268(5): 3494-3498.
23. McClue, S., E. Selzer, et al. (1992). "Gi3 does not contribute to the inhibition of adenylate cyclase when stimulation of an alpha 2-adrenergic receptor causes activation of both Gi2 and Gi3." Biochemical Journal 284(Pt 2): 565.
24. Moreau, A., M. Y. Akoume Ndong, et al. (2009). "Molecular and genetic aspects of idiopathic scoliosis. Blood test for idiopathic scoliosis." Orthopade 38(2): 114-116, 118-121.
25. Moreau, A., S. Forget, et al. (2004). "Melatonin signaling dysfunction in adolescent idiopathic scoliosis." Spine 29(16): 1772-1781.
26. Morishita, R., H. Nakayama, et al. (1995). "Primary structure of a gamma subunit of G protein, gamma 12, and its phosphorylation by protein kinase C." J Biol Chem 270(49): 29469-29475.
27. Morris, N. J., P. Young, et al. (1995). "Insulin inhibits the phosphorylation of alpha-Gi-2 in intact hepatocytes." Biochem J 308 (Pt 2): 693-696.
28. Mukai, H., E. Munekata, et al. (1992). "G protein antagonists. A novel hydrophobic peptide competes with receptor for G protein binding." J Biol Chem 267(23): 16237-16243.
29. Murthy, K. S., J. R. Grider, et al. (2000). "Heterologous desensitization of response mediated by selective PKC-dependent phosphorylation of G(i-1) and G(i-2)." Am J Physiol Cell Physiol 279(4): C925-934.
30. Negrini, S., S. Minozzi, et al. (2010). "Cochrane Review: Braces for idiopathic scoliosis in adolescents." Evidence-Based Child Health: A Cochrane Review Journal 5(4): 1681-1720.
31. Nonno, R., M. Pannacci, et al. (1999). "Ligand efficacy and potency at recombinant human MT2 melatonin receptors: evidence for agonist activity of some mt1-antagonists." Br J Pharmacol 127(5): 1288-1294.
32. Riseborough, E. J. and R. Wynne-Davies (1973). "A genetic survey of idiopathic scoliosis in Boston, Massachusetts." J Bone Joint Surg Am 55(5): 974-982.
33. Roach, J. W. (1999). "Adolescent idiopathic scoliosis." Orthop Clin North Am 30(3): 353-365, vii-viii.
34. Verdonk, E., K. Johnson, et al. (2006). "Cellular dielectric spectroscopy: a label-free comprehensive platform for functional evaluation of endogenous receptors." Assay and drug development technologies 4(5): 609-619.
35. Weinstein, S. L. (1989). "Adolescent idiopathic scoliosis: prevalence and natural history." Instr Course Lect 38: 115-128.
36. Wesslau, C. and U. Smith (1992). "The inhibitory GTP-binding protein (Gi) regulates the agonistic property of beta-adreneraic ligands in isolated rat adipocytes. Evidence for a priming effect of cyclic AMP." Biochem J 288 (Pt 1): 41-46.
37. Yatomi, Y., Y. Arata, et al. (1992). "Phosphorylation of the inhibitory guanine-nucleotide-binding protein as a possible mechanism of inhibition by protein kinase C of agonist-induced Ca2+ mobilization in human platelet." Eur J Biochem 205(3): 1003-1009.

## Claims

1. An *in vitro* method of stratifying a subject having or at risk for developing adolescent idiopathic scoliosis (AIS), said method comprising:
(i) providing a cell sample isolated from the subject;
(ii)
(a) detecting or determining the serine phosphorylation of Giα1 in the cell sample; and/or
(b) detecting or determining the serine phosphorylation of Giα3 in the cell sample; and
(iii) stratifying said subject into a clinically useful AIS subclass based on the subject's Giα1 or Giα3 protein serine phosphorylation profile.

2. The *in vitro* method of claim 1, wherein (ii) comprises (a) and (b) and wherein the method: further comprises stratifying the subject as belonging to:
(1) a first AIS subclass **characterized by** elevated levels of serine-phosphorylated Giα1 and Giα3 proteins as compared to levels corresponding to those of a control;
(2) a second AIS subclass **characterized by** elevated levels of serine-phosphorylated Giα1 but not of serine-phosphorylated Giα3 protein, as compared to levels corresponding to that of a control; or
(3) a third AIS subclass **characterized by** elevated levels of serine-phosphorylated Giα3 protein but not of serine-phosphorylated Giα1 protein as compared to levels corresponding to those of a control.

3. The *in vitro* method or claim 1 or 2, further comprising detecting or determining the serine phosphorylation of Giα2 protein in the cell sample, wherein elevated levels of serine-phosphorylated Giα2 protein are detected, as compared to levels corresponding to that of a control.

4. The *in vitro* method of claim 2 or 3, wherein:
(1) subjects belonging to said first AIS subclass have a low risk of severe AIS progression;
(2) subjects belonging to said second AIS subclass have a high risk for severe AIS progression; and
(3) subjects belonging to said third AIS subclass have a moderate risk for severe AIS progression.

5. An *in vitro* method for predicting the risk for developing a severe scoliosis in a subject having or at risk for developing scoliosis, said method comprising:
(i) providing a cell sample isolated from the subject;
(ii) detecting or determining the serine phosphorylation of Giα3 protein in the cell sample;
(iii) determining that the subject is at risk for developing a severe scoliosis when serine-phosphorylated Giα3 protein in the cell sample is not detected, or is not elevated as compared to levels corresponding to those of a control.

6. The *in vitro* method of claim 5, further comprising in step (ii) detecting or determining the serine phosphorylation of Giα1 and/or Giα2 protein in the cell sample, wherein said subject is at risk for developing a severe scoliosis when the level of said subject's serine-phosphorylated Giα1 or Giα2 protein is elevated as compared to levels corresponding to those of a control and serine-phosphorylated Giα3 protein in the cell sample is not detected, or is not elevated as compared to levels corresponding to those of a control.

7. The *in vitro* method of any one of claims 2 to 6, wherein the subject is a subject diagnosed with a scoliosis.

8. The *in vitro* method of claim 7, wherein the scoliosis is adolescent idiopathic scoliosis (AIS).

9. The *in vitro* method of any one of claims 1 to 8, wherein said cell sample comprises osteoblasts, myoblasts and/or peripheral blood mononuclear cells (PBMC).

10. The *in vitro* method of claim 9, wherein said cell sample comprises PBMCs.

11. The *in vitro* method of claim 10, wherein said PBMCs comprise lymphocytes.

12. The *in vitro* method of any one of claims 1 to 11, wherein the detecting or determining the serine phosphorylation of Giα1 and/or Giα3 protein(s) in the cell sample comprises isolating Giα1 and/or Giα3 protein(s) from the cell sample and contacting the isolated Giα1 and/or Giα3 protein(s) with an anti-phosphoserine antibody.

## Patentansprüche

1. *In vitro*-Verfahren zum Stratifizieren eines Probanden mit idiopathischer Adoleszentenskoliose (AIS) oder mit Risiko, diese zu entwickeln, wobei das Verfahren umfasst:
(i) Bereitstellen einer von dem Probanden isolierten Zellprobe;
(ii)
(a) Erfassen oder Bestimmen der Serinphosphorylierung von Giα1 in der Zellprobe; und/oder
(b) Erfassen oder Bestimmen der Serinphosphorylierung von Giα3 in der Zellprobe; und
(iii) Stratifizieren des Probanden in eine klinisch verwendbare AIS-Unterklasse auf Grundlage des Giα1- oder Giα3-Protein-Serinphosphorylierungsprofils des Probanden.

2. *In vitro*-Verfahren nach Anspruch 1, wobei (ii) (a) und (b) umfasst und wobei das Verfahren: ferner umfasst das Stratifizieren des Probanden als zugehörig zu:
(1) einer ersten AIS-Unterklasse, **gekennzeichnet durch** erhöhte Niveaus von serinphosphorylierten Giα1- und Giα3-Proteinen, verglichen mit Niveaus, entsprechend denen einer Kontrolle;
(2) einer zweiten AIS-Unterklasse, **gekennzeichnet durch** erhöhte Niveaus von serinphosphoryliertem Giα1- aber nicht von serinphosphoryliertem Giα3-Protein, verglichen mit Niveaus, entsprechend dem einer Kontrolle; oder
(3) einer dritten AIS-Unterklasse, **gekennzeichnet durch** erhöhte Niveaus von serinphosphoryliertem Giα3-Protein aber nicht von serinphosphoryliertem Giα1-Protein, verglichen mit Niveaus, entsprechend denen einer Kontrolle.

3. *In vitro*-Verfahren nach Anspruch 1 oder 2, ferner umfassend Erfassen oder Bestimmen der Serinphosphorylierung von Giα2-Protein in der Zellprobe, wobei erhöhte Niveaus von serinphosphoryliertem Giα2-Protein erfasst werden, verglichen mit Niveaus, entsprechend dem einer Kontrolle.

4. *In vitro*-Verfahren nach Anspruch 2 oder 3, wobei:
(1) Probanden, die zu der ersten AIS-Unterklasse gehören, ein geringes Risiko von schwerer AIS-Progredienz aufweisen;
(2) Probanden, die zu der zweiten AIS-Unterklasse gehören, ein hohes Risiko von schwerer AIS-Progredienz aufweisen; und
(3) Probanden, die zu der dritten AIS-Unterklasse gehören, ein moderates Risiko von schwerer AIS-Progredienz aufweisen.

5. *In vitro*-Verfahren zum Vorhersagen des Risikos des Entwickelns einer schweren Skoliose in einem Probanden mit Skoliose oder mit einem Risiko zum Entwickeln von Skoliose, wobei das Verfahren umfasst:
(i) Bereitstellen einer von dem Probanden isolierten Zellprobe;
(ii) Erfassen oder Bestimmen der Serinphosphorylierung von Giα3-Protein in der Zellprobe;
(iii) Bestimmen, dass der Proband ein Risiko zum Entwickeln einer schweren Skoliose aufweist, wenn serinphosphoryliertes Giα3-Protein in der Zellprobe nicht erfasst wird oder, verglichen mit Niveaus, entsprechend denen einer Kontrolle, nicht erhöht ist.

6. *In vitro*-Verfahren nach Anspruch 5, ferner umfassend in Schritt (ii) Erfassen oder Bestimmen der Serinphosphorylierung von Giα1- und/oder Giα2-Protein in der Zellprobe, wobei der Proband ein Risiko zum Entwickeln einer schweren Skoliose aufweist, wenn das Niveau von serinphosphoryliertem Giα1- oder Giα2-Protein des Probanden verglichen mit Niveaus, entsprechend denen einer Kontrolle, erhöht ist, und serinphosphoryliertes Giα3-Protein in der Zellprobe nicht erfasst wird oder, verglichen mit Niveaus, entsprechend denen einer Kontrolle, nicht erhöht ist.

7. *In vitro*-Verfahren nach einem der Ansprüche 2 bis 6, wobei der Proband ein Proband ist, bei dem eine Skoliose diagnostiziert wurde.

8. *In vitro*-Verfahren nach Anspruch 7, wobei die Skoliose idiopathische Adoleszentenskoliose (AIS) ist.

9. *In vitro*-Verfahren nach einem der Ansprüche 1 bis 8, wobei die Zellprobe Osteoblasten, Myoblasten und/oder periphere mononukleare Blutzellen (PBMC) umfasst.

10. *In vitro*-Verfahren nach Anspruch 9, wobei die Zellprobe PBMCs umfasst.

11. *In vitro*-Verfahren nach Anspruch 10, wobei die PBMCs Lymphozyten umfassen.

12. *In vitro*-Verfahren nach einem der Ansprüche 1 bis 11, wobei das Erfassen oder Bestimmen der Serinphosphorylierung von Giα1- und/oder Giα3-Protein(en) in der Zellprobe Isolieren von Giα1- und/oder Giα3-Protein(en) aus der Zellprobe und In-Kontakt-Bringen des/der isolierten Giα1- und/oder Giα3-Proteins/-Proteine mit einem Anti-Phosphoserin-Antikörper umfasst.

## Revendications

1. Méthode *in vitro* de stratification d'un sujet ayant ou à risque de développer une scoliose idiopathique de l'adolescence (AIS), ladite méthode comprenant :
(i) la fourniture d'un échantillon cellulaire isolé du sujet ;
(ii)
(a) la détection ou la détermination de la phosphorylation de sérine de Giα1 dans l'échantillon cellulaire ; et/ou
(b) la détection ou la détermination de la phosphorylation de sérine de Giα3 dans l'échantillon cellulaire ; et
(iii) la stratification dudit sujet dans une sous-classe d'AIS utilisée cliniquement basée sur le profil de la phosphorylation de sérine de la protéine Giα1 ou Giα3 du sujet.

2. Méthode *in vitro* selon la revendication 1, dans laquelle (ii) comprend (a) et (b) et dans laquelle la méthode : comprend en outre la stratification du sujet comme appartenant à :
(1) une première sous-classe d'AIS **caractérisée par** des niveaux élevés de protéines Giα1 et Giα3 sérine-phosphorylées comparé à des niveaux correspondant à ceux d'un témoin ;
(2) une deuxième sous-classe d'AIS **caractérisée par** des niveaux élevés de protéine Giα1 sérine-phosphorylée mais pas de protéine Giα3 sérine-phosphorylée, comparé à des niveaux correspondant à celui d'un témoin ; ou
(3) une troisième sous-classe d'AIS **caractérisée par** des niveaux élevés de protéine Giα3 sérine-phosphorylée mais pas de protéine Giα1 sérine-phosphorylée comparé à des niveaux correspondant à ceux d'un témoin.

3. Méthode *in vitro* selon la revendication 1 ou 2, comprenant en outre la détection ou la détermination de la sérine-phosphorylation de la protéine Giα2 dans l'échantillon cellulaire, dans laquelle des niveaux élevés de protéine Giα2 sérine-phosphorylée sont détectés, comparé à des niveaux correspondant à celui d'un témoin.

4. Méthode *in vitro* selon la revendication 2 ou 3, dans laquelle :
(1) les sujets appartenant à ladite première sous-classe d'AIS ont un faible risque de progression vers une AIS grave ;
(2) les sujets appartenant à ladite deuxième sous-classe d'AIS ont un risque élevé de progression vers une AIS grave ; et
(3) les sujets appartenant à ladite troisième sous-classe d'AIS ont un risque modéré de progression vers une AIS grave.

5. Méthode *in vitro* de prédiction du risque de développement d'une scoliose grave chez un sujet ayant ou à risque de développer une scoliose, ladite méthode comprenant :
(i) la fourniture d'un échantillon cellulaire isolé du sujet ;
(ii) la détection ou la détermination de la phosphorylation de srine de la protéine Giα3 dans l'échantillon cellulaire ;
(iii) la détermination du fait que le sujet est à risque de développer une scoliose grave lorsque la protéine Giα3 sérine-phosphorylée dans l'échantillon cellulaire n'est pas détectée, ou n'est pas élevée comparé aux niveaux correspondant à ceux d'un témoin.

6. Méthode *in vitro* selon la revendication 5, comprenant en outre à l'étape (ii) la détection ou la détermination de la sérine-phosphorylation de la protéine Giα1 et/ou Giα2 dans l'échantillon cellulaire, dans laquelle ledit sujet est à risque de développer une scoliose grave lorsque le niveau de ladite protéine Giα1 ou Giα2 sérine-phosphorylée du sujet est élevé comparé à des niveaux correspondant à ceux d'un témoin et la protéine Giα3 sérine-phosphorylée dans l'échantillon cellulaire n'est pas détectée, ou n'est pas élevée comparé à des niveaux correspondant à ceux d'un témoin.

7. Méthode *in vitro* selon l'une quelconque des revendications 2 à 6, dans laquelle le sujet est un sujet chez qui une scoliose a été diagnostiquée.

8. Méthode *in vitro* selon la revendication 7, dans laquelle la scoliose est la scoliose idiopathique de l'adolescence (AIS).

9. Méthode *in vitro* selon l'une quelconque des revendications 1 à 8, dans laquelle ledit échantillon cellulaire comprend des ostéoblastes, des myoblastes et/ou des cellules mononucléaires de sang périphérique (PBMC).

10. Méthode *in vitro* selon la revendication 9, dans laquelle ledit échantillon cellulaire comprend des PBMC.

11. Méthode *in vitro* selon la revendication 10, dans laquelle lesdites PBMC comprennent des lymphocytes.

12. Méthode *in vitro* selon l'une quelconque des revendications 1 à 11, dans laquelle la détection ou la détermination de la phosphorylation de sérine de la/des protéine(s) Giα1 et/ou Giα3 dans l'échantillon cellulaire comprend l'isolement de la/des protéine(s) Giα1 et/ou Giα3 de l'échantillon cellulaire et la mise en contact de la/des protéine(s) Giα1 et/ou Giα3 avec un anticorps anti-phosphosérine.
